# EUROPEAN PATENT APPLICATION

(11) **EP 4 596 580 A1**
(43) Date of publication of application: **06.08.2025**
(21) Application number: 23870576.8
(22) Date of filing: 21.09.2023
(51) Int. Cl.: C07K 16/28, C12N 15/13, A61K 39/395, A61P 11/00, A61P 37/00

(54) **ANTIBODY FOR SPECIFICALLY RECOGNIZING LIGHT AND USE THEREOF**

(30) Priority: 27.09.2022 CN 202211184544; 13.07.2023 CN 202310861858
(71) Applicant: Staidson (Beijing) Biopharmaceuticals Co., Ltd., Beijing 100176 (CN)
(72) Inventor: ZHANG, Miaomin, Beijing 100176 (CN); WANG, Guowu, Beijing 100176 (CN); YU, Debin, Beijing 100176 (CN); CUI, Jinglan, Beijing 100176 (CN)
(74) Representative: Studio Torta S.p.A.
(86) International application number: PCT/CN2023/120362
(87) International publication number: WO 2024/067344

(57) **Abstract**

The present invention relates to an antibody or an antigen-binding fragment for specifically recognizing LIGHT, a preparation method therefor, and use thereof.

## Description

### CROSS-REFERENCE TO RELATED APPLICATION

This application claims priority to the Chinese Application with the application number 202211184544.9, filed 2022.09.27, named "ANTIBODIES SPECIFICALLY RECOGNIZING LIGHT AND USES THEREOF", and the Chinese Application with the application number 202310861858.6, filed 2023.07.13, named "ANTIBODIES SPECIFICALLY RECOGNIZING LIGHT AND USES THEREOF" which are incorporated herein by reference in its entirety.

### SUBMISSION OF SEQUENCE LISTING ON XML FILE

The content of the electronic sequence listing is incorporated herein by reference in its entirety: LIGHT_WIPO_seq.xml, size: 92KB, date recorded: 2023.07.11.

### FIELD

This application pertains to antibodies that specifically recognize LIGHT, and methods of manufacture and uses thereof, including methods of treating diseases and/or conditions caused by the disorder of the LIGHT signaling pathway, such as autoimmune disease and/or inflammatory disease.

### BACKGROUND

LIGHT (lymphotoxin-like, exhibits inducible expression, and competes with herpes simplex virus glycoprotein D for HVEM, a receptor expressed by T lymphocytes), as a member of the tumor necrosis factor superfamily (TNFSF), also known as TNFSF14 or CD258, is a type II transmembrane protein containing 240 amino acids that can form homotrimers on the cell surface. The trimeric structure of LIGHT enables it to aggregate cell surface receptors that interact with it, thereby activating the co-stimulate signaling pathway (Bobmer et al., 2002, Trends Biochem. Sci. 27, 19-26). The alternative splicing of LIGHT mRNA produces two isoforms: cytoplasmic LIGHT (also known as free LIGHT) and membrane-bound LIGHT (also known as cell surface LIGHT) (Morel et al., 2000, J. Immunol. 1654397-4404).

LIGHT is not expressed on native T cells, but is upregulated in activated T cells. LIGHT is expressed on the surface of T cells in a strictly regulated manner after activation, appearing within 4 hours, reaching its peak at 12-24 hours, and disappearing at 48 hours (Castellano et al., 2002, J Biol Chem 277 42841-51). The activation of CD8+T cells upregulates the expression of LIGHT more than CD4+T cells. LIGHT is also constitutively expressed on the surface of immature dendritic cells (DCs) at a detectable level (Tamada et al., 2000, J Immunol 164 4105-10). LIGHT is also expressed in natural killer (NK) cells and monocytes (Cohavi et al., 2005, J Immunol 174 646-53).

LIGHT binds to three different receptors: lymphotoxin β receptor (LTβR) (Crowe et al., 1994, Science 264, 707-10, Browning et al., 1997, J Immunol 159, 3288-98), herpes simplex virus entry mediator (HVEM) (Montgomery et al., 1996, Cell 87 (3) 427-36), and decoy receptor 3 (DcR3) (Yu et al., 1999, J Biol Chem 274, 13733-6). The amino acid mutations at positions 119 and 174 in the LIGHT sequence can disrupt its binding ability to HVEM and LTβR receptors (Rooney et al., 2000, J. Biol. Chem. 275, 14307-14315). The signaling pathway of LTβR and HVEM both involve the recruitment of TNFR-associated factors (TRAF) and subsequent activation of caspases and transcription factors. Although there are similarities between these two LIGHT receptors, the downstream signaling events triggered by LTβR and HVEM are different due to their different expression patterns and binding to different signaling molecules (Zhai et al., 1998, J Clin Invest, 102 (6): 1142-1151).

LTβR is present on the surface of epithelial cells, stromal cells, immature DCs, and bone marrow cells, but not expressed in lymphocytes (Giles *et al.,* 2018, *Front Immunol. 9:2585*)*.* LTβR signaling induces activation of genes involved in pro-inflammatory responses, including MIP-1/2, adhesion molecules, and chemokines (Dejardin *et al.,* 2002, *Immunity. 17* (4): 525). In addition, studies have reported that the LIGHT-LTβR signal controls lipid balance and plays a role in the process of dyslipidemia (Combes JL et al., 2007, J Exp Med. 204 (8): 1757-1764). During normal biological development, the interaction between LIGHT-LTβR is considered a component of lymphatic structure development and maintenance (Lu *et al.,* 2014, *Front Immunol.* 5:47). Scheu et al. found that the absence of LIGHT and LTβ in mice resulted in the destruction of all secondary lymphoid structures (Scheu et al., 2002, J Exp Med 195:1613-1624).

After binding to LIGHT, LTβR was shown to be associated with a signal complex including TRAF2, TRAF3, and the κB inhibitor (IκB) kinase complex (IKK). In addition, the interaction between LIGHT-LTβR mediates the activation of c-Jun N-terminal kinase (JNK), activator protein-1 (AP-1), and nuclear factor-κB (NF-κB) in a TRAF2 dependent manner (Kim WJ et al., 2005, Immunology. 114 (2): 272-279.). The apoptosis mediated by LIGHT-LTβR seems to depend on TRAF3 rather than TRAF2 (Rooney IA et al., 2000, J Biol Chem. 275 (19): 14307-14315.). The effects of LIGHT-LTβR signaling are wide-ranging. On the one hand, the function of LIGHT-LTβR can positively regulate and support anti-tumor immune responses, including increasing the sensitivity of cancer cells to immune responses, repairing chaotic tumor blood vessels, and supporting the migration and infiltration of effector cells into tumors (Joseph G. et al., Frontiers in Immunology, 2020). On the other hand, the study by Conlon demonstrated that therapeutic inhibition of LTβR signaling can restore lung structure from smoking induced emphysema and airway fibrosis. Blocking the action of LTβR signaling can inhibit the formation of iBALT, apoptosis of alveolar epithelial cells, and restart endogenous Wnt/β-catenin driven alveolar regeneration (Conlon et al., 2020, Nature. 588 (7836): 151-156).

Herpes simplex virus entry mediator (HVEM), also known as tumor necrosis factor receptor superfamily member 14 (TNFRSF14), was initially thought to be a mediator of type 1 herpes simplex virus invasion into human or mouse cells (Montgomery, Cell 87 (3), "427-" 436, 1996). HVEM is expressed in major human tissues, with the highest expression level in hematopoietic system cells. HVEM is an insoluble transmembrane protein that includes intracellular domains, transmembrane domains, and extracellular domains (referred to as extracellular domains). The extracellular domain of HVEM includes three cysteine rich domains (CRDs), known as CRD1, CRD2, and CRD3. HVEM can interact with various ligands, which transmit activation or inhibition signals downstream by binding to the CRD domain of HVEM. Some of these ligands deliver co-stimulate signals, such as LIGHT and LTα; Other ligands deliver co-inhibitory signals, such as CD160, glycoprotein D (gD), and BTLA (Murphy et al., Annu Rev Immunol. 28:389, 2010). The CRD2 and CRD3 domains of HVEM interact with LIGHT (Ware et al., 2008, Immunol. Rev. 223, 186-201); BTLA and CD160 bind to CRD1 and CRD2 of HVEM and compete with HSV gD binding (Compaan et al., 2005, J. Biol. Chem. 280, 39553-39561.). There are studies reporting that the deletion of the CRD1 domain can lead to the transmission of co-stimulate signals of HVEM-Ig, indicating that CRD1 is essential for inhibitory signal transmission induced by recombinant HVEM-Ig fusion proteins (Adams et al., 2002, Am. J. Transplant. 2, 12-18).

The interaction between LIGHT and HVEM is responsible for the main immunostimulatory properties of LIGHT (Steinberg et al., 2011, Immunol Rev. 244:169-87). HVEM expressed on lymphocytes, NK cells, smooth muscle, and epithelial cells serves as an important T cell stimulant, leading to T cell activation, proliferation, and survival (Ware CF et al., 2011, Curr Opin Immunol 23:627-31). In order to activate T effector cells, the co-stimulate effect of HVEM on LIGHT is necessary, and this co-stimulate effect occurs in a way that is independent of CD28 T cells on T cells. This interaction that promotes inflammation increases the expression of Th1 cytokines IFNγ and GM-CSF. The contact between LIGHT and HVEM can induce the recruitment of TRAFs (including TRAF2), as well as the activation of NF-κB and JNK/AP-1 transcription factors, which contribute to the survival of T cells in vivo. In addition, HVEM can also trigger NK cells to produce IFNγ through the nuclear factor-κB (NF-κB) RelA/p50 signaling mediated by LIGHT (Fan et al., 2006, Blood 107:1342-51). The LIGHT produced by tumor induced NK cells is an important component of NK-DC cascade, which occurs during the initiation stage of new anti-tumor responses (Holmes et al., 2014, Proc Natl Acad Sci USA. 111: E5688-96). Therefore, LIGHT-HVEM mediated T cell co-stimulate and NK-DC cascade play an important role in generating anti-tumor immunity in the therapeutic context.

Patent application WO2008027338A2 discloses an antibody capable of specifically binding to hLIGHT peptides, as well as a method for treating hLIGHT mediated diseases in an individual, comprising administering an antibody that specifically binds to hLIGHT peptides, such as a fully humanized antibody, to the individual.

In summary, existing evidence suggests that LIGHT can activate T cells or innate immune cell-mediated inflammatory responses by binding to HVEM or LTβR receptors, leading to autoimmune and/or inflammatory diseases. Therefore, using therapeutic antibodies to antagonize LIGHT can inhibit the immune response induced by this signaling pathway, providing a new treatment method for the above-mentioned related diseases.

The disclosures of all publications, patents, patent applications and published patent applications referred to herein are hereby incorporated herein by reference in their entirety.

### BRIEF SUMMARY OF THE APPLICATION

In some embodiments, there is provided an isolated anti-LIGHT antibody comprising: a heavy chain variable domain (V_{H}) comprising a heavy chain complementarity determining region (HC-CDR) 1 comprising GYFIN (SEQ ID NO: 1); an HC-CDR2 comprising RIYPYNVX₁TFYNQNFKG (SEQ ID NO: 45), wherein X₁ is D or N; and an HC-CDR3 comprising GTHYYGSSGAMDY (SEQ ID NO: 16); and a light chain variable domain (V_{L}) comprising a light chain complementarity determining region (LC-CDR) 1 comprising KASQNVGTAVA (SEQ ID NO: 23); an LC-CDR2 comprising SASNRYT (SEQ ID NO: 30); and an LC-CDR3 comprising QQYSSYPYT (SEQ ID NO: 37).

In some embodiments, there is provided an isolated anti-LIGHT antibody comprising: a V_{H} comprising an HC-CDR1 comprising the amino acid sequence of SEQ ID NO: 1, or a variant thereof comprising up to about 3 amino acid substitutions; an HC-CDR2 comprising the amino acid sequence of any one of SEQ ID NOs: 8-9, or a variant thereof comprising up to about 3 amino acid substitutions; and an HC-CDR3 comprising the amino acid sequence of SEQ ID NO: 16, or a variant thereof comprising up to about 3 amino acid substitutions; and a V_{L} comprising an LC-CDR1 comprising the amino acid sequence of SEQ ID NO: 23, or a variant thereof comprising up to about 3 amino acid substitutions; an LC-CDR2 comprising the amino acid sequence of SEQ ID NO: 30, or a variant thereof comprising up to about 3 amino acid substitutions; and an LC-CDR3 comprising the amino acid sequence of SEQ ID NO: 37, or a variant thereof comprising up to about 3 amino acid substitutions.

In some embodiments, there is provided an isolated anti-LIGHT antibody comprising a V_{H} comprising an HC-CDR1, an HC-CDR2, and an HC-CDR3 of the V_{H} comprising the amino acid sequence of any one of SEQ ID NOs: 46-53; and a V_{L} comprising an LC-CDR1, an LC-CDR2, and an LC-CDR3 of the V_{L} comprising the amino acid sequence of any one of SEQ ID NOs: 62-65.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 46; and a V_{L} comprising an LC-CDR1, an LC-CDR2, and an LC-CDR3 of a V_{L} comprising the amino acid sequence of SEQ ID NO: 62; (ii) a V_{H} comprising an HC-CDR1, an HC-CDR2, and an HC-CDR3 of a V_{H} comprising the amino acid sequence of SEQ ID NO: 47; and a V_{L} comprising an LC-CDR1, an LC-CDR2, and an LC-CDR3 of a V_{L} comprising the amino acid sequence of SEQ ID NO: 63; (iii) a V_{H} comprising an HC-CDR1, an HC-CDR2, and an HC-CDR3 of a V_{H} comprising the amino acid sequence of SEQ ID NO: 48; and a V_{L} comprising an LC-CDR1, an LC-CDR2, and an LC-CDR3 of a V_{L} comprising the amino acid sequence of SEQ ID NO: 63; (iv) a V_{H} comprising an HC-CDR1, an HC-CDR2, and an HC-CDR3 of a V_{H} comprising the amino acid sequence of SEQ ID NO: 49; and a V_{L} comprising an LC-CDR1, an LC-CDR2, and an LC-CDR3 of a V_{L} comprising the amino acid sequence of SEQ ID NO: 63; (v) a V_{H} comprising an HC-CDR1, an HC-CDR2, and an HC-CDR3 of a V_{H} comprising the amino acid sequence of SEQ ID NO: 50; and a V_{L} comprising an LC-CDR1, an LC-CDR2, and an LC-CDR3 of a V_{L} comprising the amino acid sequence of SEQ ID NO: 63; (vi) a V_{H} comprising an HC-CDR1, an HC-CDR2, and an HC-CDR3 of a V_{H} comprising the amino acid sequence of SEQ ID NO: 47; and a V_{L} comprising an LC-CDR1, an LC-CDR2, and an LC-CDR3 of a V_{L} comprising the amino acid sequence of SEQ ID NO: 64; (vii) a V_{H} comprising an HC-CDR1, an HC-CDR2, and an HC-CDR3 of a V_{H} comprising the amino acid sequence of SEQ ID NO: 48; and a V_{L} comprising an LC-CDR1, an LC-CDR2, and an LC-CDR3 of a V_{L} comprising the amino acid sequence of SEQ ID NO: 64; (viii) a V_{H} comprising an HC-CDR1, an HC-CDR2, and an HC-CDR3 of a V_{H} comprising the amino acid sequence of SEQ ID NO: 49; and a V_{L} comprising an LC-CDR1, an LC-CDR2, and an LC-CDR3 of a V_{L} comprising the amino acid sequence of SEQ ID NO: 64; (ix) a V_{H} comprising an HC-CDR1, an HC-CDR2, and an HC-CDR3 of a V_{H} comprising the amino acid sequence of SEQ ID NO: 50; and a V_{L} comprising an LC-CDR1, an LC-CDR2, and an LC-CDR3 of a V_{L} comprising the amino acid sequence of SEQ ID NO: 64; (x) a V_{H} comprising an HC-CDR1, an HC-CDR2, and an HC-CDR3 of a V_{H} comprising the amino acid sequence of SEQ ID NO: 47; and a V_{L} comprising an LC-CDR1, an LC-CDR2, and an LC-CDR3 of a V_{L} comprising the amino acid sequence of SEQ ID NO: 65; (xi) a V_{H} comprising an HC-CDR1, an HC-CDR2, and an HC-CDR3 of a V_{H} comprising the amino acid sequence of SEQ ID NO: 48; and a V_{L} comprising an LC-CDR1, an LC-CDR2, and an LC-CDR3 of a V_{L} comprising the amino acid sequence of SEQ ID NO: 65; (xii) a V_{H} comprising an HC-CDR1, an HC-CDR2, and an HC-CDR3 of a V_{H} comprising the amino acid sequence of SEQ ID NO: 49; and a V_{L} comprising an LC-CDR1, an LC-CDR2, and an LC-CDR3 of a V_{L} comprising the amino acid sequence of SEQ ID NO: 65; (xiii) a V_{H} comprising an HC-CDR1, an HC-CDR2, and an HC-CDR3 of a V_{H} comprising the amino acid sequence of SEQ ID NO: 50; and a V_{L} comprising an LC-CDR1, an LC-CDR2, and an LC-CDR3 of a V_{L} comprising the amino acid sequence of SEQ ID NO: 65; (xiv) a V_{H} comprising an HC-CDR1, an HC-CDR2, and an HC-CDR3 of a V_{H} comprising the amino acid sequence of SEQ ID NO: 51; and a V_{L} comprising an LC-CDR1, an LC-CDR2, and an LC-CDR3 of a V_{L} comprising the amino acid sequence of SEQ ID NO: 63; (xv) a V_{H} comprising an HC-CDR1, an HC-CDR2, and an HC-CDR3 of a V_{H} comprising the amino acid sequence of SEQ ID NO: 52; and a V_{L} comprising an LC-CDR1, an LC-CDR2, and an LC-CDR3 of a V_{L} comprising the amino acid sequence of SEQ ID NO: 65; (xvi) a V_{H} comprising an HC-CDR1, an HC-CDR2, and an HC-CDR3 of a V_{H} comprising the amino acid sequence of SEQ ID NO: 53; and a V_{L} comprising an LC-CDR1, an LC-CDR2, and an LC-CDR3 of a V_{L} comprising the amino acid sequence of SEQ ID NO: 65; or (xvii) a V_{H} comprising an HC-CDR1, an HC-CDR2, and an HC-CDR3 of a V_{H} comprising the amino acid sequence of SEQ ID NO: 51; and a V_{L} comprising an LC-CDR1, an LC-CDR2, and an LC-CDR3 of a V_{L} comprising the amino acid sequence of SEQ ID NO: 65.

In some embodiments, there is provided an isolated anti-LIGHT antibody comprising: (i) a V_{H} comprising an HC-CDR1 comprising the amino acid sequence of SEQ ID NO: 1, an HC-CDR2 comprising the amino acid sequence of SEQ ID NO: 8, and an HC-CDR3 comprising the amino acid sequence of SEQ ID NO: 16, or a variant thereof comprising up to about 5 amino acid substitutions in the HC-CDRs; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37, or a variant thereof comprising up to about 5 amino acid substitutions in the LC-CDRs; or (ii) a V_{H} comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 16, or a variant thereof comprising up to about 5 amino acid substitutions in the HC-CDRs; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of any one of SEQ ID NOs: 46-53, or a variant thereof having at least about 80% sequence identity to the amino acid sequence of any one of SEQ ID NOs: 46-53; and a V_{L} comprising the amino acid sequence of any one of SEQ ID NOs: 62-65, or a variant thereof having at least about 80% sequence identity to the amino acid sequence of any one of SEQ ID NOs: 62-65.

In some embodiments, the isolated anti-LIGHT antibody comprises: (i) a V_{H} comprising the amino acid sequence of SEQ ID NO: 46, or a variant thereof having at least about 80% sequence identity to the amino acid sequence of SEQ ID NO: 46; and a V_{L} comprising the amino acid sequence of SEQ ID NO: 62, or a variant thereof having at least about 80% sequence identity to the amino acid sequence of SEQ ID NO: 62; (ii) a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 63, or a variant thereof having at least about 80% sequence identity to the amino acid sequence of SEQ ID NO: 63; (iii) a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 63, or a variant thereof having at least about 80% sequence identity to the amino acid sequence of SEQ ID NO: 63; (iv) a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 63, or a variant thereof having at least about 80% sequence identity to the amino acid sequence of SEQ ID NO: 63; (v) a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 63, or a variant thereof having at least about 80% sequence identity to the amino acid sequence of SEQ ID NO: 63; (vi) a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 64, or a variant thereof having at least about 80% sequence identity to the amino acid sequence of SEQ ID NO: 64; (vii) a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 64, or a variant thereof having at least about 80% sequence identity to the amino acid sequence of SEQ ID NO: 64; (viii) a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 64, or a variant thereof having at least about 80% sequence identity to the amino acid sequence of SEQ ID NO: 64; (ix) a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 64, or a variant thereof having at least about 80% sequence identity to the amino acid sequence of SEQ ID NO: 64; (x) a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 65, or a variant thereof having at least about 80% sequence identity to the amino acid sequence of SEQ ID NO: 65; (xi) a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 65, or a variant thereof having at least about 80% sequence identity to the amino acid sequence of SEQ ID NO: 65; (xii) a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 65, or a variant thereof having at least about 80% sequence identity to the amino acid sequence of SEQ ID NO: 65; (xiii) a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 65, or a variant thereof having at least about 80% sequence identity to the amino acid sequence of SEQ ID NO: 65; (xiv) a V_{H} comprising the amino acid sequence of SEQ ID NO: 51, or a variant thereof having at least about 80% sequence identity to the amino acid sequence of SEQ ID NO: 51; and a V_{L} comprising the amino acid sequence of SEQ ID NO: 63, or a variant thereof having at least about 80% sequence identity to the amino acid sequence of SEQ ID NO: 63; (xv) a V_{H} comprising the amino acid sequence of SEQ ID NO: 52, or a variant thereof having at least about 80% sequence identity to the amino acid sequence of SEQ ID NO: 52; and a V_{L} comprising the amino acid sequence of SEQ ID NO: 65, or a variant thereof having at least about 80% sequence identity to the amino acid sequence of SEQ ID NO: 65; (xvi) a V_{H} comprising the amino acid sequence of SEQ ID NO: 53, or a variant thereof having at least about 80% sequence identity to the amino acid sequence of SEQ ID NO: 53; and a V_{L} comprising the amino acid sequence of SEQ ID NO: 65, or a variant thereof having at least about 80% sequence identity to the amino acid sequence of SEQ ID NO: 65; or (xvii) a V_{H} comprising the amino acid sequence of SEQ ID NO: 51, or a variant thereof having at least about 80% sequence identity to the amino acid sequence of SEQ ID NO: 51; and a V_{L} comprising the amino acid sequence of SEQ ID NO: 65, or a variant thereof having at least about 80% sequence identity to the amino acid sequence of SEQ ID NO: 65.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 54; and a V_{L} comprising an LC-CDR1, an LC-CDR2, and an LC-CDR3 of a V_{L} comprising the amino acid sequence of SEQ ID NO: 66.

In some embodiments, there is provided an isolated anti-LIGHT antibody comprising: (i) a V_{H} comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17, or a variant thereof comprising up to about 5 amino acid substitutions in the HC-CDRs; and a V_{L} comprising an LC-CDR1 comprising the amino acid sequence of SEQ ID NO: 24, an LC-CDR2 comprising the amino acid sequence of SEQ ID NO: 31, and an LC-CDR3 comprising the amino acid sequence of SEQ ID NO: 38, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of SEQ ID NO: 54, or a variant thereof having at least about 80% sequence identity to the amino acid sequence of SEQ ID NO: 54; and a V_{L} comprising the amino acid sequence of SEQ ID NO: 66, or a variant thereof having at least about 80% sequence identity to the amino acid sequence of SEQ ID NO: 66.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 55; and a V_{L} comprising an LC-CDR1, an LC-CDR2, and an LC-CDR3 of a V_{L} comprising the amino acid sequence of SEQ ID NO: 67.

In some embodiments, there is provided an isolated anti-LIGHT antibody comprising: (i) a V_{H} comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17, or a variant thereof comprising up to about 5 amino acid substitutions in the HC-CDRs; and a V_{L} comprising an LC-CDR1 comprising the amino acid sequence of SEQ ID NO: 25, an LC-CDR2 comprising the amino acid sequence of SEQ ID NO: 31, and an LC-CDR3 comprising the amino acid sequence of SEQ ID NO: 39, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of SEQ ID NO: 55, or a variant thereof having at least about 80% sequence identity to the amino acid sequence of SEQ ID NO: 55; and a V_{L} comprising the amino acid sequence of SEQ ID NO: 67, or a variant thereof having at least about 80% sequence identity to the amino acid sequence of SEQ ID NO: 67.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 56; and a V_{L} comprising an LC-CDR1, an LC-CDR2, and an LC-CDR3 of a V_{L} comprising the amino acid sequence of SEQ ID NO: 68.

In some embodiments, there is provided an isolated anti-LIGHT antibody comprising: (i) a V_{H} comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18, or a variant thereof comprising up to about 5 amino acid substitutions in the HC-CDRs; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 40, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of SEQ ID NO: 56, or a variant thereof having at least about 80% sequence identity to the amino acid sequence of SEQ ID NO: 56; and a V_{L} comprising the amino acid sequence of SEQ ID NO: 68, or a variant thereof having at least about 80% sequence identity to the amino acid sequence of SEQ ID NO: 68.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 57; and a V_{L} comprising an LC-CDR1, an LC-CDR2, and an LC-CDR3 of a V_{L} comprising the amino acid sequence of SEQ ID NO: 69.

In some embodiments, there is provided an isolated anti-LIGHT antibody comprising: (i) a V_{H} comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18, or a variant thereof comprising up to about 5 amino acid substitutions in the HC-CDRs; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 41, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of SEQ ID NO: 57, or a variant thereof having at least about 80% sequence identity to the amino acid sequence of SEQ ID NO: 57; and a V_{L} comprising the amino acid sequence of SEQ ID NO: 69, or a variant thereof having at least about 80% sequence identity to the amino acid sequence of SEQ ID NO: 69.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 58; and a V_{L} comprising an LC-CDR1, an LC-CDR2, and an LC-CDR3 of a V_{L} comprising the amino acid sequence of SEQ ID NO: 70.

In some embodiments, there is provided an isolated anti-LIGHT antibody comprising: (i) a V_{H} comprising an HC-CDR1 comprising the amino acid sequence of SEQ ID NO: 4, an HC-CDR2 comprising the amino acid sequence of SEQ ID NO: 12, and an HC-CDR3 comprising the amino acid sequence of SEQ ID NO: 19, or a variant thereof comprising up to about 5 amino acid substitutions in the HC-CDRs; and a V_{L} comprising an LC-CDR1 comprising the amino acid sequence of SEQ ID NO: 27, an LC-CDR2 comprising the amino acid sequence of SEQ ID NO: 33, and an LC-CDR3 comprising the amino acid sequence of SEQ ID NO: 42, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of SEQ ID NO: 58, or a variant thereof having at least about 80% sequence identity to the amino acid sequence of SEQ ID NO: 58; and a V_{L} comprising the amino acid sequence of SEQ ID NO: 70, or a variant thereof having at least about 80% sequence identity to the amino acid sequence of SEQ ID NO: 70.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 59; and a V_{L} comprising an LC-CDR1, an LC-CDR2, and an LC-CDR3 of a V_{L} comprising the amino acid sequence of SEQ ID NO: 71.

In some embodiments, there is provided an isolated anti-LIGHT antibody comprising: (i) a V_{H} comprising an HC-CDR1 comprising the amino acid sequence of SEQ ID NO: 5, an HC-CDR2 comprising the amino acid sequence of SEQ ID NO: 13, and an HC-CDR3 comprising the amino acid sequence of SEQ ID NO: 20, or a variant thereof comprising up to about 5 amino acid substitutions in the HC-CDRs; and a V_{L} comprising an LC-CDR1 comprising the amino acid sequence of SEQ ID NO: 28, an LC-CDR2 comprising the amino acid sequence of SEQ ID NO: 34, and an LC-CDR3 comprising the amino acid sequence of SEQ ID NO: 43, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of SEQ ID NO: 59, or a variant thereof having at least about 80% sequence identity to the amino acid sequence of SEQ ID NO: 59; and a V_{L} comprising the amino acid sequence of SEQ ID NO: 71, or a variant thereof having at least about 80% sequence identity to the amino acid sequence of SEQ ID NO: 71.

In some embodiments, there is provided an isolated anti-LIGHT antibody comprising: a heavy chain variable domain (V_{H}) comprising a heavy chain complementarity determining region (HC-CDR) 1 comprising DHIMN (SEQ ID NO: 6); an HC-CDR2 comprising RIYPVSGETNYNQKFMG (SEQ ID NO: 14); and an HC-CDR3 comprising GSYYWNAMDY (SEQ ID NO: 21); and a light chain variable domain (V_{L}) comprising a light chain complementarity determining region (LC-CDR) 1 comprising KASQSVDFDGDSYMN (SEQ ID NO: 29) or KASQSVDFDGESYMN (SEQ ID NO: 83); an LC-CDR2 comprising SASNLES (SEQ ID NO: 35); and an LC-CDR3 comprising QQSIEDPWT (SEQ ID NO: 43).

In some embodiments, there is provided an isolated anti-LIGHT antibody comprising: a V_{H} comprising an HC-CDR1 comprising the amino acid sequence of SEQ ID NO: 6, or a variant thereof comprising up to about 3 amino acid substitutions; an HC-CDR2 comprising the amino acid sequence of SEQ ID NO: 14, or a variant thereof comprising up to about 3 amino acid substitutions; and an HC-CDR3 comprising the amino acid sequence of SEQ ID NO: 21, or a variant thereof comprising up to about 3 amino acid substitutions; and a V_{L} comprising an LC-CDR1 comprising the amino acid sequence of any one of SEQ ID NOs: 29 or 83, or a variant thereof comprising up to about 3 amino acid substitutions; an LC-CDR2 comprising the amino acid sequence of SEQ ID NO: 35, or a variant thereof comprising up to about 3 amino acid substitutions; and an LC-CDR3 comprising the amino acid sequence of SEQ ID NO: 43, or a variant thereof comprising up to about 3 amino acid substitutions.

In some embodiments, there is provided an isolated anti-LIGHT antibody comprising a V_{H} comprising an HC-CDR1, an HC-CDR2, and an HC-CDR3 of the V_{H} comprising the amino acid sequence of any one of SEQ ID NOs: 60, 84-85; and a V_{L} comprising an LC-CDR1, an LC-CDR2, and an LC-CDR3 of the V_{L} comprising the amino acid sequence of any one of SEQ ID NOs: 72, 86-91.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 60; and a V_{L} comprising an LC-CDR1, an LC-CDR2, and an LC-CDR3 of a V_{L} comprising the amino acid sequence of SEQ ID NO: 72; (ii) a V_{H} comprising an HC-CDR1, an HC-CDR2, and an HC-CDR3 of a V_{H} comprising the amino acid sequence of SEQ ID NO: 84; and a V_{L} comprising an LC-CDR1, an LC-CDR2, and an LC-CDR3 of a V_{L} comprising the amino acid sequence of SEQ ID NO: 86; (iii) a V_{H} comprising an HC-CDR1, an HC-CDR2, and an HC-CDR3 of a V_{H} comprising the amino acid sequence of SEQ ID NO: 85; and a V_{L} comprising an LC-CDR1, an LC-CDR2, and an LC-CDR3 of a V_{L} comprising the amino acid sequence of SEQ ID NO: 87; (iv) a V_{H} comprising an HC-CDR1, an HC-CDR2, and an HC-CDR3 of a V_{H} comprising the amino acid sequence of SEQ ID NO: 84; and a V_{L} comprising an LC-CDR1, an LC-CDR2, and an LC-CDR3 of a V_{L} comprising the amino acid sequence of SEQ ID NO: 88; (v) a V_{H} comprising an HC-CDR1, an HC-CDR2, and an HC-CDR3 of a V_{H} comprising the amino acid sequence of SEQ ID NO: 84; and a V_{L} comprising an LC-CDR1, an LC-CDR2, and an LC-CDR3 of a V_{L} comprising the amino acid sequence of SEQ ID NO: 89; (vi) a V_{H} comprising an HC-CDR1, an HC-CDR2, and an HC-CDR3 of a V_{H} comprising the amino acid sequence of SEQ ID NO: 85; and a V_{L} comprising an LC-CDR1, an LC-CDR2, and an LC-CDR3 of a V_{L} comprising the amino acid sequence of SEQ ID NO: 90; or (vii) a V_{H} comprising an HC-CDR1, an HC-CDR2, and an HC-CDR3 of a V_{H} comprising the amino acid sequence of SEQ ID NO: 84; and a V_{L} comprising an LC-CDR1, an LC-CDR2, and an LC-CDR3 of a V_{L} comprising the amino acid sequence of SEQ ID NO: 91.

In some embodiments, there is provided an isolated anti-LIGHT antibody comprising: (i) a V_{H} comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21, or a variant thereof comprising up to about 5 amino acid substitutions in the HC-CDRs; and a V_{L} comprising an LC-CDR1 comprising the amino acid sequence of SEQ ID NO: 29, an LC-CDR2 comprising the amino acid sequence of SEQ ID NO: 35, and an LC-CDR3 comprising the amino acid sequence of SEQ ID NO: 43, or a variant thereof comprising up to about 5 amino acid substitutions in the LC-CDRs; or (ii) a VH comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21, or a variant thereof comprising up to about 5 amino acid substitutions in the HC-CDRs; and a VL comprising an LC-CDR1 comprising the amino acid sequence of SEQ ID NO: 83, an LC-CDR2 comprising the amino acid sequence of SEQ ID NO: 35, and an LC-CDR3 comprising the amino acid sequence of SEQ ID NO: 43, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of any one of SEQ ID NOs: 60, 84-85, or a variant thereof having at least about 80% sequence identity to the amino acid sequence of any one of SEQ ID NOs: 60, 84-85; and a V_{L} comprising the amino acid sequence of any one of SEQ ID NOs: 72, 86-91, or a variant thereof having at least about 80% sequence identity to the amino acid sequence of any one of SEQ ID NOs: 72, 86-91.

In some embodiments, according to any one of the isolated anti-LIGHT antibodies described above, the isolated anti-LIGHT antibody comprises: (i) a V_{H} comprising the amino acid sequence of SEQ ID NO: 60, or a variant thereof having at least about 80% sequence identity to the amino acid sequence of SEQ ID NO: 60; and a V_{L} comprising the amino acid sequence of SEQ ID NO: 72, or a variant thereof having at least about 80% sequence identity to the amino acid sequence of SEQ ID NO: 72; (ii) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 86, or a variant thereof having at least about 80% sequence identity to the amino acid sequence of SEQ ID NO: 86; (iii) a V_{H} comprising the amino acid sequence of SEQ ID NO: 85, or a variant thereof having at least about 80% sequence identity to the amino acid sequence of SEQ ID NO: 85; and a V_{L} comprising the amino acid sequence of SEQ ID NO: 87, or a variant thereof having at least about 80% sequence identity to the amino acid sequence of SEQ ID NO: 87; (iv) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 88, or a variant thereof having at least about 80% sequence identity to the amino acid sequence of SEQ ID NO: 88; (v) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 89, or a variant thereof having at least about 80% sequence identity to the amino acid sequence of SEQ ID NO: 89; (vi) a V_{H} comprising the amino acid sequence of SEQ ID NO: 85, or a variant thereof having at least about 80% sequence identity to the amino acid sequence of SEQ ID NO: 85; and a V_{L} comprising the amino acid sequence of SEQ ID NO: 90, or a variant thereof having at least about 80% sequence identity to the amino acid sequence of SEQ ID NO: 90; or (vii) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 91, or a variant thereof having at least about 80% sequence identity to the amino acid sequence of SEQ ID NO: 91.

In some embodiments, there is provided an isolated anti-LIGHT antibody comprising: a heavy chain variable domain (V_{H}) comprising a heavy chain complementarity determining region (HC-CDR) 1 comprising SYNVH (SEQ ID NO: 7); an HC-CDR2 comprising AVYPGNGDTSYNQKFKG (SEQ ID NO: 15); and an HC-CDR3 comprising GSYYYTSSYFDH (SEQ ID NO: 22); and a light chain variable domain (V_{L}) comprising a light chain complementarity determining region (LC-CDR) 1 comprising KASQSVDFDGDSYMN (SEQ ID NO: 29); an LC-CDR2 comprising TASNLES (SEQ ID NO: 36); and an LC-CDR3 comprising QQSYEDPFT (SEQ ID NO: 44).

In some embodiments, there is provided an isolated anti-LIGHT antibody comprising a V_{H} comprising an HC-CDR1, an HC-CDR2, and an HC-CDR3 of the V_{H} comprising the amino acid sequence of any one of SEQ ID NOs: 61, 92-94; and a V_{L} comprising an LC-CDR1, an LC-CDR2, and an LC-CDR3 of the V_{L} comprising the amino acid sequence of any one of SEQ ID NOs: 73, 95-99.

In some embodiments, there is provided an isolated anti-LIGHT antibody comprising: (i) a V_{H} comprising an HC-CDR1, an HC-CDR2, and an HC-CDR3 of a V_{H} comprising the amino acid sequence of SEQ ID NO: 61; and a V_{L} comprising an LC-CDR1, an LC-CDR2, and an LC-CDR3 of a V_{L} comprising the amino acid sequence of SEQ ID NO: 73.

In some embodiments, there is provided an isolated anti-LIGHT antibody comprising: (i) a V_{H} comprising an HC-CDR1 comprising the amino acid sequence of SEQ ID NO: 7, an HC-CDR2 comprising the amino acid sequence of SEQ ID NO: 15, and an HC-CDR3 comprising the amino acid sequence of SEQ ID NO: 22, or a variant thereof comprising up to about 5 amino acid substitutions in the HC-CDRs; and a V_{L} comprising an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44, or a variant thereof comprising up to about 5 amino acid substitutions in the LC-CDRs.

In some embodiments, according to any one of the isolated anti-LIGHT antibodies described above, the isolated anti-LIGHT antibody comprises: a V_{H} comprising the amino acid sequence of any one of SEQ ID NOs: 61, 92-94, or a variant thereof having at least about 80% sequence identity to the amino acid sequence of any one of SEQ ID NOs: 61, 92-94; and a V_{L} comprising the amino acid sequence of any one of SEQ ID NOs: 73, 95-99, or a variant thereof having at least about 80% sequence identity to the amino acid sequence of any one of SEQ ID NOs: 73, 95-99.

In some embodiments, according to any one of the isolated anti-LIGHT antibodies described above, the isolated anti-LIGHT antibody comprises: (i) a V_{H} comprising the amino acid sequence of SEQ ID NO: 61, or a variant thereof having at least about 80% sequence identity to the amino acid sequence of SEQ ID NO: 61; and a V_{L} comprising the amino acid sequence of SEQ ID NO: 73, or a variant thereof having at least about 80% sequence identity to the amino acid sequence of SEQ ID NO: 73; (ii) a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 95, or a variant thereof having at least about 80% sequence identity to the amino acid sequence of SEQ ID NO: 95; (iii) a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 96, or a variant thereof having at least about 80% sequence identity to the amino acid sequence of SEQ ID NO: 96; (iv) a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 96, or a variant thereof having at least about 80% sequence identity to the amino acid sequence of SEQ ID NO: 96; (v) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 97, or a variant thereof having at least about 80% sequence identity to the amino acid sequence of SEQ ID NO: 97; (vi) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 98, or a variant thereof having at least about 80% sequence identity to the amino acid sequence of SEQ ID NO: 98; (vii) a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 98, or a variant thereof having at least about 80% sequence identity to the amino acid sequence of SEQ ID NO: 98; (viii) a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 98, or a variant thereof having at least about 80% sequence identity to the amino acid sequence of SEQ ID NO: 98; (ix) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 99, or a variant thereof having at least about 80% sequence identity to the amino acid sequence of SEQ ID NO: 99; or (x) a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 99, or a variant thereof having at least about 80% sequence identity to the amino acid sequence of SEQ ID NO: 99.

In some embodiments, there is provided an isolated anti-LIGHT antibody that specifically binds to the human LIGHT with a Kd from about 0.1 pM to about 10 nM.

In some embodiments, there is provided an isolated anti-LIGHT antibody that specifically binds to LIGHT competitively with any one of the isolated anti-LIGHT antibodies described above. In some embodiments, there is provided an isolated anti-LIGHT antibody that specifically binds to the same epitope as any one of isolated anti-LIGHT antibodies described above.

In some embodiments according to any of the isolated anti-LIGHT antibodies described above, the isolated anti-LIGHT antibody comprises an Fc fragment. In some embodiments, the isolated anti-LIGHT antibody is a full-length IgG antibody. In some embodiments, the isolated anti-LIGHT antibody is a full-length IgG1, IgG2, IgG3, or IgG4 antibody. In some embodiments, the anti-LIGHT antibody is a chimeric, human, or humanized antibody. In some embodiments, the anti-LIGHT antibody is an antigen binding fragment selected from the group consisting of a Fab, a Fab', a F(ab)'₂, a Fab'-SH, a single-chain Fv (scFv), an Fv fragment, a dAb, a Fd, a nanobody, a diabody, and a linear antibody.

In some embodiments, there is provided isolated nucleic acid molecule(s) that encodes any one of the anti-LIGHT antibodies described above. In some embodiments, there is provided a vector comprising any one of the nucleic acid molecules described above. In some embodiments, there is provided a host cell comprising any one of the anti-LIGHT antibodies described above, any one of the nucleic acid molecules described above, or any one of the vectors described above. In some embodiments, there is provided a method of producing an anti-LIGHT antibody, comprising: a) culturing any one of the host cells described above under conditions effective to express the anti-LIGHT antibody; and b) obtaining the expressed anti-LIGHT antibody from the host cell.

In some embodiments, there is provided a method of treating a disease or condition in an individual in need thereof, comprising administering to the individual an effective amount of any one of the anti-LIGHT antibodies described above. In some embodiments, there is provided the use of any one of the anti-LIGHT antibodies described herein for the preparation of pharmaceutical compositions for treating a disease or condition in an individual in need. In some embodiments, provided is the use of any one of the anti-LIGHT antibodies described above, or a pharmaceutical composition comprising any one of anti-LIGHT antibodies described above in the manufacture of a medicament for treating a disease or condition. In some embodiments, the disease or condition is associated with LIGHT, comprising autoimmune disease and/or inflammatory disease or condition. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19.

Also provided are pharmaceutical compositions, kits and articles of manufacture comprising any one of the anti-LIGHT antibodies described above.

### BRIEF DESCRIPTION OF THE DRAWINGS

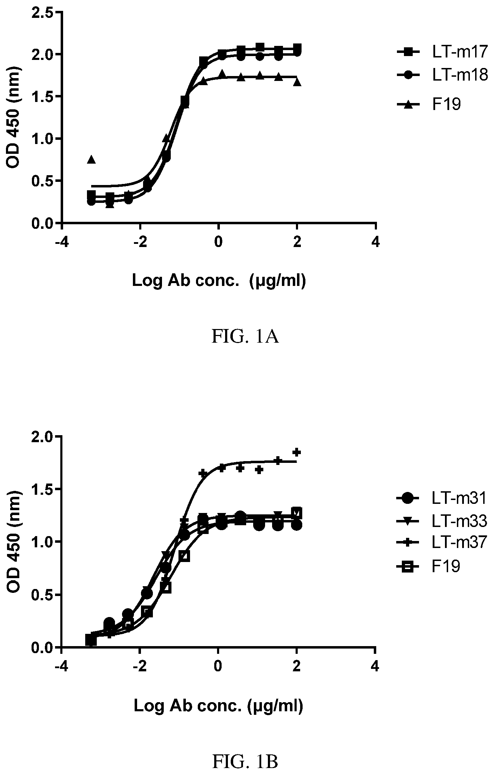

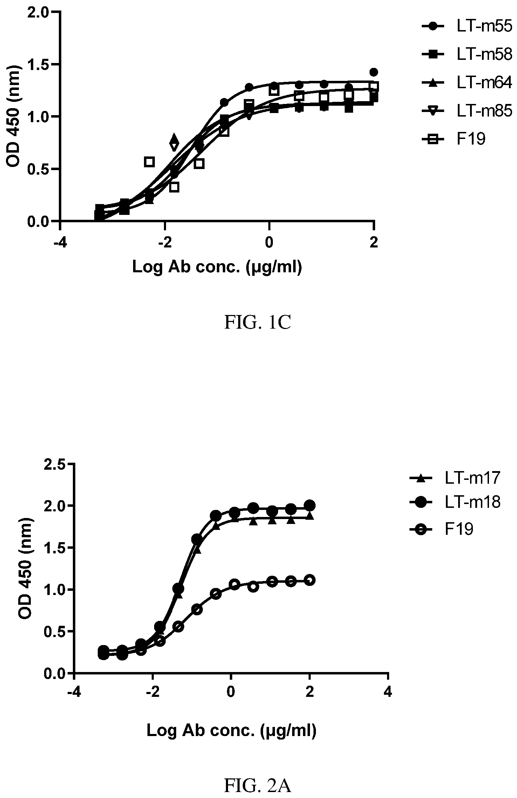

Figure 1 shows the binding affinity of chimeric anti-LIGHT antibodies for human LIGHT analyzed by ELISA. Figure 1A shows the binding affinity of exemplary chimeric anti-LIGHT antibodies LT-m17, and LT-m18 for human LIGHT. Figure 1B shows the binding affinity of exemplary chimeric anti-LIGHT antibodies LT-m31, LT-m33, and LT-m37 for human LIGHT. Figure 1C shows the binding affinity of exemplary chimeric anti-LIGHT antibodies LT-m55, LT-m58, LT-m64, and LT-m85 for human LIGHT.

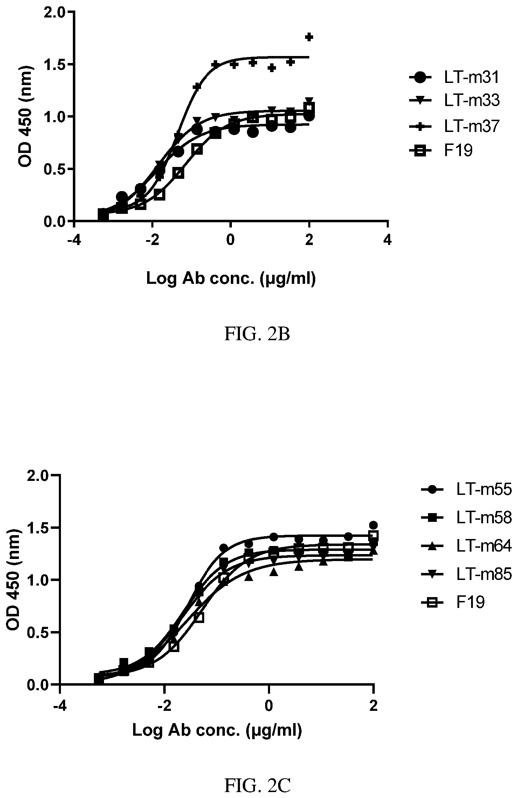

Figure 2 shows the cross-binding activity of chimeric anti-LIGHT antibodies for cynomolgus monkey LIGHT analyzed by ELISA. Figure 2A shows the cross-binding activity of exemplary chimeric anti-LIGHT antibodies LT-m17, and LT-m18 for cynomolgus monkey LIGHT. Figure 2B shows the cross-binding activity of exemplary chimeric anti-LIGHT antibodies LT-m31, LT-m33, and LT-m37 for cynomolgus monkey LIGHT. Figure 2C shows the cross-binding activity of exemplary chimeric anti-LIGHT antibodies LT-m55, LT-m58, LT-m64, and LT-m85 for cynomolgus monkey LIGHT.

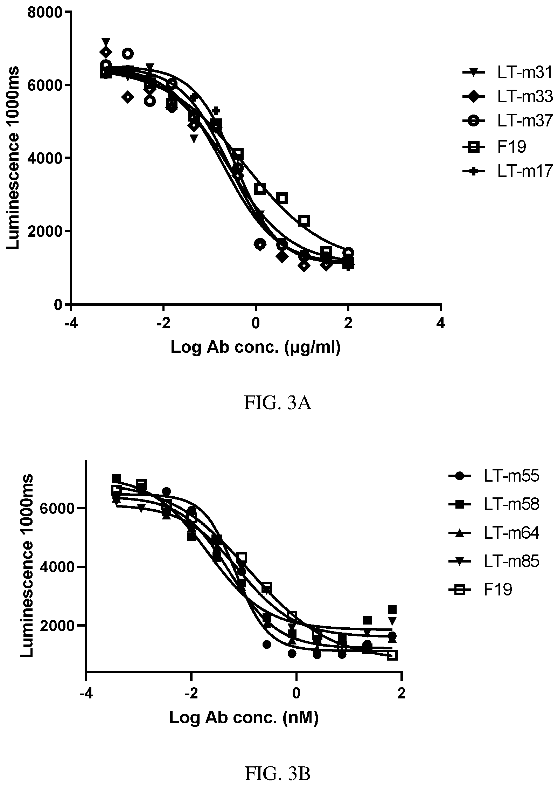

Figure 3A shows the inhibitory activity of exemplary chimeric anti-LIGHT antibodies LT-m17, LT-m31, LT-m33, and LT-m37 on the activation of the LTβR reporter gene by free LIGHT. Figure 3B shows the inhibitory activity of exemplary chimeric anti-LIGHT antibodies LT-m55, LT-m58, LT-m64, and LT-m85 on the activation of the LTβR reporter gene by free LIGHT.

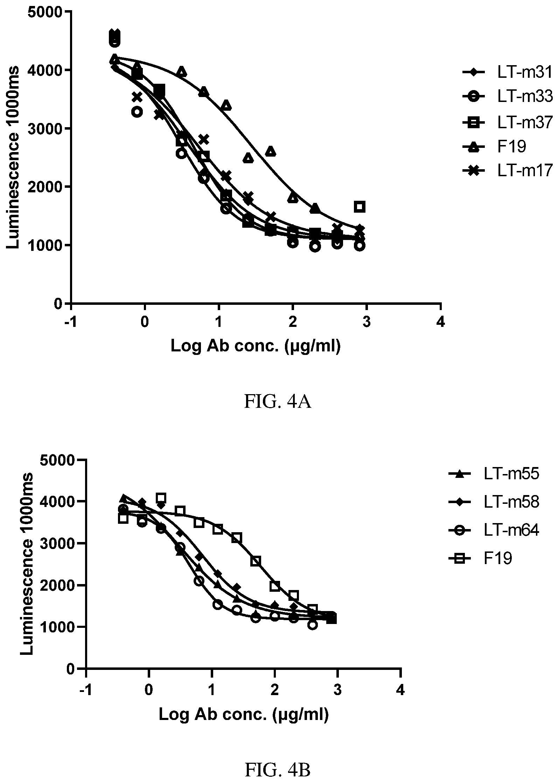

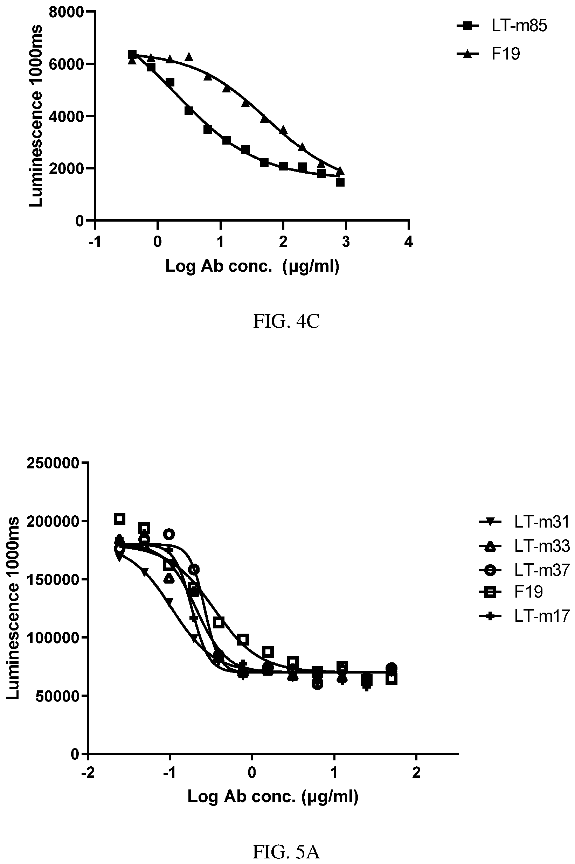

Figure 4A shows the inhibitory activity of exemplary chimeric anti-LIGHT antibodies LT-m17, LT-m31, LT-m33, and LT-m37 on the activation of the LTβR reporter gene by cellular LIGHT. Figure 4B shows the inhibitory activity of exemplary chimeric anti-LIGHT antibodies LT-m55, LT-m58, and LT-m64 on the activation of the LTβR reporter gene by cellular LIGHT. Figure 4C shows the inhibitory activity of an exemplary chimeric anti-LIGHT antibody LT-m85 on the activation of the LTβR reporter gene by cellular LIGHT.

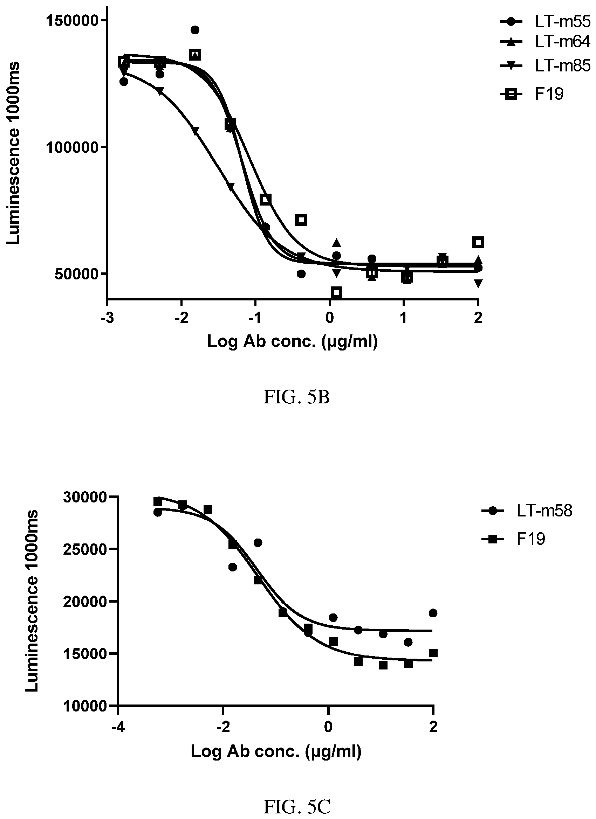

Figure 5A shows the inhibitory activity of exemplary chimeric anti-LIGHT antibodies LT-m17, LT-m31, LT-m33, and LT-m37 on the activation of the HVEM reporter gene by free LIGHT. Figure 5B shows the inhibitory activity of exemplary chimeric anti-LIGHT antibodies LT-m55, LT-m64, and LT-m85 on the activation of the HVEM reporter gene by free LIGHT. Figure 5C shows the inhibitory activity of an exemplary chimeric anti-LIGHT antibody LT-m58 on the activation of the HVEM reporter gene by free LIGHT.

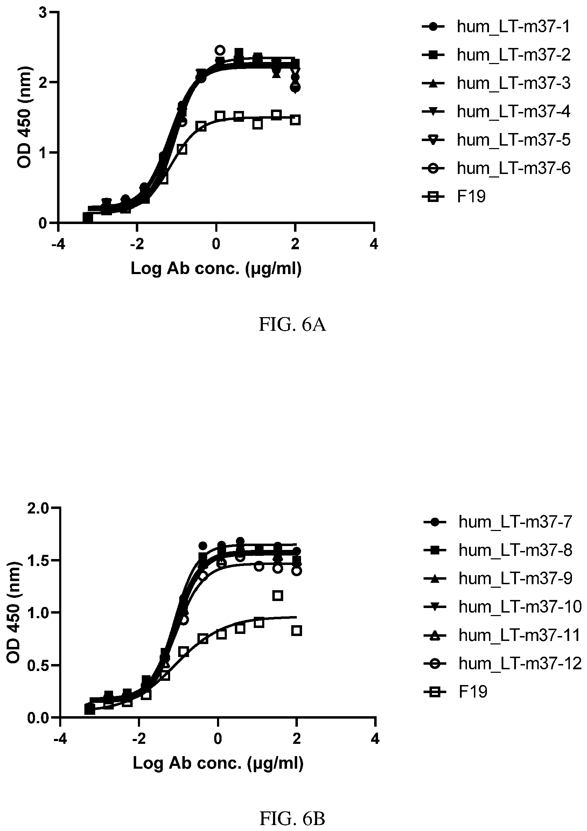

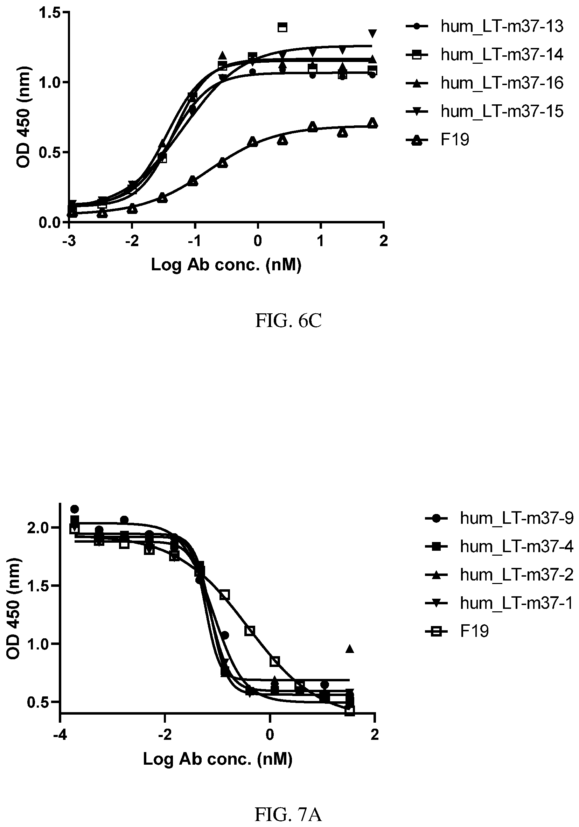

Figure 6 shows the binding affinity of humanized anti-LIGHT antibodies for human LIGHT analyzed by ELISA. Figure 6A shows the binding affinity of exemplary humanized anti-LIGHT antibodies hum_LT-m37-1-hum_LT-m37-6 for human LIGHT. Figure 6B shows the binding affinity of exemplary humanized anti-LIGHT antibodies hum_LT-m37-7~hum_LT-m37-12 for human LIGHT. Figure 6C shows the binding affinity between exemplary humanized anti-LIGHT antibodies hum_LT-m37-13~hum_LT-m37-16 for human LIGHT.

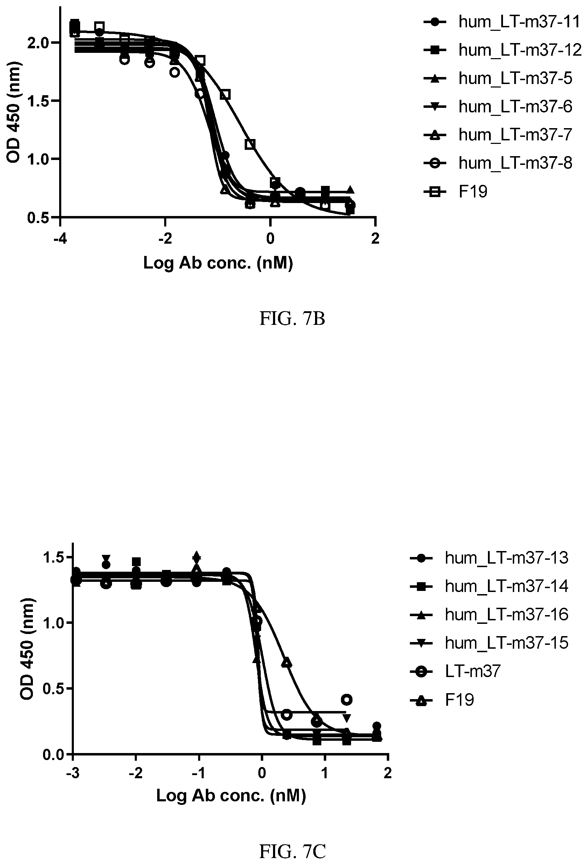

Figure 7 shows the activity of humanized anti-LIGHT antibodies blocking the binding of LIGHT to LTβR by ELISA. Figure 7A shows the activity of exemplary humanized anti-LIGHT antibodies hum_LT-m37-1, hum_LT-m37-2, hum_LT-m37-4, and hum_LT-m37-9 blocking the binding of LIGHT to LTβR by ELISA. Figure 7B shows the activity of exemplary humanized anti-LIGHT antibodies hum_LT-m37-5, hum_LT-m37-6, hum_LT-m37-7, hum_LT-m37-8, hum_LT-m37-11, and hum_LT-m37-12 blocking the binding of LIGHT to LTβR by ELISA. Figure 7C shows the activity of exemplary humanized anti-LIGHT antibodies hum_LT-m37-13, hum_LT-m37-14, hum_LT-m37-15, and hum_LT-m37-16 blocking the binding of LIGHT to LTβR by ELISA.

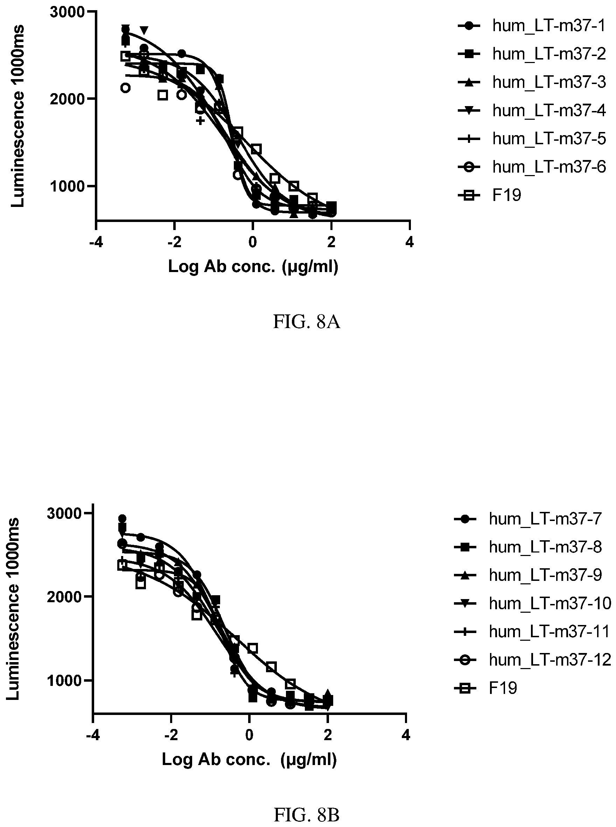

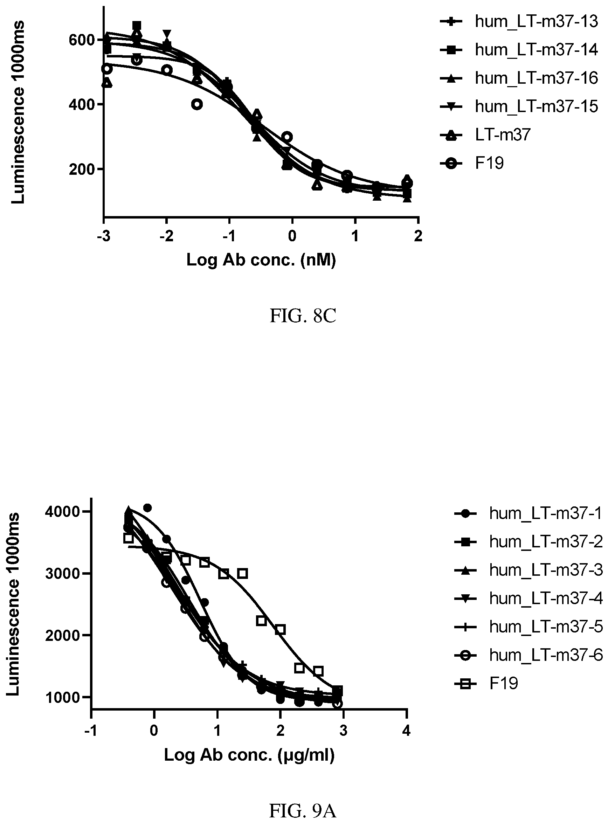

Figure 8A shows the inhibitory activity of exemplary humanized anti-LIGHT antibodies hum_LT-m37-1~hum_LT-m37-6 on the activation of the LTβR reporter gene by free LIGHT. Figure 8B shows the inhibitory activity of exemplary humanized anti-LIGHT antibodies hum_LT-m37-7~hum_LT-m37-12 on the activation of the LTβR reporter gene by free LIGHT. Figure 8C shows the inhibitory activity of exemplary humanized anti-LIGHT antibodies hum_LT-m37-13~hum_LT-m37-16 on the activation of the LTβR reporter gene by free LIGHT.

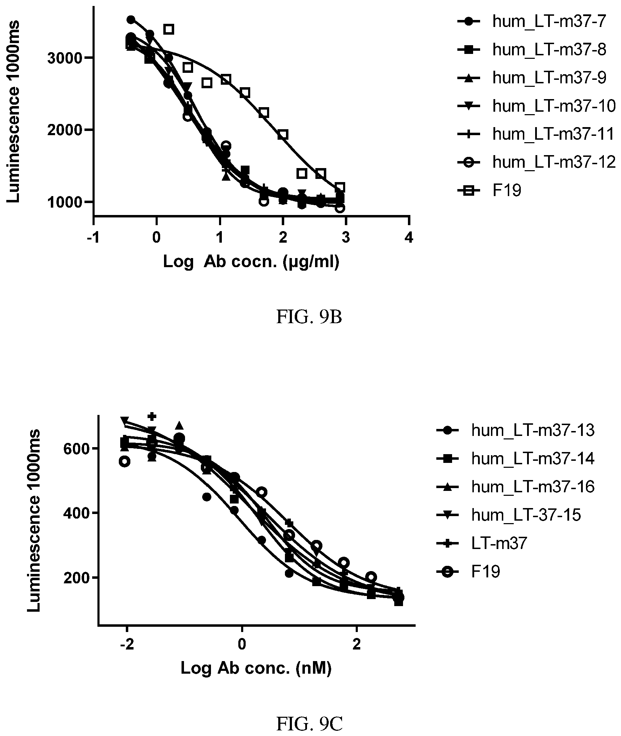

Figure 9A shows the inhibitory activity of exemplary humanized anti-LIGHT antibodies hum_LT-m37-1~hum_LT-m37-6 on the activation of the LTβR reporter gene by cellular LIGHT. Figure 9B shows the inhibitory activity of exemplary humanized anti-LIGHT antibodies hum_LT-m37-7~hum_LT-m37-12 on the activation of the LTβR reporter gene by cellular LIGHT. Figure 9C shows the inhibitory activity of exemplary humanized anti-LIGHT antibodies hum_LT-m37-13~hum_LT-m37-16 on the activation of the LTβR reporter gene by cellular LIGHT.

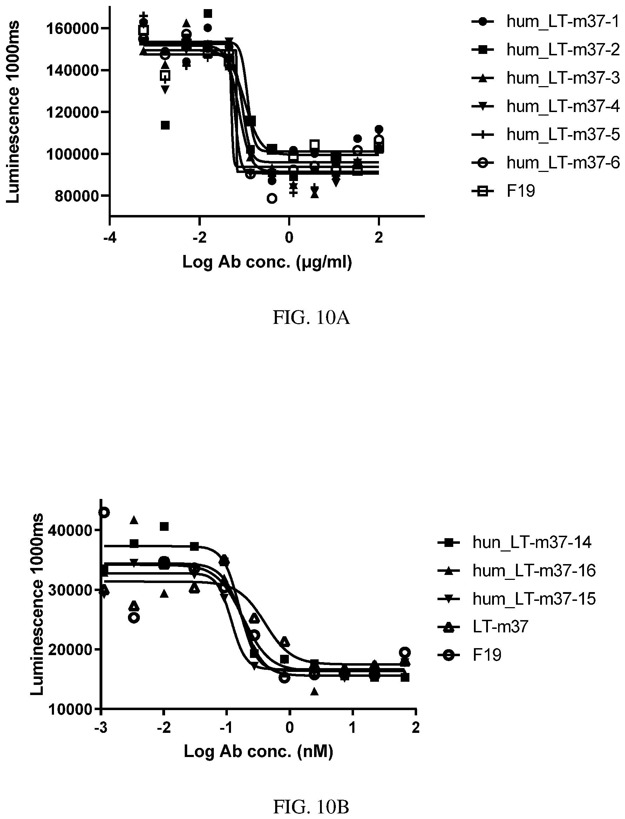

Figure 10A shows the inhibitory activity of exemplary humanized anti-LIGHT antibodies hum_LT-m37-1~hum_LT-m37-6 on the activation of the HVEM reporter gene by free LIGHT. Figure 10B shows the inhibitory activity of exemplary humanized anti-LIGHT antibodies hum_LT-m37-14, hum_LT-m37-15, and hum_LT-m37-16 on the activation of the HVEM reporter gene by free LIGHT.

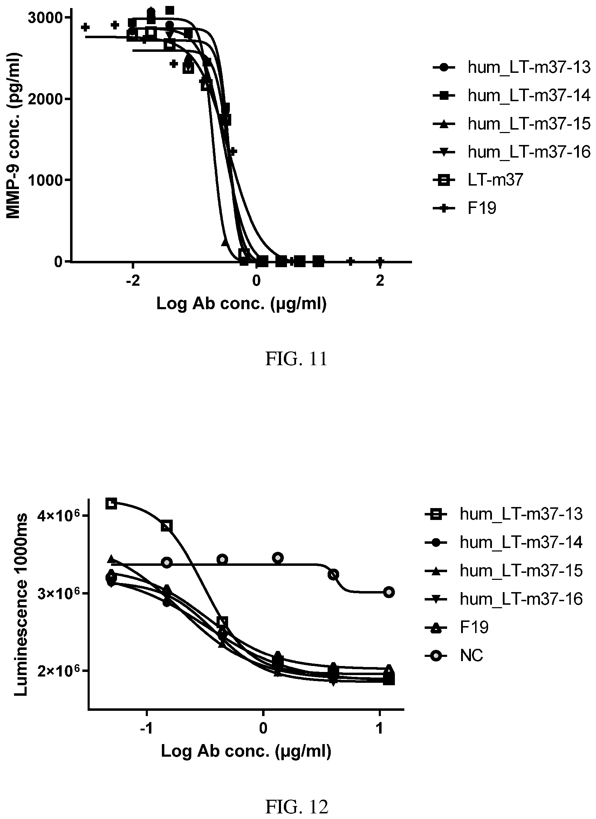

Figure 11 shows the inhibitory activity of exemplary humanized anti-LIGHT antibodies hum_LT-m37-13, hum_LT-m37-14, hum_LT-m37-15, and hum_LT-m37-16 on the production of MMP-9 in BEAS-2B bronchial epithelial cells induced by LIGHT.
Figure 12 shows the inhibitory activity of exemplary humanized anti-LIGHT antibodies hum_LT-m37-13, hum_LT-m37-14, hum_LT-m37-15, and hum_LT-m37-16 on the activation and proliferation of CD3+T cells stimulated by LIGHT.

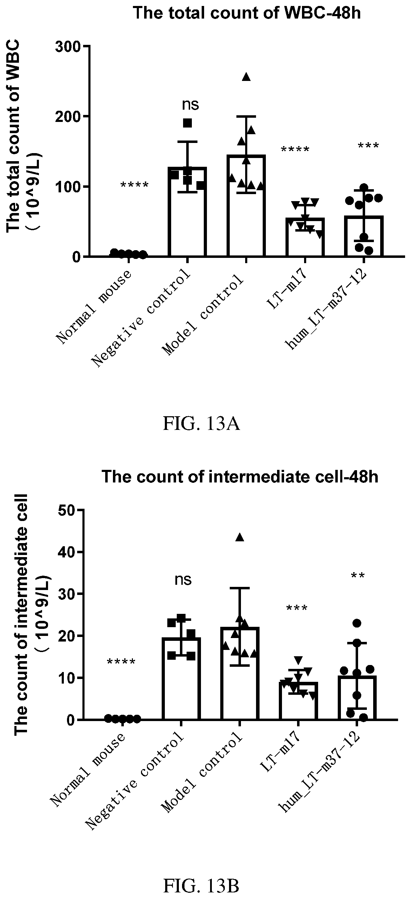

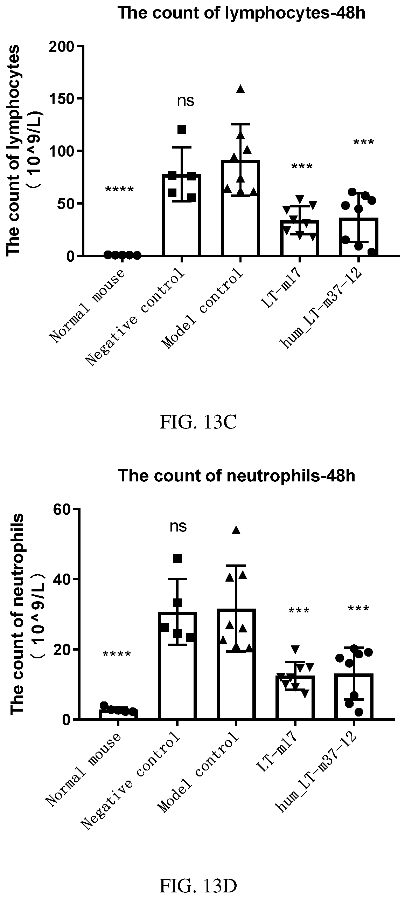

Figure 13 shows the inhibitory activity of an exemplary anti-LIGHT antibody at the leukocyte level in a LPS induced mouse ARDS model. Figure 13A shows the inhibitory activity of exemplary anti-LIGHT antibodies LT-m17, and hum_LT-m37-12 on the total leukocyte level in a LPS induced mouse ARDS model. Figure 13B shows the inhibitory activity of exemplary anti-LIGHT antibodies LT-m17, and hum_LT-m37-12 on the intermediate cell level in a LPS induced mouse ARDS model. Figure 13C shows the inhibitory activity of exemplary anti-LIGHT antibodies LT-m17, and hum_LT-m37-12 on the lymphocyte level in a LPS induced mouse ARDS model. Figure 13D shows the inhibitory activity of exemplary anti-LIGHT antibodies LT-m17, and hum_LT-m37-12 on the neutrophil level in a LPS induced mouse ARDS model.

### DETAILED DESCRIPTION OF THE APPLICATION

The present application in one aspect provides an isolated anti-LIGHT antibody. By using a combination of selections on naïve scFv phage libraries, affinity maturation and appropriately designed biochemical and biological assays, we have identified highly potent antibody molecules that bind to human LIGHT and inhibit the action of human LIGHT to its receptor. The results presented herein indicate that the present application antibodies bind LIGHT with different regions or epitopes, compared with the known anti-LIGHT antibody F19 (also known as CERC-002, Quisovalimab, Avalo Therapeutics), and surprisingly are even more potent than F19 as demonstrated in a variety of biological assays.

The anti-LIGHT antibodies provided by the present application include, for example, full-length anti-LIGHT antibodies, anti-LIGHT scFvs, anti-LIGHT Fc fusion proteins, multispecific (such as bispecific) anti-LIGHT antibodies, anti-LIGHT immunoconjugates, and the like.

In another aspect, the present application provides an isolated anti-LIGHT antibody comprises: a heavy chain variable domain (V_{H}) comprising a heavy chain complementarity determining region (HC-CDR) 1 comprising GYFIN (SEQ ID NO: 1); an HC-CDR2 comprising RIYPYNVX₁TFYNQNFKG (SEQ ID NO: 45), wherein X₁ is D or N; and an HC-CDR3 comprising GTHYYGSSGAMDY (SEQ ID NO: 16); and a light chain variable domain (V_{L}) comprising a light chain complementarity determining region (LC-CDR) 1 comprising KASQNVGTAVA (SEQ ID NO: 23); an LC-CDR2 comprising SASNRYT (SEQ ID NO: 30); and an LC-CDR3 comprising QQYSSYPYT (SEQ ID NO: 37).

In another aspect, the present application provides an isolated anti-LIGHT antibody comprises: a heavy chain variable domain (V_{H}) comprising a heavy chain complementarity determining region (HC-CDR) 1 comprising GYFMN (SEQ ID NO: 2); an HC-CDR2 comprising RIYPYSGDTFYNQKFNN (SEQ ID NO: 10); and an HC-CDR3 comprising SEHFGRNYGTGAVDY (SEQ ID NO: 17); and a light chain variable domain (V_{L}) comprising a light chain complementarity determining region (LC-CDR) 1 comprising RSSQGLVHSNGNTYLH (SEQ ID NO: 24); an LC-CDR2 comprising KVSNRFS (SEQ ID NO: 31); and an LC-CDR3 comprising SQSTHVPYT (SEQ ID NO: 38).

In another aspect, the present application provides an isolated anti-LIGHT antibody comprises: a heavy chain variable domain (V_{H}) comprising a heavy chain complementarity determining region (HC-CDR) 1 comprising GYFMN (SEQ ID NO: 2); an HC-CDR2 comprising RIYPYSGDTFYNQKFNN (SEQ ID NO: 10); and an HC-CDR3 comprising SEHFGRNYGTGAVDY (SEQ ID NO: 17); and a light chain variable domain (V_{L}) comprising a light chain complementarity determining region (LC-CDR) 1 comprising RSSQSLVHSNGNTYLH (SEQ ID NO: 25); an LC-CDR2 comprising KVSNRFS (SEQ ID NO: 31); and an LC-CDR3 comprising SQSTHVPLT (SEQ ID NO: 39).

In another aspect, the present application provides an isolated anti-LIGHT antibody comprises: a heavy chain variable domain (V_{H}) comprising a heavy chain complementarity determining region (HC-CDR) 1 comprising NYYVY (SEQ ID NO: 3); an HC-CDR2 comprising EINPTNGDPNFNEKFKS (SEQ ID NO: 11); and an HC-CDR3 comprising SNWDYGNAMDF (SEQ ID NO: 18); and a light chain variable domain (V_{L}) comprising a light chain complementarity determining region (LC-CDR) 1 comprising KASQSVDFDGDGYMN (SEQ ID NO: 26); an LC-CDR2 comprising KASHLDS (SEQ ID NO: 32); and an LC-CDR3 comprising QQSIEDPLT (SEQ ID NO: 40).

In another aspect, the present application provides an isolated anti-LIGHT antibody comprises: a heavy chain variable domain (V_{H}) comprising a heavy chain complementarity determining region (HC-CDR) 1 comprising NYYVY (SEQ ID NO: 3); an HC-CDR2 comprising EINPTNGDPNFNEKFKS (SEQ ID NO: 11); and an HC-CDR3 comprising SNWDYGNAMDF (SEQ ID NO: 18); and a light chain variable domain (V_{L}) comprising a light chain complementarity determining region (LC-CDR) 1 comprising KASQSVDFDGDGYMN (SEQ ID NO: 26); an LC-CDR2 comprising KASHLDS (SEQ ID NO: 32); and an LC-CDR3 comprising QQSIEDPFT (SEQ ID NO: 41).

In another aspect, the present application provides an isolated anti-LIGHT antibody comprises: a heavy chain variable domain (V_{H}) comprising a heavy chain complementarity determining region (HC-CDR) 1 comprising NYGMN (SEQ ID NO: 4); an HC-CDR2 comprising WINTYTGEPTY ADDFKG (SEQ ID NO: 12); and an HC-CDR3 comprising DNWDGKGMDY (SEQ ID NO: 19); and a light chain variable domain (V_{L}) comprising a light chain complementarity determining region (LC-CDR) 1 comprising KASQDVDTAVA (SEQ ID NO: 27); an LC-CDR2 comprising WASTRHT (SEQ ID NO: 33); and an LC-CDR3 comprising HQYGSYPLT (SEQ ID NO: 42).

In another aspect, the present application provides an isolated anti-LIGHT antibody comprises: a heavy chain variable domain (V_{H}) comprising a heavy chain complementarity determining region (HC-CDR) 1 comprising TYYMY (SEQ ID NO: 5); an HC-CDR2 comprising EINPTNGGTIFNEKFKT (SEQ ID NO: 13); and an HC-CDR3 comprising GNWPYWSFDV (SEQ ID NO: 20); and a light chain variable domain (V_{L}) comprising a light chain complementarity determining region (LC-CDR) 1 comprising KASQSVDFDGDNYMN (SEQ ID NO: 28); an LC-CDR2 comprising AASNLDS (SEQ ID NO: 34); and an LC-CDR3 comprising QQSIEDPWT (SEQ ID NO: 43).

In another aspect, the present application provides an isolated anti-LIGHT antibody comprises: a heavy chain variable domain (V_{H}) comprising a heavy chain complementarity determining region (HC-CDR) 1 comprising DHIMN (SEQ ID NO: 6); an HC-CDR2 comprising RIYPVSGETNYNQKFMG (SEQ ID NO: 14); and an HC-CDR3 comprising GSYYWNAMDY (SEQ ID NO: 21); and a light chain variable domain (V_{L}) comprising a light chain complementarity determining region (LC-CDR) 1 comprising KASQSVDFDGDSYMN (SEQ ID NO: 29); an LC-CDR2 comprising SASNLES (SEQ ID NO: 35); and an LC-CDR3 comprising QQSIEDPWT (SEQ ID NO: 43).

In another aspect, the present application provides an isolated anti-LIGHT antibody comprises: a heavy chain variable domain (V_{H}) comprising a heavy chain complementarity determining region (HC-CDR) 1 comprising SYNVH (SEQ ID NO: 7); an HC-CDR2 comprising AVYPGNGDTSYNQKFKG (SEQ ID NO: 15); and an HC-CDR3 comprising GSYYYTSSYFDH (SEQ ID NO: 22); and a light chain variable domain (V_{L}) comprising a light chain complementarity determining region (LC-CDR) 1 comprising KASQSVDFDGDSYMN (SEQ ID NO: 29); an LC-CDR2 comprising TASNLES (SEQ ID NO: 36); and an LC-CDR3 comprising QQSYEDPFT (SEQ ID NO: 44).

Also provided are nucleic acids encoding the anti-LIGHT antibodies, compositions comprising the anti-LIGHT antibodies, and methods of making and using the anti-LIGHT antibodies.

### Definitions

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread (e.g., metastasis) of the disease, preventing or delaying the recurrence of the disease, delaying or slowing the progression of the disease, ameliorating the disease state, providing a remission (partial or total) of the disease, decreasing the dose of one or more of other medications required to treat the disease, delaying the progression of the disease, increasing or improving the quality of life, increasing weight gain, and/or prolonging survival. Also encompassed by "treatment" is a reduction of pathological consequence of the disease (such as, for example, forced expiratory volume for asthma). The methods of the application contemplate any one or more of these aspects of treatment.

The term "antibody" includes full-length antibodies and antigen-binding fragments thereof. A full-length antibody comprises two heavy chains and two light chains. The variable regions of the light and heavy chains are responsible for antigen binding. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain).

The term "antigen-binding fragment" as used herein includes an antibody fragment including, for example, a diabody, a Fab, a Fab', a F(ab')₂, an Fv fragment, a disulfide stabilized Fv fragment (dsFv), a (dsFv)₂, a bispecific dsFv (dsFv-dsFv'), a disulfide stabilized diabody (ds diabody), a single-chain Fv (scFv), an scFv dimer (bivalent diabody), a multispecific antibody formed from a portion of an antibody comprising one or more CDRs, a single domain antibody, a nanobody, a domain antibody, a bivalent domain antibody, or any other antibody fragments that bind to an antigen but do not comprise a complete antibody structure. An antigen-binding fragment also includes a fusion protein comprising the antibody fragment described above. An antigen-binding fragment is capable of binding to the same antigen to which the parent antibody or a parent antibody fragment (e.g., a parent scFv) binds. In some embodiments, an antigen-binding fragment may comprise one or more CDRs from a particular human antibody grafted to a framework region from one or more different human antibodies.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

As used herein, a first antibody "competes" for binding to a target LIGHT with a second antibody when the first antibody inhibits target LIGHT binding of the second antibody by at least about 50% (such as at least about any of 55%, 60%, 65%, 70%, 75%, 80%, 85%, 90%, 95%, 98% or 99%) in the presence of an equimolar concentration of the first antibody, or *vice versa.* A high throughput process for "binning" antibodies based upon their cross-competition is described in PCT Publication No. WO 03/48731.

As used herein, the term "specifically binds", "specifically recognizing", or "is specific for" refers to measurable and reproducible interactions, such as binding between a target and an antibody that is determinative of the presence of the target in the presence of a heterogeneous population of molecules, including biological molecules. For example, an antibody that specifically recognizes a target (which can be an epitope) is an antibody that binds to this target with greater affinity, avidity, more readily, and/or with greater duration than its binding to other targets. In some embodiments, an antibody that specifically recognizes an antigen reacts with one or more antigenic determinants of the antigen with a binding affinity that is at least about 10 times its binding affinity for other targets.

An "isolated" anti-LIGHT antibody as used herein refers to an anti-LIGHT antibody that (1) is not associated with proteins found in nature, (2) is free of other proteins from the same source, (3) is expressed by a cell from a different species, or, (4) does not occur in nature.

The term "isolated nucleic acid" as used herein is intended to mean a nucleic acid of genomic, cDNA, or synthetic origin or some combination thereof, which by virtue of its origin the "isolated nucleic acid" (1) is not associated with all or a portion of a polynucleotide in which the "isolated nucleic acid" is found in nature, (2) is operably linked to a polynucleotide which it is not linked to in nature, or (3) does not occur in nature as part of a larger sequence.

As used herein, the term "CDR" or "complementarity determining region" is intended to mean the non-contiguous antigen combining sites found within the variable region of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**TABLE 1: CDR DEFINITIONS**

| | **Kabat¹** | **Chothia²** | **MacCallum³** | **IMGT⁴** | **AHo⁵** |
|---|---|---|---|---|---|
| V_{H} CDR1 | 31-35 | 26-32 | 30-35 | 27-38 | 25-40 |
| V_{H} CDR2 | 50-65 | 53-55 | 47-58 | 56-65 | 58-77 |
| V_{H} CDR3 | 95-102 | 96-101 | 93-101 | 105-117 | 109-137 |
| V_{L} CDR1 | 24-34 | 26-32 | 30-36 | 27-38 | 25-40 |
| V_{L} CDR2 | 50-56 | 50-52 | 46-55 | 56-65 | 58-77 |
| V_{L} CDR3 | 89-97 | 91-96 | 89-96 | 105-117 | 109-137 |

| | | | | | |
|---|---|---|---|---|---|
| ¹Residue numbering follows the nomenclature of Kabat *et al., supra* ²Residue numbering follows the nomenclature of Chothia *et al., supra* ³Residue numbering follows the nomenclature of MacCallum *et al., supra* ⁴Residue numbering follows the nomenclature of Lefranc *et al., supra* ⁵Residue numbering follows the nomenclature of Honegger and Plückthun, *supra* | | | | | |

The term "chimeric antibody" refers to an antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit a biological activity of this application (*see* U.S. Patent No. 4,816,567; and Morrison et al., Proc. Natl. Acad. Sci. USA, 81:6851-6855 (1984)).

"Fv" is the minimum antibody fragment which contains a complete antigen-recognition and binding site. This fragment consists of a dimer of one heavy- and one light-chain variable region domain in tight, non-covalent association. From the folding of these two domains emanate six hypervariable loops (3 loops each from the heavy and light chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv", also abbreviated as "sFv" or "scFv", are antibody fragments that comprise the V_{H} and V_{L} antibody domains connected into a single polypeptide chain. In some embodiments, the scFv polypeptide further comprises a polypeptide linker between the V_{H} and V_{L} domains which enables the scFv to form the desired structure for antigen binding. For a review of scFv, *see* Pluckthun in The Pharmacology of Monoclonal Antibodies, vol. 113, Rosenburg and Moore eds., Springer-Verlag, New York, pp. 269-315 (1994).

The term "diabodies" refers to small antibody fragments prepared by constructing scFv fragments (see preceding paragraph) typically with short linkers (such as about 5 to about 10 residues) between the V_{H} and V_{L} domains such that inter-chain but not intra-chain pairing of the V domains is achieved, resulting in a bivalent fragment, *i.e.,* fragment having two antigen-binding sites. Bispecific diabodies are heterodimers of two "crossover" scFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (e.g., rodent) antibodies are chimeric antibodies that contain minimal sequence derived from the non-human antibody. For the most part, humanized antibodies are human immunoglobulins (recipient antibody) in which residues from a hypervariable region (HVR) of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skilled in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1):113, 2004).

The terms "Fc receptor" or "FcR" are used to describe a receptor that binds to the Fc region of an antibody. In some embodiments, an FcR of this application is one that binds to an IgG antibody (a γ receptor) and includes receptors of the FcγRI, FcγRII, and FcγRIII subclasses, including allelic variants and alternatively spliced forms of these receptors. FcγRII receptors include FcγRIIA (an "activating receptor") and FcyRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review M. in Daëron, Annu. Rev. Immunol. 15:203-234 (1997)). The term includes allotypes, such as FcγRIIIA allotypes: FcγRIIIA-Phe158, FcγRIIIA-Val158, FcγRIIA-R131 and/or FcγRIIA-H131. FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The term "FcRn" refers to the neonatal Fc receptor (FcRn). FcRn is structurally similar to major histocompatibility complex (MHC) and consists of an α-chain noncovalently bound to β2-microglobulin. The multiple functions of the neonatal Fc receptor FcRn are reviewed in Ghetie and Ward (2000) Annu. Rev. Immunol. 18, 739-766. FcRn plays a role in the passive delivery of immunoglobulin IgGs from mother to young and the regulation of serum IgG levels. FcRn can act as a salvage receptor, binding and transporting pinocytosed IgGs in intact form both within and across cells, and rescuing them from a default degradative pathway.

The "CH1 domain" of a human IgG Fc region usually extends from about amino acid 118 to about amino acid 215 (EU numbering system).

"Hinge region" is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec Immunol. 22:161-206 (1985).

The "CH3 domain" comprises the stretch of residues of C-terminal to a CH2 domain in an Fc region (*i.e.* from about amino acid residue 341 to the C-terminal end of an antibody sequence, typically at amino acid residue 446 or 447 of an IgG).

A "functional Fc fragment" possesses an "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (*e.g.,* B cell receptor; BCR), etc. Such effector functions generally require the Fc region to be combined with a binding domain (*e.g*., an antibody variable domain) and can be assessed using various assays known in the art.

An antibody with a variant IgG Fc with "altered" FcR binding affinity or ADCC activity is one which has either enhanced or diminished FcR binding activity (*e.g*., FcγR or FcRn) and/or ADCC activity compared to a parent polypeptide or to a polypeptide comprising a native sequence Fc region. The variant Fc which "exhibits increased binding" to an FcR binds at least one FcR with higher affinity (*e.g*., lower apparent Kd or IC₅₀ value) than the parent polypeptide or a native sequence IgG Fc. According to some embodiments, the improvement in binding compared to a parent polypeptide is about 3-fold, such as about any of 5, 10, 25, 50, 60, 100, 150, 200, or up to 500-fold, or about 25% to 1000% improvement in binding. The polypeptide variant which "exhibits decreased binding" to an FcR, binds at least one FcR with lower affinity (*e.g*., higher apparent Kd or IC₅₀ value) than a parent polypeptide. The decrease in binding compared to a parent polypeptide may be about 40% or more decrease in binding.

"Antibody-dependent cell-mediated cytotoxicity" or "ADCC" refers to a form of cytotoxicity in which secreted Ig bound to Fc receptors (FcRs) present on certain cytotoxic cells (*e.g.,* Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. PNAS (USA) 95:652-656 (1998).

The polypeptide comprising a variant Fc region which "exhibits increased ADCC" or mediates ADCC in the presence of human effector cells more effectively than a polypeptide having wild type IgG Fc or a parent polypeptide is one which *in vitro* or *in vivo* is substantially more effective at mediating ADCC, when the amounts of polypeptide with variant Fc region and the polypeptide with wild type Fc region (or the parent polypeptide) in the assay are essentially the same. Generally, such variants will be identified using any *in vitro* ADCC assay known in the art, such as assays or methods for determining ADCC activity, *e.g*., in an animal model etc. In some embodiments, the variant is from about 5-fold to about 100-fold, *e.g.* from about 25 to about 50-fold, more effective at mediating ADCC than the wild type Fc (or parent polypeptide).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed. Polypeptide variants with altered Fc region amino acid sequences and increased or decreased C1q binding capability are described in US patent No. 6,194,551B1 and WO99/51642. The contents of those patent publications are specifically incorporated herein by reference. *See* also, Idusogie et al. J. Immunol. 164: 4178-4184 (2000).

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

An "effective amount" of an anti-LIGHT antibody or composition as disclosed herein, is an amount sufficient to carry out a specifically stated purpose. An "effective amount" can be determined empirically and by known methods relating to the stated purpose.

The term "therapeutically effective amount" refers to an amount of an anti-LIGHT antibody or composition as disclosed herein, effective to "treat" a disease or disorder in an individual. In the case of asthma, "asthma related parameters" are used as indicators to evaluate the effect of asthma treatment, for example, "asthma related parameters" include: (a) forced expiratory volume within 1 second (FEV1); (b) peak expiratory flow (PEF), including morning PEF (AM PEF) and evening PEF (PM PEF); (c) use inhaled bronchodilators such as salbutamol or levosalbutamol; (d) five asthma control questionnaire (ACQ5) score; (d) nighttime awakening; and (e) 22 Sino Nasal result tests (SNOT-22) score. The therapeutic effective amount of anti-LIGHT antibody or combination disclosed in the article can increase one or more of FEV1, AM PEF, or PM PEF from baseline, and/or reduce one or more of daily salbutamol/levosalbutamol use, ACQ5 score, average nighttime awakening, or SNOT-22 score from baseline. As used in the article, the term "baseline" refers to the numerical values of asthma related parameters in patients before or during the administration of the pharmaceutical combination of the present invention. In some embodiments, improvement in asthma related parameters refers to an increase in FEV1 of at least 0.10L from baseline. In some embodiments, improvement in asthma related parameters refers to an increase in AM PEF from baseline of at least 10.0 L/min. In some embodiments, improvement in asthma related parameters refers to an increase in PM PEF from baseline of at least 1.0 L/min. In some embodiments, improvement in asthma related parameters refers to reducing the daily use of at least 1 puff (s) of salbutamol/levosalbutamol from baseline. In some embodiments, improvement in asthma related parameters refers to a decrease in ACQ5 score of at least 0.5 points from baseline. In some embodiments, improvement in asthma related parameters refers to a reduction in nighttime awakening of at least 0.2 from baseline. In some embodiments, improvement in asthma related parameters refers to a decrease in SNOT-22 of at least 5 points score from baseline. In some embodiments, the therapeutically effective amount is a growth inhibitory amount. In some embodiments, the therapeutically effective amount is an amount that extends the survival of a patient. In some embodiments, the therapeutically effective amount is an amount that improves progression free survival of a patient.

As used herein, by "pharmaceutically acceptable" or "pharmacologically compatible" is meant a material that is not biological or otherwise undesirable, *e.g*., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug Administration.

It is understood that embodiments of the application described herein include "consisting of" and/or "consisting essentially of" embodiments.

Reference to "about" a value or parameter herein includes (and describes) variations that are directed to that value or parameter per se. For example, description referring to "about X" includes description of "X".

As used herein, reference to "not" a value or parameter generally means and describes "other than" a value or parameter. For example, the method is not used to treat cancer of type X means the method is used to treat cancer of types other than X.

As used herein and in the appended claims, the singular forms "a", "an", and "the" include plural referents unless the context clearly dictates otherwise.

### Anti-LIGHT antibodies

In one aspect, the present application provides anti-LIGHT antibodies that specifically bind to human and/or cynomolgus monkey LIGHT. Anti-LIGHT antibodies include, but are not limited to, humanized antibodies, chimeric antibodies, mouse antibodies, human antibodies, and antibodies comprising the heavy chain and/or light chain CDRs discussed herein. In one aspect, the present application provides isolated antibodies that bind to LIGHT. Contemplated anti-LIGHT antibodies include, for example, full-length anti-LIGHT antibodies (*e.g*., full-length IgG1 or IgG4), anti-LIGHT scFvs, anti-LIGHT Fc fusion proteins, multispecific (such as bispecific) anti-LIGHT antibodies, anti-LIGHT immunoconjugates, and the like. In some embodiments, the anti-LIGHT antibody is a full-length antibody (*e.g*., full-length IgG1 or IgG4) or antigen-binding fragment thereof, which specifically binds to LIGHT. In some embodiments, the anti-LIGHT antibody is a Fab, a Fab', a F(ab)'₂, a Fab'-SH, a single-chain Fv (scFv), an Fv fragment, a dAb, a Fd,a nanobody, a diabody, or a linear antibody. In some embodiments, reference to an antibody that specifically binds to LIGHT means that the antibody binds to LIGHT with an affinity that is at least about 10 times (including for example at least about any one of 10, 10², 10³, 10⁴, 10⁵, 10⁶, or 10⁷ times) more tightly than its binding affinity for a non-target. In some embodiments, the non-target is an antigen that is not LIGHT. Binding affinity can be determined by methods known in the art, such as ELISA, fluorescence activated cell sorting (FACS) analysis, or radioimmunoprecipitation assay (RIA). Kd can be determined by methods known in the art, such as surface plasmon resonance (SPR) assay or biolayer interferometry (BLI).

Although anti-LIGHT antibodies containing human sequences (*e.g*., human heavy and light chain variable domain sequences comprising human CDR sequences) are extensively discussed herein, non-human anti-LIGHT antibodies are also contemplated. In some embodiments, non-human anti-LIGHT antibodies comprise human CDR sequences from an anti-LIGHT antibody as described herein and non-human framework sequences. Non-human framework sequences include, in some embodiments, any sequence that can be used for generating synthetic heavy and/or light chain variable domains using one or more human CDR sequences as described herein, including, *e.g.,* mammals, *e.g.,* mouse, rat, rabbit, pig, bovine (*e.g.,* cow, bull, buffalo), deer, sheep, goat, chicken, cat, dog, ferret, primate (*e.g.,* marmoset, rhesus monkey), *etc.* In some embodiments, a non-human anti-LIGHT antibody includes an anti-LIGHT antibody generated by grafting one or more human CDR sequences as described herein onto a non-human framework sequence (*e.g*., a mouse or chicken framework sequence).

The complete amino acid sequence of an exemplary human LIGHT comprises or consists of the amino acid sequence of SEQ ID NO: 78.

In some embodiments, the anti-LIGHT antibody described herein specifically recognizes an epitope within human LIGHT. In some embodiments, the anti-LIGHT antibody cross-reacts with LIGHT from species other than human. In some embodiments, the anti-LIGHT antibody is completely specific for human LIGHT and does not exhibit cross-reactivity with LIGHTs from other non-human species.

In some embodiments, the anti-LIGHT antibody cross-reacts with at least one allelic variant of the LIGHT protein (or fragments thereof). In some embodiments, the allelic variant has up to about 30 (such as about any of 1, 2, 3, 4, 5, 6, 7, 8, 9, 10, 15, 20, 25, or 30) amino acid substitutions (such as a conservative substitution) when compared to the naturally occurring LIGHT (or fragments thereof). In some embodiments, the anti-LIGHT antibody does not cross-react with any allelic variants of the LIGHT protein (or fragments thereof).

In some embodiments, the anti-LIGHT antibody cross-reacts with at least one interspecies variant of the LIGHT protein. In some embodiments, for example, the LIGHT protein (or fragments thereof) is human LIGHT and the interspecies variant of the LIGHT protein (or fragments thereof) is a cynomolgus monkey variant thereof. In some embodiments, the anti-LIGHT antibody does not cross-react with any interspecies variants of the LIGHT protein.

In some embodiments, according to any of the anti-LIGHT antibodies described herein, the anti-LIGHT antibody comprises an antibody heavy chain constant region and an antibody light chain constant region. In some embodiments, the anti-LIGHT antibody comprises an IgG1 heavy chain constant region. In some embodiments, the anti-LIGHT antibody comprises an IgG2 heavy chain constant region. In some embodiments, the anti-LIGHT antibody comprises an IgG3 heavy chain constant region. In some embodiments, the anti-LIGHT antibody comprises an IgG4 heavy chain constant region. In some embodiments, the heavy chain constant region comprises (including consisting of or consisting essentially of) the amino acid sequence of SEQ ID NO: 74. In some embodiments, the heavy chain constant region comprises (including consisting of or consisting essentially of) the amino acid sequence of SEQ ID NO: 75. In some embodiments, the anti-LIGHT antibody comprises a kappa light chain constant region. In some embodiments, the light chain constant region comprises (including consisting of or consisting essentially of) the amino acid sequence of SEQ ID NO: 76. In some embodiments, the anti-LIGHT antibody comprises a lambda light chain constant region. In some embodiments, the light chain constant region comprises (including consisting of or consisting essentially of) the amino acid sequence of SEQ ID NO: 77. In some embodiments, the anti-LIGHT antibody comprises an antibody heavy chain variable domain and an antibody light chain variable domain.

In some embodiments, the isolated anti-LIGHT antibody comprises: a heavy chain variable domain (V_{H}) comprising a heavy chain complementarity determining region (HC-CDR) 1 comprising GYFIN (SEQ ID NO: 1); an HC-CDR2 comprising RIYPYNVX₁TFYNQNFKG (SEQ ID NO: 45), wherein X₁ is D or N; and an HC-CDR3 comprising GTHYYGSSGAMDY (SEQ ID NO: 16); and a light chain variable domain (V_{L}) comprising a light chain complementarity determining region (LC-CDR) 1 comprising KASQNVGTAVA (SEQ ID NO: 23); an LC-CDR2 comprising SASNRYT (SEQ ID NO: 30); and an LC-CDR3 comprising QQYSSYPYT (SEQ ID NO: 37)

In some embodiments, the anti-LIGHT antibody comprises a V_{H} comprising: an HC-CDR1 comprising the amino acid sequence of SEQ ID NO: 1, or a variant thereof comprising up to about 3 (such as about any of 1, 2, or 3) amino acid substitutions, an HC-CDR2 comprising the amino acid sequence of any one of SEQ ID NOs: 8-9, or a variant thereof comprising up to about 3 (such as about any of 1, 2, or 3) amino acid substitutions, and an HC-CDR3 comprising the amino acid sequence of SEQ ID NO: 16, or a variant thereof comprising up to about 3 (such as about any of 1, 2, or 3) amino acid substitutions.

In some embodiments, the anti-LIGHT antibody comprises a V_{H} comprising: an HC-CDR1 comprising the amino acid sequence of SEQ ID NO: 1, an HC-CDR2 comprising the amino acid sequence of any one of SEQ ID NOs: 8-9, and an HC-CDR3 comprising the amino acid sequence of SEQ ID NO: 16.

In some embodiments, the anti-LIGHT antibody comprises a V_{L} comprising: an LC-CDR1 comprising the amino acid sequence of SEQ ID NO: 23, or a variant thereof comprising up to about 3 (such as about any of 1, 2, or 3) amino acid substitutions, an LC-CDR2 comprising the amino acid sequence of SEQ ID NO: 30, or a variant thereof comprising up to about 3 (such as about any of 1, 2, or 3) amino acid substitutions, and an LC-CDR3 comprising the amino acid sequence of SEQ ID NO: 37, or a variant thereof comprising up to about 3 (such as about any of 1, 2, or 3) amino acid substitutions.

In some embodiments, the anti-LIGHT antibody comprises a V_{L} comprising: an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37.

In some embodiments, the anti-LIGHT antibody comprises a V_{H} comprising: an HC-CDR1 comprising the amino acid sequence of SEQ ID NO: 1, or a variant thereof comprising up to about 3 (such as about any of 1, 2, or 3) amino acid substitutions, an HC-CDR2 comprising the amino acid sequence of any one of SEQ ID NOs: 8-9, or a variant thereof comprising up to about 3 (such as about any of 1, 2, or 3) amino acid substitutions, and an HC-CDR3 comprising the amino acid sequence of SEQ ID NO: 16, or a variant thereof comprising up to about 3 (such as about any of 1, 2, or 3) amino acid substitutions; and a V_{L} comprising: an LC-CDR1 comprising the amino acid sequence of SEQ ID NO: 23, or a variant thereof comprising up to about 3 (such as about any of 1, 2, or 3) amino acid substitutions, an LC-CDR2 comprising the amino acid sequence of SEQ ID NO: 30, or a variant thereof comprising up to about 3 (such as about any of 1, 2, or 3) amino acid substitutions, and an LC-CDR3 comprising the amino acid sequence of SEQ ID NO: 37, or a variant thereof comprising up to about 3 (such as about any of 1, 2, or 3) amino acid substitutions.

In some embodiments, the anti-LIGHT antibody comprises a V_{H} comprising: an HC-CDR1 comprising the amino acid sequence of SEQ ID NO: 1, an HC-CDR2 comprising the amino acid sequence of any one of SEQ ID NOs: 8-9, and an HC-CDR3 comprising the amino acid sequence of SEQ ID NO: 16; and a V_{L} comprising: an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37.

In some embodiments, the anti-LIGHT antibody comprises a V_{H} comprising: an HC-CDR1 comprising the amino acid sequence of SEQ ID NO: 1, an HC-CDR2 comprising the amino acid sequence of SEQ ID NO: 8, and an HC-CDR3 comprising the amino acid sequence of SEQ ID NO: 16, or a variant thereof comprising up to about 5 amino acid substitutions in the HC-CDRs; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 1, an HC-CDR2 comprising the amino acid sequence of SEQ ID NO: 8, and an HC-CDR3 comprising the amino acid sequence of SEQ ID NO: 16; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37.

In some embodiments, the anti-LIGHT antibody comprises a V_{H} comprising: an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 16, or a variant thereof comprising up to about 5 amino acid substitutions in the HC-CDRs; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 16; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37.

In some embodiments, the anti-LIGHT antibody comprises a V_{H} comprising an HC-CDR1, an HC-CDR2 and an HC-CDR3 of the V_{H} comprising the amino acid sequence of any one of SEQ ID NOs: 46-53; and a V_{L} comprising an LC-CDR1, an LC-CDR2, and an LC-CDR3 of the V_{L} comprising the amino acid sequence of any one of SEQ ID NOs: 62-65.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 46, and a V_{L} comprising an LC-CDR1, an LC-CDR2 and an LC-CDR3 of the V_{L} comprising the amino acid sequence of SEQ ID NO: 62.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 47, and a V_{L} comprising an LC-CDR1, an LC-CDR2 and an LC-CDR3 of the V_{L} comprising the amino acid sequence of SEQ ID NO: 63.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 48, and a V_{L} comprising an LC-CDR1, an LC-CDR2 and an LC-CDR3 of the V_{L} comprising the amino acid sequence of SEQ ID NO: 63.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 49, and a V_{L} comprising an LC-CDR1, an LC-CDR2 and an LC-CDR3 of the V_{L} comprising the amino acid sequence of SEQ ID NO: 63.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 50, and a V_{L} comprising an LC-CDR1, an LC-CDR2 and an LC-CDR3 of the V_{L} comprising the amino acid sequence of SEQ ID NO: 63.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 47, and a V_{L} comprising an LC-CDR1, an LC-CDR2 and an LC-CDR3 of the V_{L} comprising the amino acid sequence of SEQ ID NO: 64.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 48, and a V_{L} comprising an LC-CDR1, an LC-CDR2 and an LC-CDR3 of the V_{L} comprising the amino acid sequence of SEQ ID NO: 64.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 49, and a V_{L} comprising an LC-CDR1, an LC-CDR2 and an LC-CDR3 of the V_{L} comprising the amino acid sequence of SEQ ID NO: 64.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 50, and a V_{L} comprising an LC-CDR1, an LC-CDR2 and an LC-CDR3 of the V_{L} comprising the amino acid sequence of SEQ ID NO: 64.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 47, and a V_{L} comprising an LC-CDR1, an LC-CDR2 and an LC-CDR3 of the V_{L} comprising the amino acid sequence of SEQ ID NO: 65.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 48, and a V_{L} comprising an LC-CDR1, an LC-CDR2 and an LC-CDR3 of the V_{L} comprising the amino acid sequence of SEQ ID NO: 65.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 49, and a V_{L} comprising an LC-CDR1, an LC-CDR2 and an LC-CDR3 of the V_{L} comprising the amino acid sequence of SEQ ID NO: 65.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 50, and a V_{L} comprising an LC-CDR1, an LC-CDR2 and an LC-CDR3 of the V_{L} comprising the amino acid sequence of SEQ ID NO: 65.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 51, and a V_{L} comprising an LC-CDR1, an LC-CDR2 and an LC-CDR3 of the V_{L} comprising the amino acid sequence of SEQ ID NO: 63.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 52, and a V_{L} comprising an LC-CDR1, an LC-CDR2 and an LC-CDR3 of the V_{L} comprising the amino acid sequence of SEQ ID NO: 65.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 53, and a V_{L} comprising an LC-CDR1, an LC-CDR2 and an LC-CDR3 of the V_{L} comprising the amino acid sequence of SEQ ID NO: 65.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 51, and a V_{L} comprising an LC-CDR1, an LC-CDR2 and an LC-CDR3 of the V_{L} comprising the amino acid sequence of SEQ ID NO: 65.

In some embodiments, the anti-LIGHT antibody comprises a V_{H} comprising the amino acid sequence of any one of SEQ ID NOs: 46-53, or a variant thereof having at least about 80% (such as at least about any of 80%, 85%, 90%, 95%, 96%, 97%, 98%, or 99%) sequence identity to the amino acid sequence of any one of SEQ ID NOs: 46-53, and a V_{L} comprising the amino acid sequence of any one of SEQ ID NOs: 62-65, or a variant thereof having at least about 80% (such as at least about any of 80%, 85%, 90%, 95%, 96%, 97%, 98%, or 99%) sequence identity to the amino acid sequence of any one of SEQ ID NOs: 62-65. In some embodiments, the anti-LIGHT antibody comprises a V_{H} comprising the amino acid sequence of any one of SEQ ID NOs: 46-53, and a V_{L} comprising the amino acid sequence of any one of SEQ ID NOs: 62-65.

In some embodiments, the anti-LIGHT antibody comprises a V_{H} comprising the amino acid sequence of SEQ ID NO: 46, or a variant thereof having at least about 80% (such as at least about any of 80%, 85%, 90%, 95%, 96%, 97%, 98%, or 99%) sequence identity to the amino acid sequence of SEQ ID NO: 46, and a V_{L} comprising the amino acid sequence of SEQ ID NO: 62, or a variant thereof having at least about 80% (such as at least about any of 80%, 85%, 90%, 95%, 96%, 97%, 98%, or 99%) sequence identity to the amino acid sequence of SEQ ID NO: 62. In some embodiments, the anti-LIGHT antibody comprises a V_{H} comprising the amino acid sequence of SEQ ID NO: 46 and a V_{L} comprising the amino acid sequence of SEQ ID NO: 62.

In some embodiments, the anti-LIGHT antibody comprises a V_{H} comprising the amino acid sequence of SEQ ID NO: 47, or a variant thereof having at least about 80% (such as at least about any of 80%, 85%, 90%, 95%, 96%, 97%, 98%, or 99%) sequence identity to the amino acid sequence of SEQ ID NO: 47, and a V_{L} comprising the amino acid sequence of SEQ ID NO: 63, or a variant thereof having at least about 80% (such as at least about any of 80%, 85%, 90%, 95%, 96%, 97%, 98%, or 99%) sequence identity to the amino acid sequence of SEQ ID NO: 63. In some embodiments, the anti-LIGHT antibody comprises a V_{H} comprising the amino acid sequence of SEQ ID NO: 47 and a V_{L} comprising the amino acid sequence of SEQ ID NO: 63.

In some embodiments, the anti-LIGHT antibody comprises a V_{H} comprising the amino acid sequence of SEQ ID NO: 48, or a variant thereof having at least about 80% (such as at least about any of 80%, 85%, 90%, 95%, 96%, 97%, 98%, or 99%) sequence identity to the amino acid sequence of SEQ ID NO: 48, and a V_{L} comprising the amino acid sequence of SEQ ID NO: 63, or a variant thereof having at least about 80% (such as at least about any of 80%, 85%, 90%, 95%, 96%, 97%, 98%, or 99%) sequence identity to the amino acid sequence of SEQ ID NO: 63. In some embodiments, the anti-LIGHT antibody comprises a V_{H} comprising the amino acid sequence of SEQ ID NO: 48 and a V_{L} comprising the amino acid sequence of SEQ ID NO: 63.

In some embodiments, the anti-LIGHT antibody comprises a V_{H} comprising the amino acid sequence of SEQ ID NO: 49, or a variant thereof having at least about 80% (such as at least about any of 80%, 85%, 90%, 95%, 96%, 97%, 98%, or 99%) sequence identity to the amino acid sequence of SEQ ID NO: 49, and a V_{L} comprising the amino acid sequence of SEQ ID NO: 63, or a variant thereof having at least about 80% (such as at least about any of 80%, 85%, 90%, 95%, 96%, 97%, 98%, or 99%) sequence identity to the amino acid sequence of SEQ ID NO: 63. In some embodiments, the anti-LIGHT antibody comprises a V_{H} comprising the amino acid sequence of SEQ ID NO: 49 and a V_{L} comprising the amino acid sequence of SEQ ID NO: 63.

In some embodiments, the anti-LIGHT antibody comprises a V_{H} comprising the amino acid sequence of SEQ ID NO: 50, or a variant thereof having at least about 80% (such as at least about any of 80%, 85%, 90%, 95%, 96%, 97%, 98%, or 99%) sequence identity to the amino acid sequence of SEQ ID NO: 50, and a V_{L} comprising the amino acid sequence of SEQ ID NO: 63, or a variant thereof having at least about 80% (such as at least about any of 80%, 85%, 90%, 95%, 96%, 97%, 98%, or 99%) sequence identity to the amino acid sequence of SEQ ID NO: 63. In some embodiments, the anti-LIGHT antibody comprises a V_{H} comprising the amino acid sequence of SEQ ID NO: 50 and a V_{L} comprising the amino acid sequence of SEQ ID NO: 63.

In some embodiments, the anti-LIGHT antibody comprises a V_{H} comprising the amino acid sequence of SEQ ID NO: 47, or a variant thereof having at least about 80% (such as at least about any of 80%, 85%, 90%, 95%, 96%, 97%, 98%, or 99%) sequence identity to the amino acid sequence of SEQ ID NO: 47, and a V_{L} comprising the amino acid sequence of SEQ ID NO: 64, or a variant thereof having at least about 80% (such as at least about any of 80%, 85%, 90%, 95%, 96%, 97%, 98%, or 99%) sequence identity to the amino acid sequence of SEQ ID NO: 64. In some embodiments, the anti-LIGHT antibody comprises a V_{H} comprising the amino acid sequence of SEQ ID NO: 47 and a V_{L} comprising the amino acid sequence of SEQ ID NO: 64.

In some embodiments, the anti-LIGHT antibody comprises a V_{H} comprising the amino acid sequence of SEQ ID NO: 48, or a variant thereof having at least about 80% (such as at least about any of 80%, 85%, 90%, 95%, 96%, 97%, 98%, or 99%) sequence identity to the amino acid sequence of SEQ ID NO: 48, and a V_{L} comprising the amino acid sequence of SEQ ID NO: 64, or a variant thereof having at least about 80% (such as at least about any of 80%, 85%, 90%, 95%, 96%, 97%, 98%, or 99%) sequence identity to the amino acid sequence of SEQ ID NO: 64. In some embodiments, the anti-LIGHT antibody comprises a V_{H} comprising the amino acid sequence of SEQ ID NO: 48 and a V_{L} comprising the amino acid sequence of SEQ ID NO: 64.

In some embodiments, the anti-LIGHT antibody comprises a V_{H} comprising the amino acid sequence of SEQ ID NO: 49, or a variant thereof having at least about 80% (such as at least about any of 80%, 85%, 90%, 95%, 96%, 97%, 98%, or 99%) sequence identity to the amino acid sequence of SEQ ID NO: 49, and a V_{L} comprising the amino acid sequence of SEQ ID NO: 64, or a variant thereof having at least about 80% (such as at least about any of 80%, 85%, 90%, 95%, 96%, 97%, 98%, or 99%) sequence identity to the amino acid sequence of SEQ ID NO: 64. In some embodiments, the anti-LIGHT antibody comprises a V_{H} comprising the amino acid sequence of SEQ ID NO: 49 and a V_{L} comprising the amino acid sequence of SEQ ID NO: 64.

In some embodiments, the anti-LIGHT antibody comprises a V_{H} comprising the amino acid sequence of SEQ ID NO: 50, or a variant thereof having at least about 80% (such as at least about any of 80%, 85%, 90%, 95%, 96%, 97%, 98%, or 99%) sequence identity to the amino acid sequence of SEQ ID NO: 50, and a V_{L} comprising the amino acid sequence of SEQ ID NO: 64, or a variant thereof having at least about 80% (such as at least about any of 80%, 85%, 90%, 95%, 96%, 97%, 98%, or 99%) sequence identity to the amino acid sequence of SEQ ID NO: 64. In some embodiments, the anti-LIGHT antibody comprises a V_{H} comprising the amino acid sequence of SEQ ID NO: 50 and a V_{L} comprising the amino acid sequence of SEQ ID NO: 64.

In some embodiments, the anti-LIGHT antibody comprises a V_{H} comprising the amino acid sequence of SEQ ID NO: 47, or a variant thereof having at least about 80% (such as at least about any of 80%, 85%, 90%, 95%, 96%, 97%, 98%, or 99%) sequence identity to the amino acid sequence of SEQ ID NO: 47, and a V_{L} comprising the amino acid sequence of SEQ ID NO: 65, or a variant thereof having at least about 80% (such as at least about any of 80%, 85%, 90%, 95%, 96%, 97%, 98%, or 99%) sequence identity to the amino acid sequence of SEQ ID NO: 65. In some embodiments, the anti-LIGHT antibody comprises a V_{H} comprising the amino acid sequence of SEQ ID NO: 47 and a V_{L} comprising the amino acid sequence of SEQ ID NO: 65.

In some embodiments, the anti-LIGHT antibody comprises a V_{H} comprising the amino acid sequence of SEQ ID NO: 48, or a variant thereof having at least about 80% (such as at least about any of 80%, 85%, 90%, 95%, 96%, 97%, 98%, or 99%) sequence identity to the amino acid sequence of SEQ ID NO: 48, and a V_{L} comprising the amino acid sequence of SEQ ID NO: 65, or a variant thereof having at least about 80% (such as at least about any of 80%, 85%, 90%, 95%, 96%, 97%, 98%, or 99%) sequence identity to the amino acid sequence of SEQ ID NO: 65. In some embodiments, the anti-LIGHT antibody comprises a V_{H} comprising the amino acid sequence of SEQ ID NO: 48 and a V_{L} comprising the amino acid sequence of SEQ ID NO: 65.

In some embodiments, the anti-LIGHT antibody comprises a V_{H} comprising the amino acid sequence of SEQ ID NO: 49, or a variant thereof having at least about 80% (such as at least about any of 80%, 85%, 90%, 95%, 96%, 97%, 98%, or 99%) sequence identity to the amino acid sequence of SEQ ID NO: 49, and a V_{L} comprising the amino acid sequence of SEQ ID NO: 65, or a variant thereof having at least about 80% (such as at least about any of 80%, 85%, 90%, 95%, 96%, 97%, 98%, or 99%) sequence identity to the amino acid sequence of SEQ ID NO: 65. In some embodiments, the anti-LIGHT antibody comprises a V_{H} comprising the amino acid sequence of SEQ ID NO: 49 and a V_{L} comprising the amino acid sequence of SEQ ID NO: 65.

In some embodiments, the anti-LIGHT antibody comprises a V_{H} comprising the amino acid sequence of SEQ ID NO: 50, or a variant thereof having at least about 80% (such as at least about any of 80%, 85%, 90%, 95%, 96%, 97%, 98%, or 99%) sequence identity to the amino acid sequence of SEQ ID NO: 50, and a V_{L} comprising the amino acid sequence of SEQ ID NO: 65, or a variant thereof having at least about 80% (such as at least about any of 80%, 85%, 90%, 95%, 96%, 97%, 98%, or 99%) sequence identity to the amino acid sequence of SEQ ID NO: 65. In some embodiments, the anti-LIGHT antibody comprises a V_{H} comprising the amino acid sequence of SEQ ID NO: 50 and a V_{L} comprising the amino acid sequence of SEQ ID NO: 65.

In some embodiments, the anti-LIGHT antibody comprises a V_{H} comprising the amino acid sequence of SEQ ID NO: 51, or a variant thereof having at least about 80% (such as at least about any of 80%, 85%, 90%, 95%, 96%, 97%, 98%, or 99%) sequence identity to the amino acid sequence of SEQ ID NO: 51, and a V_{L} comprising the amino acid sequence of SEQ ID NO: 63, or a variant thereof having at least about 80% (such as at least about any of 80%, 85%, 90%, 95%, 96%, 97%, 98%, or 99%) sequence identity to the amino acid sequence of SEQ ID NO: 63. In some embodiments, the anti-LIGHT antibody comprises a V_{H} comprising the amino acid sequence of SEQ ID NO: 51 and a V_{L} comprising the amino acid sequence of SEQ ID NO: 63.

In some embodiments, the anti-LIGHT antibody comprises a V_{H} comprising the amino acid sequence of SEQ ID NO: 52, or a variant thereof having at least about 80% (such as at least about any of 80%, 85%, 90%, 95%, 96%, 97%, 98%, or 99%) sequence identity to the amino acid sequence of SEQ ID NO: 52, and a V_{L} comprising the amino acid sequence of SEQ ID NO: 65, or a variant thereof having at least about 80% (such as at least about any of 80%, 85%, 90%, 95%, 96%, 97%, 98%, or 99%) sequence identity to the amino acid sequence of SEQ ID NO: 65. In some embodiments, the anti-LIGHT antibody comprises a V_{H} comprising the amino acid sequence of SEQ ID NO: 52 and a V_{L} comprising the amino acid sequence of SEQ ID NO: 65.

In some embodiments, the anti-LIGHT antibody comprises a V_{H} comprising the amino acid sequence of SEQ ID NO: 53, or a variant thereof having at least about 80% (such as at least about any of 80%, 85%, 90%, 95%, 96%, 97%, 98%, or 99%) sequence identity to the amino acid sequence of SEQ ID NO: 53, and a V_{L} comprising the amino acid sequence of SEQ ID NO: 65, or a variant thereof having at least about 80% (such as at least about any of 80%, 85%, 90%, 95%, 96%, 97%, 98%, or 99%) sequence identity to the amino acid sequence of SEQ ID NO: 65. In some embodiments, the anti-LIGHT antibody comprises a V_{H} comprising the amino acid sequence of SEQ ID NO: 53 and a V_{L} comprising the amino acid sequence of SEQ ID NO: 65.

In some embodiments, the anti-LIGHT antibody comprises a V_{H} comprising the amino acid sequence of SEQ ID NO: 51, or a variant thereof having at least about 80% (such as at least about any of 80%, 85%, 90%, 95%, 96%, 97%, 98%, or 99%) sequence identity to the amino acid sequence of SEQ ID NO: 51, and a V_{L} comprising the amino acid sequence of SEQ ID NO: 65, or a variant thereof having at least about 80% (such as at least about any of 80%, 85%, 90%, 95%, 96%, 97%, 98%, or 99%) sequence identity to the amino acid sequence of SEQ ID NO: 65. In some embodiments, the anti-LIGHT antibody comprises a V_{H} comprising the amino acid sequence of SEQ ID NO: 51 and a V_{L} comprising the amino acid sequence of SEQ ID NO: 65.

In some embodiments, the anti-LIGHT antibody comprises a V_{H} comprising: an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17, or a variant thereof comprising up to about 5 amino acid substitutions in the HC-CDRs; and a V_{L} comprising an LC-CDR1 comprising the amino acid sequence of SEQ ID NO: 24, an LC-CDR2 comprising the amino acid sequence of SEQ ID NO: 31, and an LC-CDR3 comprising the amino acid sequence of SEQ ID NO: 38, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and a V_{L} comprising an LC-CDR1 comprising the amino acid sequence of SEQ ID NO: 24, an LC-CDR2 comprising the amino acid sequence of SEQ ID NO: 31, and an LC-CDR3 comprising the amino acid sequence of SEQ ID NO: 38.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 54, and a V_{L} comprising an LC-CDR1, an LC-CDR2 and an LC-CDR3 of the V_{L} comprising the amino acid sequence of SEQ ID NO: 66.

In some embodiments, the anti-LIGHT antibody comprises a V_{H} comprising the amino acid sequence of SEQ ID NO: 54, or a variant thereof having at least about 80% (such as at least about any of 80%, 85%, 90%, 95%, 96%, 97%, 98%, or 99%) sequence identity to the amino acid sequence of SEQ ID NO: 54, and a V_{L} comprising the amino acid sequence of SEQ ID NO: 66, or a variant thereof having at least about 80% (such as at least about any of 80%, 85%, 90%, 95%, 96%, 97%, 98%, or 99%) sequence identity to the amino acid sequence of SEQ ID NO: 66. In some embodiments, the anti-LIGHT antibody comprises a V_{H} comprising the amino acid sequence of SEQ ID NO: 54 and a V_{L} comprising the amino acid sequence of SEQ ID NO: 66.

In some embodiments, the anti-LIGHT antibody comprises a V_{H} comprising: an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17, or a variant thereof comprising up to about 5 amino acid substitutions in the HC-CDRs; and a V_{L} comprising an LC-CDR1 comprising the amino acid sequence of SEQ ID NO: 25, an LC-CDR2 comprising the amino acid sequence of SEQ ID NO: 31, and an LC-CDR3 comprising the amino acid sequence of SEQ ID NO: 39, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and a V_{L} comprising an LC-CDR1 comprising the amino acid sequence of SEQ ID NO: 25, an LC-CDR2 comprising the amino acid sequence of SEQ ID NO: 31, and an LC-CDR3 comprising the amino acid sequence of SEQ ID NO: 39.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 55, and a V_{L} comprising an LC-CDR1, an LC-CDR2 and an LC-CDR3 of the V_{L} comprising the amino acid sequence of SEQ ID NO: 67.

In some embodiments, the anti-LIGHT antibody comprises a V_{H} comprising the amino acid sequence of SEQ ID NO: 55, or a variant thereof having at least about 80% (such as at least about any of 80%, 85%, 90%, 95%, 96%, 97%, 98%, or 99%) sequence identity to the amino acid sequence of SEQ ID NO: 55, and a V_{L} comprising the amino acid sequence of SEQ ID NO: 67, or a variant thereof having at least about 80% (such as at least about any of 80%, 85%, 90%, 95%, 96%, 97%, 98%, or 99%) sequence identity to the amino acid sequence of SEQ ID NO: 67. In some embodiments, the anti-LIGHT antibody comprises a V_{H} comprising the amino acid sequence of SEQ ID NO: 55 and a V_{L} comprising the amino acid sequence of SEQ ID NO: 67.

In some embodiments, the anti-LIGHT antibody comprises a V_{H} comprising: an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18, or a variant thereof comprising up to about 5 amino acid substitutions in the HC-CDRs; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 40, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 40.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 56, and a V_{L} comprising an LC-CDR1, an LC-CDR2 and an LC-CDR3 of the V_{L} comprising the amino acid sequence of SEQ ID NO: 68.

In some embodiments, the anti-LIGHT antibody comprises a V_{H} comprising the amino acid sequence of SEQ ID NO: 56, or a variant thereof having at least about 80% (such as at least about any of 80%, 85%, 90%, 95%, 96%, 97%, 98%, or 99%) sequence identity to the amino acid sequence of SEQ ID NO: 56, and a V_{L} comprising the amino acid sequence of SEQ ID NO: 68, or a variant thereof having at least about 80% (such as at least about any of 80%, 85%, 90%, 95%, 96%, 97%, 98%, or 99%) sequence identity to the amino acid sequence of SEQ ID NO: 68. In some embodiments, the anti-LIGHT antibody comprises a V_{H} comprising the amino acid sequence of SEQ ID NO: 56 and a V_{L} comprising the amino acid sequence of SEQ ID NO: 68.

In some embodiments, the anti-LIGHT antibody comprises a V_{H} comprising: an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18, or a variant thereof comprising up to about 5 amino acid substitutions in the HC-CDRs; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 41, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 41.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 57, and a V_{L} comprising an LC-CDR1, an LC-CDR2 and an LC-CDR3 of the V_{L} comprising the amino acid sequence of SEQ ID NO: 69.

In some embodiments, the anti-LIGHT antibody comprises a V_{H} comprising the amino acid sequence of SEQ ID NO: 57, or a variant thereof having at least about 80% (such as at least about any of 80%, 85%, 90%, 95%, 96%, 97%, 98%, or 99%) sequence identity to the amino acid sequence of SEQ ID NO: 57, and a V_{L} comprising the amino acid sequence of SEQ ID NO: 69, or a variant thereof having at least about 80% (such as at least about any of 80%, 85%, 90%, 95%, 96%, 97%, 98%, or 99%) sequence identity to the amino acid sequence of SEQ ID NO: 69. In some embodiments, the anti-LIGHT antibody comprises a V_{H} comprising the amino acid sequence of SEQ ID NO: 57 and a V_{L} comprising the amino acid sequence of SEQ ID NO: 69.

In some embodiments, the anti-LIGHT antibody comprises a V_{H} comprising: an HC-CDR1 comprising the amino acid sequence of SEQ ID NO: 4, an HC-CDR2 comprising the amino acid sequence of SEQ ID NO: 12, and an HC-CDR3 comprising the amino acid sequence of SEQ ID NO: 19, or a variant thereof comprising up to about 5 amino acid substitutions in the HC-CDRs; and a V_{L} comprising an LC-CDR1 comprising the amino acid sequence of SEQ ID NO: 27, an LC-CDR2 comprising the amino acid sequence of SEQ ID NO: 33, and an LC-CDR3 comprising the amino acid sequence of SEQ ID NO: 42, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 4, an HC-CDR2 comprising the amino acid sequence of SEQ ID NO: 12, and an HC-CDR3 comprising the amino acid sequence of SEQ ID NO: 19; and a V_{L} comprising an LC-CDR1 comprising the amino acid sequence of SEQ ID NO: 27, an LC-CDR2 comprising the amino acid sequence of SEQ ID NO: 33, and an LC-CDR3 comprising the amino acid sequence of SEQ ID NO: 42.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 58, and a V_{L} comprising an LC-CDR1, an LC-CDR2 and an LC-CDR3 of the V_{L} comprising the amino acid sequence of SEQ ID NO: 70.

In some embodiments, the anti-LIGHT antibody comprises a V_{H} comprising the amino acid sequence of SEQ ID NO: 58, or a variant thereof having at least about 80% (such as at least about any of 80%, 85%, 90%, 95%, 96%, 97%, 98%, or 99%) sequence identity to the amino acid sequence of SEQ ID NO: 58, and a V_{L} comprising the amino acid sequence of SEQ ID NO: 70, or a variant thereof having at least about 80% (such as at least about any of 80%, 85%, 90%, 95%, 96%, 97%, 98%, or 99%) sequence identity to the amino acid sequence of SEQ ID NO: 70. In some embodiments, the anti-LIGHT antibody comprises a V_{H} comprising the amino acid sequence of SEQ ID NO: 58 and a V_{L} comprising the amino acid sequence of SEQ ID NO: 70.

In some embodiments, the anti-LIGHT antibody comprises a V_{H} comprising: an HC-CDR1 comprising the amino acid sequence of SEQ ID NO: 5, an HC-CDR2 comprising the amino acid sequence of SEQ ID NO: 13, and an HC-CDR3 comprising the amino acid sequence of SEQ ID NO: 20, or a variant thereof comprising up to about 5 amino acid substitutions in the HC-CDRs; and a V_{L} comprising an LC-CDR1 comprising the amino acid sequence of SEQ ID NO: 28, an LC-CDR2 comprising the amino acid sequence of SEQ ID NO: 34, and an LC-CDR3 comprising the amino acid sequence of SEQ ID NO: 43, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 5, an HC-CDR2 comprising the amino acid sequence of SEQ ID NO: 13, and an HC-CDR3 comprising the amino acid sequence of SEQ ID NO: 20; and a V_{L} comprising an LC-CDR1 comprising the amino acid sequence of SEQ ID NO: 28, an LC-CDR2 comprising the amino acid sequence of SEQ ID NO: 34, and an LC-CDR3 comprising the amino acid sequence of SEQ ID NO: 43.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 59, and a V_{L} comprising an LC-CDR1, an LC-CDR2 and an LC-CDR3 of the V_{L} comprising the amino acid sequence of SEQ ID NO: 71.

In some embodiments, the anti-LIGHT antibody comprises a V_{H} comprising the amino acid sequence of SEQ ID NO: 59, or a variant thereof having at least about 80% (such as at least about any of 80%, 85%, 90%, 95%, 96%, 97%, 98%, or 99%) sequence identity to the amino acid sequence of SEQ ID NO: 59, and a V_{L} comprising the amino acid sequence of SEQ ID NO: 71, or a variant thereof having at least about 80% (such as at least about any of 80%, 85%, 90%, 95%, 96%, 97%, 98%, or 99%) sequence identity to the amino acid sequence of SEQ ID NO: 71. In some embodiments, the anti-LIGHT antibody comprises a V_{H} comprising the amino acid sequence of SEQ ID NO: 59 and a V_{L} comprising the amino acid sequence of SEQ ID NO: 71.

In some embodiments, the isolated anti-LIGHT antibody comprises: a heavy chain variable domain (V_{H}) comprising a heavy chain complementarity determining region (HC-CDR) 1 comprising DHIMN (SEQ ID NO: 6); an HC-CDR2 comprising RIYPVSGETNYNQKFMG (SEQ ID NO: 14); and an HC-CDR3 comprising GSYYWNAMDY (SEQ ID NO: 21); and a light chain variable domain (V_{L}) comprising a light chain complementarity determining region (LC-CDR) 1 comprising KASQSVDFDGDSYMN (SEQ ID NO: 29) or KASQSVDFDGESYMN (SEQ ID NO: 83); an LC-CDR2 comprising SASNLES (SEQ ID NO: 35); and an LC-CDR3 comprising QQSIEDPWT (SEQ ID NO: 43)

In some embodiments, the anti-LIGHT antibody comprises a V_{H} comprising: an HC-CDR1 comprising the amino acid sequence of SEQ ID NO: 6, or a variant thereof comprising up to about 3 (such as about any of 1, 2, or 3) amino acid substitutions, an HC-CDR2 comprising the amino acid sequence of SEQ ID NO: 14, or a variant thereof comprising up to about 3 (such as about any of 1, 2, or 3) amino acid substitutions, and an HC-CDR3 comprising the amino acid sequence of SEQ ID NO: 21, or a variant thereof comprising up to about 3 (such as about any of 1, 2, or 3) amino acid substitutions.

In some embodiments, the anti-LIGHT antibody comprises a V_{H} comprising: an HC-CDR1 comprising the amino acid sequence of SEQ ID NO: 6, an HC-CDR2 comprising the amino acid sequence of any one of SEQ ID NO: 14, and an HC-CDR3 comprising the amino acid sequence of SEQ ID NO: 21.

In some embodiments, the anti-LIGHT antibody comprises a V_{L} comprising: an LC-CDR1 comprising the amino acid sequence of any one of SEQ ID NOs: 29 or 83, or a variant thereof comprising up to about 3 (such as about any of 1, 2, or 3) amino acid substitutions, an LC-CDR2 comprising the amino acid sequence of SEQ ID NO: 35, or a variant thereof comprising up to about 3 (such as about any of 1, 2, or 3) amino acid substitutions, and an LC-CDR3 comprising the amino acid sequence of SEQ ID NO: 43, or a variant thereof comprising up to about 3 (such as about any of 1, 2, or 3) amino acid substitutions.

In some embodiments, the anti-LIGHT antibody comprises a V_{L} comprising: an LC-CDR1 comprising the amino acid sequence of any one of SEQ ID NOs: 29 or 83, an LC-CDR2 comprising the amino acid sequence of SEQ ID NO: 35, and an LC-CDR3 comprising the amino acid sequence of SEQ ID NO: 43.

In some embodiments, the anti-LIGHT antibody comprises a V_{H} comprising: an HC-CDR1 comprising the amino acid sequence of SEQ ID NO: 6, or a variant thereof comprising up to about 3 (such as about any of 1, 2, or 3) amino acid substitutions, an HC-CDR2 comprising the amino acid sequence of SEQ ID NO: 14, or a variant thereof comprising up to about 3 (such as about any of 1, 2, or 3) amino acid substitutions, and an HC-CDR3 comprising the amino acid sequence of SEQ ID NO: 21, or a variant thereof comprising up to about 3 (such as about any of 1, 2, or 3) amino acid substitutions; and a V_{L} comprising: an LC-CDR1 comprising the amino acid sequence of any one of SEQ ID NOs: 29 or 83, or a variant thereof comprising up to about 3 (such as about any of 1, 2, or 3) amino acid substitutions, an LC-CDR2 comprising the amino acid sequence of SEQ ID NO: 35, or a variant thereof comprising up to about 3 (such as about any of 1, 2, or 3) amino acid substitutions, and an LC-CDR3 comprising the amino acid sequence of SEQ ID NO: 43, or a variant thereof comprising up to about 3 (such as about any of 1, 2, or 3) amino acid substitutions.

In some embodiments, the anti-LIGHT antibody comprises a V_{H} comprising: an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and a V_{L} comprising: an LC-CDR1 comprising the amino acid sequence of any one of SEQ ID NOs: 29 or 83, an LC-CDR2 comprising the amino acid sequence of SEQ ID NO: 35, and an LC-CDR3 comprising the amino acid sequence of SEQ ID NO: 43.

In some embodiments, the anti-LIGHT antibody comprises a V_{H} comprising: an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21, or a variant thereof comprising up to about 5 amino acid substitutions in the HC-CDRs; and a V_{L} comprising an LC-CDR1 comprising the amino acid sequence of SEQ ID NO: 29, an LC-CDR2 comprising the amino acid sequence of SEQ ID NO: 35, and an LC-CDR3 comprising the amino acid sequence of SEQ ID NO: 43, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and a V_{L} comprising an LC-CDR1 comprising the amino acid sequence of SEQ ID NO: 29, an LC-CDR2 comprising the amino acid sequence of SEQ ID NO: 35, and an LC-CDR3 comprising the amino acid sequence of SEQ ID NO: 43.

In some embodiments, the anti-LIGHT antibody comprises a V_{H} comprising: an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21, or a variant thereof comprising up to about 5 amino acid substitutions in the HC-CDRs; and a V_{L} comprising an LC-CDR1 comprising the amino acid sequence of SEQ ID NO: 83, an LC-CDR2 comprising the amino acid sequence of SEQ ID NO: 35, and an LC-CDR3 comprising the amino acid sequence of SEQ ID NO: 43, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and a V_{L} comprising an LC-CDR1 comprising the amino acid sequence of SEQ ID NO: 83, an LC-CDR2 comprising the amino acid sequence of SEQ ID NO: 35, and an LC-CDR3 comprising the amino acid sequence of SEQ ID NO: 43.

In some embodiments, the anti-LIGHT antibody comprises a V_{H} comprising an HC-CDR1, an HC-CDR2 and an HC-CDR3 of the V_{H} comprising the amino acid sequence of any one of SEQ ID NOs: 60, 84-85; and a V_{L} comprising an LC-CDR1, an LC-CDR2, and an LC-CDR3 of the V_{L} comprising the amino acid sequence of any one of SEQ ID NOs: 72, 86-91.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 60, and a V_{L} comprising an LC-CDR1, an LC-CDR2 and an LC-CDR3 of the V_{L} comprising the amino acid sequence of SEQ ID NO: 72.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 85, and a V_{L} comprising an LC-CDR1, an LC-CDR2 and an LC-CDR3 of the V_{L} comprising the amino acid sequence of SEQ ID NO: 90.

In some embodiments, the anti-LIGHT antibody comprises a V_{H} comprising the amino acid sequence of any one of SEQ ID NOs: 60, 84-85, or a variant thereof having at least about 80% (such as at least about any of 80%, 85%, 90%, 95%, 96%, 97%, 98%, or 99%) sequence identity to the amino acid sequence of any one of SEQ ID NOs: 60, 84-85, and a V_{L} comprising the amino acid sequence of any one of SEQ ID NOs: 72, 86-91, or a variant thereof having at least about 80% (such as at least about any of 80%, 85%, 90%, 95%, 96%, 97%, 98%, or 99%) sequence identity to the amino acid sequence of any one of SEQ ID NOs: 72, 86-91. In some embodiments, the anti-LIGHT antibody comprises a V_{H} comprising the amino acid sequence of any one of SEQ ID NOs: 60, 84-85, and a V_{L} comprising the amino acid sequence of any one of SEQ ID NOs: 72, 86-91.

In some embodiments, the anti-LIGHT antibody comprises a V_{H} comprising the amino acid sequence of SEQ ID NO: 60, or a variant thereof having at least about 80% (such as at least about any of 80%, 85%, 90%, 95%, 96%, 97%, 98%, or 99%) sequence identity to the amino acid sequence of SEQ ID NO: 60, and a V_{L} comprising the amino acid sequence of SEQ ID NO: 72, or a variant thereof having at least about 80% (such as at least about any of 80%, 85%, 90%, 95%, 96%, 97%, 98%, or 99%) sequence identity to the amino acid sequence of SEQ ID NO: 72. In some embodiments, the anti-LIGHT antibody comprises a V_{H} comprising the amino acid sequence of SEQ ID NO: 60 and a V_{L} comprising the amino acid sequence of SEQ ID NO: 72.

In some embodiments, the anti-LIGHT antibody comprises a V_{H} comprising the amino acid sequence of SEQ ID NO: 84, or a variant thereof having at least about 80% (such as at least about any of 80%, 85%, 90%, 95%, 96%, 97%, 98%, or 99%) sequence identity to the amino acid sequence of SEQ ID NO: 84, and a V_{L} comprising the amino acid sequence of SEQ ID NO: 86, or a variant thereof having at least about 80% (such as at least about any of 80%, 85%, 90%, 95%, 96%, 97%, 98%, or 99%) sequence identity to the amino acid sequence of SEQ ID NO: 86. In some embodiments, the anti-LIGHT antibody comprises a V_{H} comprising the amino acid sequence of SEQ ID NO: 84 and a V_{L} comprising the amino acid sequence of SEQ ID NO: 86.

In some embodiments, the anti-LIGHT antibody comprises a V_{H} comprising the amino acid sequence of SEQ ID NO: 85, or a variant thereof having at least about 80% (such as at least about any of 80%, 85%, 90%, 95%, 96%, 97%, 98%, or 99%) sequence identity to the amino acid sequence of SEQ ID NO: 85, and a V_{L} comprising the amino acid sequence of SEQ ID NO: 87, or a variant thereof having at least about 80% (such as at least about any of 80%, 85%, 90%, 95%, 96%, 97%, 98%, or 99%) sequence identity to the amino acid sequence of SEQ ID NO: 87. In some embodiments, the anti-LIGHT antibody comprises a V_{H} comprising the amino acid sequence of SEQ ID NO: 85 and a V_{L} comprising the amino acid sequence of SEQ ID NO: 87.

In some embodiments, the anti-LIGHT antibody comprises a V_{H} comprising the amino acid sequence of SEQ ID NO: 84, or a variant thereof having at least about 80% (such as at least about any of 80%, 85%, 90%, 95%, 96%, 97%, 98%, or 99%) sequence identity to the amino acid sequence of SEQ ID NO: 84, and a V_{L} comprising the amino acid sequence of SEQ ID NO: 88, or a variant thereof having at least about 80% (such as at least about any of 80%, 85%, 90%, 95%, 96%, 97%, 98%, or 99%) sequence identity to the amino acid sequence of SEQ ID NO: 88. In some embodiments, the anti-LIGHT antibody comprises a V_{H} comprising the amino acid sequence of SEQ ID NO: 84 and a V_{L} comprising the amino acid sequence of SEQ ID NO: 88.

In some embodiments, the anti-LIGHT antibody comprises a V_{H} comprising the amino acid sequence of SEQ ID NO: 84, or a variant thereof having at least about 80% (such as at least about any of 80%, 85%, 90%, 95%, 96%, 97%, 98%, or 99%) sequence identity to the amino acid sequence of SEQ ID NO: 84, and a V_{L} comprising the amino acid sequence of SEQ ID NO: 89, or a variant thereof having at least about 80% (such as at least about any of 80%, 85%, 90%, 95%, 96%, 97%, 98%, or 99%) sequence identity to the amino acid sequence of SEQ ID NO: 89. In some embodiments, the anti-LIGHT antibody comprises a V_{H} comprising the amino acid sequence of SEQ ID NO: 84 and a V_{L} comprising the amino acid sequence of SEQ ID NO: 89.

In some embodiments, the anti-LIGHT antibody comprises a V_{H} comprising the amino acid sequence of SEQ ID NO: 85, or a variant thereof having at least about 80% (such as at least about any of 80%, 85%, 90%, 95%, 96%, 97%, 98%, or 99%) sequence identity to the amino acid sequence of SEQ ID NO: 85, and a V_{L} comprising the amino acid sequence of SEQ ID NO: 90, or a variant thereof having at least about 80% (such as at least about any of 80%, 85%, 90%, 95%, 96%, 97%, 98%, or 99%) sequence identity to the amino acid sequence of SEQ ID NO: 90. In some embodiments, the anti-LIGHT antibody comprises a V_{H} comprising the amino acid sequence of SEQ ID NO: 85 and a V_{L} comprising the amino acid sequence of SEQ ID NO: 90.

In some embodiments, the anti-LIGHT antibody comprises a V_{H} comprising the amino acid sequence of SEQ ID NO: 84, or a variant thereof having at least about 80% (such as at least about any of 80%, 85%, 90%, 95%, 96%, 97%, 98%, or 99%) sequence identity to the amino acid sequence of SEQ ID NO: 84, and a V_{L} comprising the amino acid sequence of SEQ ID NO: 91, or a variant thereof having at least about 80% (such as at least about any of 80%, 85%, 90%, 95%, 96%, 97%, 98%, or 99%) sequence identity to the amino acid sequence of SEQ ID NO: 91. In some embodiments, the anti-LIGHT antibody comprises a V_{H} comprising the amino acid sequence of SEQ ID NO: 84 and a V_{L} comprising the amino acid sequence of SEQ ID NO: 91.

In some embodiments, the isolated anti-LIGHT antibody comprises: a heavy chain variable domain (V_{H}) comprising a heavy chain complementarity determining region (HC-CDR) 1 comprising SYNVH (SEQ ID NO: 7); an HC-CDR2 comprising AVYPGNGDTSYNQKFKG (SEQ ID NO: 15); and an HC-CDR3 comprising GSYYYTSSYFDH (SEQ ID NO: 22); and a light chain variable domain (V_{L}) comprising a light chain complementarity determining region (LC-CDR) 1 comprising KASQSVDFDGDSYMN (SEQ ID NO: 29); an LC-CDR2 comprising TASNLES (SEQ ID NO: 36); and an LC-CDR3 comprising QQSYEDPFT (SEQ ID NO: 44)

In some embodiments, the anti-LIGHT antibody comprises a V_{H} comprising: an HC-CDR1 comprising the amino acid sequence of SEQ ID NO: 7, or a variant thereof comprising up to about 3 (such as about any of 1, 2, or 3) amino acid substitutions, an HC-CDR2 comprising the amino acid sequence of SEQ ID NO: 15, or a variant thereof comprising up to about 3 (such as about any of 1, 2, or 3) amino acid substitutions, and an HC-CDR3 comprising the amino acid sequence of SEQ ID NO: 22, or a variant thereof comprising up to about 3 (such as about any of 1, 2, or 3) amino acid substitutions.

In some embodiments, the anti-LIGHT antibody comprises a V_{H} comprising: an HC-CDR1 comprising the amino acid sequence of SEQ ID NO: 7, an HC-CDR2 comprising the amino acid sequence of any one of SEQ ID NO: 15, and an HC-CDR3 comprising the amino acid sequence of SEQ ID NO: 22.

In some embodiments, the anti-LIGHT antibody comprises a V_{L} comprising: an LC-CDR1 comprising the amino acid sequence of SEQ ID NO: 29, or a variant thereof comprising up to about 3 (such as about any of 1, 2, or 3) amino acid substitutions, an LC-CDR2 comprising the amino acid sequence of SEQ ID NO: 36, or a variant thereof comprising up to about 3 (such as about any of 1, 2, or 3) amino acid substitutions, and an LC-CDR3 comprising the amino acid sequence of SEQ ID NO: 44, or a variant thereof comprising up to about 3 (such as about any of 1, 2, or 3) amino acid substitutions.

In some embodiments, the anti-LIGHT antibody comprises a V_{L} comprising: an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44.

In some embodiments, the anti-LIGHT antibody comprises a V_{H} comprising: an HC-CDR1 comprising the amino acid sequence of SEQ ID NO: 7, or a variant thereof comprising up to about 3 (such as about any of 1, 2, or 3) amino acid substitutions, an HC-CDR2 comprising the amino acid sequence of SEQ ID NO: 15, or a variant thereof comprising up to about 3 (such as about any of 1, 2, or 3) amino acid substitutions, and an HC-CDR3 comprising the amino acid sequence of SEQ ID NO: 22, or a variant thereof comprising up to about 3 (such as about any of 1, 2, or 3) amino acid substitutions; and a V_{L} comprising: an LC-CDR1 comprising the amino acid sequence of SEQ ID NO: 29, or a variant thereof comprising up to about 3 (such as about any of 1, 2, or 3) amino acid substitutions, an LC-CDR2 comprising the amino acid sequence of SEQ ID NO: 36, or a variant thereof comprising up to about 3 (such as about any of 1, 2, or 3) amino acid substitutions, and an LC-CDR3 comprising the amino acid sequence of SEQ ID NO: 44, or a variant thereof comprising up to about 3 (such as about any of 1, 2, or 3) amino acid substitutions.

In some embodiments, the anti-LIGHT antibody comprises a V_{H} comprising: an HC-CDR1 comprising the amino acid sequence of SEQ ID NO: 7, an HC-CDR2 comprising the amino acid sequence of SEQ ID NO: 15, and an HC-CDR3 comprising the amino acid sequence of SEQ ID NO: 22; and a V_{L} comprising: an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44.

In some embodiments, the anti-LIGHT antibody comprises a V_{H} comprising: an HC-CDR1 comprising the amino acid sequence of SEQ ID NO: 7, an HC-CDR2 comprising the amino acid sequence of SEQ ID NO: 15, and an HC-CDR3 comprising the amino acid sequence of SEQ ID NO: 22, or a variant thereof comprising up to about 5 amino acid substitutions in the HC-CDRs; and a V_{L} comprising an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44, or a variant thereof comprising up to about 5 amino acid substitutions in the LC-CDRs.

In some embodiments, the anti-LIGHT antibody comprises a V_{H} comprising: an HC-CDR1 comprising the amino acid sequence of SEQ ID NO: 7, an HC-CDR2 comprising the amino acid sequence of SEQ ID NO: 15, and an HC-CDR3 comprising the amino acid sequence of SEQ ID NO: 22; and a V_{L} comprising an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44.

In some embodiments, the anti-LIGHT antibody comprises a V_{H} comprising an HC-CDR1, an HC-CDR2 and an HC-CDR3 of the V_{H} comprising the amino acid sequence of any one of SEQ ID NOs: 61, 92-94; and a V_{L} comprising an LC-CDR1, an LC-CDR2, and an LC-CDR3 of the V_{L} comprising the amino acid sequence of any one of SEQ ID NOs: 73, 95-99.

In some embodiments, the anti-LIGHT antibody comprises a V_{H} comprising an HC-CDR1, an HC-CDR2 and an HC-CDR3 of the V_{H} comprising the amino acid sequence of SEQ ID NO: 61, and a V_{L} comprising an LC-CDR1, an LC-CDR2 and an LC-CDR3 of the V_{L} comprising the amino acid sequence of SEQ ID NO: 73.

In some embodiments, the anti-LIGHT antibody comprises a V_{H} comprising the amino acid sequence of any one of SEQ ID NOs: 61, 92-94, or a variant thereof having at least about 80% (such as at least about any of 80%, 85%, 90%, 95%, 96%, 97%, 98%, or 99%) sequence identity to the amino acid sequence of any one of SEQ ID NOs: 61, 92-94, and a V_{L} comprising the amino acid sequence of any one of SEQ ID NOs: 73, 95-99, or a variant thereof having at least about 80% (such as at least about any of 80%, 85%, 90%, 95%, 96%, 97%, 98%, or 99%) sequence identity to the amino acid sequence of any one of SEQ ID NOs: 73, 95-99. In some embodiments, the anti-LIGHT antibody comprises a V_{H} comprising the amino acid sequence of any one of SEQ ID NOs: 61, 92-94, and a V_{L} comprising the amino acid sequence of any one of SEQ ID NOs: 73, 95-99.

In some embodiments, the anti-LIGHT antibody comprises a V_{H} comprising the amino acid sequence of SEQ ID NO: 61, or a variant thereof having at least about 80% (such as at least about any of 80%, 85%, 90%, 95%, 96%, 97%, 98%, or 99%) sequence identity to the amino acid sequence of SEQ ID NO: 61, and a V_{L} comprising the amino acid sequence of SEQ ID NO: 73, or a variant thereof having at least about 80% (such as at least about any of 80%, 85%, 90%, 95%, 96%, 97%, 98%, or 99%) sequence identity to the amino acid sequence of SEQ ID NO: 73. In some embodiments, the anti-LIGHT antibody comprises a V_{H} comprising the amino acid sequence of SEQ ID NO: 61 and a V_{L} comprising the amino acid sequence of SEQ ID NO: 73.

In some embodiments, the anti-LIGHT antibody comprises a V_{H} comprising the amino acid sequence of SEQ ID NO: 92, or a variant thereof having at least about 80% (such as at least about any of 80%, 85%, 90%, 95%, 96%, 97%, 98%, or 99%) sequence identity to the amino acid sequence of SEQ ID NO: 92, and a V_{L} comprising the amino acid sequence of SEQ ID NO: 95, or a variant thereof having at least about 80% (such as at least about any of 80%, 85%, 90%, 95%, 96%, 97%, 98%, or 99%) sequence identity to the amino acid sequence of SEQ ID NO: 95. In some embodiments, the anti-LIGHT antibody comprises a V_{H} comprising the amino acid sequence of SEQ ID NO: 92 and a V_{L} comprising the amino acid sequence of SEQ ID NO: 95.

In some embodiments, the anti-LIGHT antibody comprises a V_{H} comprising the amino acid sequence of SEQ ID NO: 93, or a variant thereof having at least about 80% (such as at least about any of 80%, 85%, 90%, 95%, 96%, 97%, 98%, or 99%) sequence identity to the amino acid sequence of SEQ ID NO: 93, and a V_{L} comprising the amino acid sequence of SEQ ID NO: 96, or a variant thereof having at least about 80% (such as at least about any of 80%, 85%, 90%, 95%, 96%, 97%, 98%, or 99%) sequence identity to the amino acid sequence of SEQ ID NO: 96. In some embodiments, the anti-LIGHT antibody comprises a V_{H} comprising the amino acid sequence of SEQ ID NO: 93 and a V_{L} comprising the amino acid sequence of SEQ ID NO: 96.

In some embodiments, the anti-LIGHT antibody comprises a V_{H} comprising the amino acid sequence of SEQ ID NO: 92, or a variant thereof having at least about 80% (such as at least about any of 80%, 85%, 90%, 95%, 96%, 97%, 98%, or 99%) sequence identity to the amino acid sequence of SEQ ID NO: 92, and a V_{L} comprising the amino acid sequence of SEQ ID NO: 96, or a variant thereof having at least about 80% (such as at least about any of 80%, 85%, 90%, 95%, 96%, 97%, 98%, or 99%) sequence identity to the amino acid sequence of SEQ ID NO: 96. In some embodiments, the anti-LIGHT antibody comprises a V_{H} comprising the amino acid sequence of SEQ ID NO: 92 and a V_{L} comprising the amino acid sequence of SEQ ID NO: 96.

In some embodiments, the anti-LIGHT antibody comprises a V_{H} comprising the amino acid sequence of SEQ ID NO: 94, or a variant thereof having at least about 80% (such as at least about any of 80%, 85%, 90%, 95%, 96%, 97%, 98%, or 99%) sequence identity to the amino acid sequence of SEQ ID NO: 94, and a V_{L} comprising the amino acid sequence of SEQ ID NO: 97, or a variant thereof having at least about 80% (such as at least about any of 80%, 85%, 90%, 95%, 96%, 97%, 98%, or 99%) sequence identity to the amino acid sequence of SEQ ID NO: 97. In some embodiments, the anti-LIGHT antibody comprises a V_{H} comprising the amino acid sequence of SEQ ID NO: 94 and a V_{L} comprising the amino acid sequence of SEQ ID NO: 97.

In some embodiments, the anti-LIGHT antibody comprises a V_{H} comprising the amino acid sequence of SEQ ID NO: 94, or a variant thereof having at least about 80% (such as at least about any of 80%, 85%, 90%, 95%, 96%, 97%, 98%, or 99%) sequence identity to the amino acid sequence of SEQ ID NO: 94, and a V_{L} comprising the amino acid sequence of SEQ ID NO: 98, or a variant thereof having at least about 80% (such as at least about any of 80%, 85%, 90%, 95%, 96%, 97%, 98%, or 99%) sequence identity to the amino acid sequence of SEQ ID NO: 98. In some embodiments, the anti-LIGHT antibody comprises a V_{H} comprising the amino acid sequence of SEQ ID NO: 94 and a V_{L} comprising the amino acid sequence of SEQ ID NO: 98.

In some embodiments, the anti-LIGHT antibody comprises a V_{H} comprising the amino acid sequence of SEQ ID NO: 93, or a variant thereof having at least about 80% (such as at least about any of 80%, 85%, 90%, 95%, 96%, 97%, 98%, or 99%) sequence identity to the amino acid sequence of SEQ ID NO: 93, and a V_{L} comprising the amino acid sequence of SEQ ID NO: 98, or a variant thereof having at least about 80% (such as at least about any of 80%, 85%, 90%, 95%, 96%, 97%, 98%, or 99%) sequence identity to the amino acid sequence of SEQ ID NO: 98. In some embodiments, the anti-LIGHT antibody comprises a V_{H} comprising the amino acid sequence of SEQ ID NO: 93 and a V_{L} comprising the amino acid sequence of SEQ ID NO: 98.

In some embodiments, the anti-LIGHT antibody comprises a V_{H} comprising the amino acid sequence of SEQ ID NO: 92, or a variant thereof having at least about 80% (such as at least about any of 80%, 85%, 90%, 95%, 96%, 97%, 98%, or 99%) sequence identity to the amino acid sequence of SEQ ID NO: 92, and a V_{L} comprising the amino acid sequence of SEQ ID NO: 98, or a variant thereof having at least about 80% (such as at least about any of 80%, 85%, 90%, 95%, 96%, 97%, 98%, or 99%) sequence identity to the amino acid sequence of SEQ ID NO: 98. In some embodiments, the anti-LIGHT antibody comprises a V_{H} comprising the amino acid sequence of SEQ ID NO: 92 and a V_{L} comprising the amino acid sequence of SEQ ID NO: 98.

In some embodiments, the anti-LIGHT antibody comprises a V_{H} comprising the amino acid sequence of SEQ ID NO: 94, or a variant thereof having at least about 80% (such as at least about any of 80%, 85%, 90%, 95%, 96%, 97%, 98%, or 99%) sequence identity to the amino acid sequence of SEQ ID NO: 94, and a V_{L} comprising the amino acid sequence of SEQ ID NO: 99, or a variant thereof having at least about 80% (such as at least about any of 80%, 85%, 90%, 95%, 96%, 97%, 98%, or 99%) sequence identity to the amino acid sequence of SEQ ID NO: 99. In some embodiments, the anti-LIGHT antibody comprises a V_{H} comprising the amino acid sequence of SEQ ID NO: 94 and a V_{L} comprising the amino acid sequence of SEQ ID NO: 99.

In some embodiments, the anti-LIGHT antibody comprises a V_{H} comprising the amino acid sequence of SEQ ID NO: 92, or a variant thereof having at least about 80% (such as at least about any of 80%, 85%, 90%, 95%, 96%, 97%, 98%, or 99%) sequence identity to the amino acid sequence of SEQ ID NO: 92, and a V_{L} comprising the amino acid sequence of SEQ ID NO: 99, or a variant thereof having at least about 80% (such as at least about any of 80%, 85%, 90%, 95%, 96%, 97%, 98%, or 99%) sequence identity to the amino acid sequence of SEQ ID NO: 99. In some embodiments, the anti-LIGHT antibody comprises a V_{H} comprising the amino acid sequence of SEQ ID NO: 92 and a V_{L} comprising the amino acid sequence of SEQ ID NO: 99.

In some embodiments, the amino acid substitutions described above are limited to "exemplary substitutions" shown in Table 4 of this application. In some embodiments, the amino acid substitutions are limited to "preferred substitutions" shown in Table 4 of this application.

In some embodiments, functional epitopes can be mapped by combinatorial alanine scanning. In this process, a combinatorial alanine-scanning strategy can be used to identify amino acids in the LIGHT protein that are necessary for interaction with LIGHT antibodies. In some embodiments, the epitope is conformational and crystal structure of anti-LIGHT antibodies bound to LIGHT may be employed to identify the epitopes.

In some embodiments, the present application provides antibodies which compete with any one of the LIGHT antibodies described herein for binding to LIGHT. In some embodiments, the present application provides antibodies which compete with any one of the anti-LIGHT antibodies provided herein for binding to an epitope on the LIGHT. In some embodiments, an anti-LIGHT antibody is provided that binds to the same epitope as an anti-LIGHT antibody comprising a V_{H} comprising the amino acid sequence of any one of SEQ ID NOs: 46-61, 92-94, and a V_{L} comprising the amino acid sequence of any one of SEQ ID NOs: 62-73, 95-99. In some embodiments, an anti-LIGHT antibody is provided that specifically binds to LIGHT competitively with an anti-LIGHT antibody comprising a V_{H} comprising the amino acid sequence of any one of SEQ ID NOs: 46-61, 92-94 and a V_{L} comprising the amino acid sequence of any one of SEQ ID NOs: 62-73, 95-99.

In some embodiments, competition assays may be used to identify a monoclonal antibody that competes with an anti-LIGHT antibody described herein for binding to LIGHT. Competition assays can be used to determine whether two antibodies bind to the same epitope by recognizing identical or sterically overlapping epitopes or one antibody competitively inhibits binding of another antibody to the antigen. In certain embodiments, such a competing antibody binds to the same epitope that is bound by an antibody described herein. Exemplary competition assays include, but are not limited to, routine assays such as those provided in Harlow and Lane (1988) Antibodies: A Laboratory Manual ch.14 (Cold Spring Harbor Laboratory, Cold Spring Harbor, N.Y.). Detailed exemplary methods for mapping an epitope to which an antibody binds are provided in Morris (1996) "Epitope Mapping Protocols", in Methods in Molecular Biology vol. 66 (Humana Press, Totowa, N.J.). In some embodiments, two antibodies are said to bind to the same epitope if each blocks binding of the other by 50% or more. In some embodiments, the antibody that competes with an anti-LIGHT antibody described herein is a chimeric, humanized or human antibody.

Exemplary anti-LIGHT antibody sequences are shown in Tables 2 and 3, wherein the CDR numbering is according to the EU index of Kabat. Those skilled in the art will recognize that many algorithms are known for prediction of CDR positions and for delimitation of antibody heavy chain and light chain variable regions. Anti-LIGHT antibodies comprising CDRs, V_{H} and/or V_{L} sequences from antibodies described herein, but based on prediction algorithms other than those exemplified in the tables below, are within the scope of this invention.

**Table 2. Exemplary anti-LIGHT antibody CDR sequences**

| **Antibody Name** | **HC-CDR1** | **HC-CDR2** | **HC-CDR3** |
|---|---|---|---|
| LT-m37 | GYFIN (SEQ ID NO: 1) | RIYPYNVNTFYNQNFKG (SEQ ID NO: 8) | GTHYYGSSGAMDY (SEQ ID NO: 16) |
| hum_LT-m37-1 | | | |
| hum_LT-m37-2 | | | |
| hum_LT-m37-3 | | | |
| hum_LT-m37-4 | | | |
| hum_LT-m37-5 | | | |
| hum_LT-m37-6 | | | |
| hum_LT-m37-7 | | | |
| hum_LT-m37-8 | | | |
| hum_LT-m37-9 | | | |
| hum_LT-m37-10 | | | |
| hum_LT-m37-11 | | | |
| hum_LT-m37-12 | | | |
| hum_LT-m37-13 | GYFIN (SEQ ID NO: 1) | RIYPYNVDTFYNQNFKG (SEQ ID NO: 9) | GTHYYGSSGAMDY (SEQ ID NO: 16) |
| hum_LT-m37-14 | | | |
| hum_LT-m37-15 | | | |
| hum_LT-m37-16 | | | |
| Formula 1 | GYFIN (SEQ ID NO: 1) | RIYPYNVX₁TFYNQNFKG (SEQ ID NO: 45) wherein, X₁ is D or N | GTHYYGSSGAMDY (SEQ ID NO: 16) |
| LT-m17 | GYFMN (SEQ ID NO: 2) | RIYPYSGDTFYNQKFNN (SEQ ID NO: 10) | SEHFGRNYGTGAVDY (SEQ ID NO: 17) |
| LT-m18 | | | |
| LT-m33 | NYYVY (SEQ ID NO: 3) | EINPTNGDPNFNEKFKS (SEQ ID NO: 11) | SNWDYGNAMDF (SEQ ID NO: 18) |
| LT-m64 | | | |
| LT-m31 | NYGMN (SEQ ID NO: 4) | WINTYTGEPTYADDFKG (SEQ ID NO: 12) | DNWDGKGMDY (SEQ ID NO: 19) |
| LT-m55 | TYYMY (SEQ ID NO: 5) | EINPTNGGTIFNEKFKT (SEQ ID NO: 13) | GNWPYWSFDV (SEQ ID NO: 20) |
| LT-m58 | DHIMN (SEQ ID NO: 6) | RIYPVSGETNYNQKFMG (SEQ ID NO: 14) | GSYYWNAMDY (SEQ ID NO: 21) |
| LT-m85 | SYNVH (SEQ ID NO: 7) | AVYPGNGDTSYNQKFKG (SEQ ID NO: 15) | GSYYYTSSYFDH (SEQ ID NO: 22) |
| | | | |

| **Antibody Name** | **LC-CDR1** | **LC-CDR2** | **LC-CDR3** |
|---|---|---|---|
| LT-m37 | KASQNVGTAVA (SEQ ID NO: 23) | SASNRYT (SEQ ID NO: 30) | QQYSSYPYT (SEQ ID NO: 37) |
| hum_LT-m37-1 | | | |
| hum_LT-m37-2 | | | |
| hum_LT-m37-3 | | | |
| hum_LT-m37-4 | | | |
| hum_LT-m37-5 | | | |
| hum_LT-m37-6 | | | |
| hum_LT-m37-7 | | | |
| hum_LT-m37-8 | | | |
| hum_LT-m37-9 | | | |
| hum_LT-m37-10 | | | |
| hum_LT-m37-11 | | | |
| hum_LT-m37-12 | | | |
| hum_LT-m37-13 | | | |
| hum_LT-m37-14 | | | |
| hum_LT-m37-15 | | | |
| hum_LT-m37-16 | | | |
| LT-m17 | RSSQGLVHSNGNTYLH (SEQ ID NO: 24) | KVSNRFS (SEQ ID NO: 31) | SQSTHVPYT (SEQ ID NO: 38) |
| LT-m18 | RSSQSLVHSNGNTYLH (SEQ ID NO: 25) | KVSNRFS (SEQ ID NO: 31) | SQSTHVPLT (SEQ ID NO: 39) |
| LT-m33 | KASQSVDFDGDGYMN (SEQ ID NO: 26) | KASHLDS (SEQ ID NO: 32) | QQSIEDPLT (SEQ ID NO: 40) |
| LT-m64 | KASQSVDFDGDGYMN (SEQ ID NO: 26) | KASHLDS (SEQ ID NO: 32) | QQSIEDPFT (SEQ ID NO: 41) |
| LT-m31 | KASQDVDTAVA (SEQ ID NO: 27) | WASTRHT (SEQ ID NO: 33) | HQYGSYPLT (SEQ ID NO: 42) |
| LT-m55 | KASQSVDFDGDNYMN (SEQ ID NO: 28) | AASNLDS (SEQ ID NO: 34) | QQSIEDPWT (SEQ ID NO: 43) |
| LT-m58 | KASQSVDFDGDSYMN (SEQ ID NO: 29) | SASNLES (SEQ ID NO: 35) | QQSIEDPWT (SEQ ID NO: 43) |
| LT-m85 | KASQSVDFDGDSYMN (SEQ ID NO: 29) | TASNLES (SEQ ID NO: 36) | QQSYEDPFT (SEQ ID NO: 44) |

**Table 2A. Exemplary anti-LIGHT antibody CDR sequences**

| **Antibody Name** | **HC-CDR1** | **HC-CDR2** | **HC-CDR3** |
|---|---|---|---|
| LT-m58 | DHIMN (SEQ ID NO: 6) | RIYPVSGETNYNQKFMG (SEQ ID NO: 14) | GSYYWNAMDY (SEQ ID NO: 21) |
| hum_LT-m58-1 | | | |
| hum_LT-m58-2 | | | |
| hum_LT-m58-3 | | | |
| hum_LT-m58-4 | | | |
| hum_LT-m58-5 | | | |
| hum_LT-m58-6 | | | |
| | | | |

| **Antibody Name** | **LC-CDR1** | **LC-CDR2** | **LC-CDR3** |
|---|---|---|---|
| LT-m58 | KASQSVDFDGDSYMN (SEQ ID NO: 29) | SASNLES (SEQ ID NO: 35) | QQSIEDPWT (SEQ ID NO: 43) |
| hum_LT-m58-1 | | | |
| hum_LT-m58-2 | | | |
| hum_LT-m58-3 | | | |
| hum_LT-m58-4 | KASQSVDFDGESYMN (SEQ ID NO: 83) | | |
| hum_LT-m58-5 | | | |
| hum_LT-m58-6 | | | |

**Table 2B. Exemplary anti-LIGHT antibody CDR sequences**

| **Antibody Name** | **HC-CDR1** | **HC-CDR2** | **HC-CDR3** |
|---|---|---|---|
| LT-m85 | SYNVH (SEQ ID NO: 7) | AVYPGNGDTSYNQ KFKG (SEQ ID NO: 15) | GSYYYTSSYFDH (SEQ ID NO: 22) |
| hum_LT-m85-1 | | | |
| hum_LT-m85-2 | | | |
| hum_LT-m85-3 | | | |
| hum_LT-m85-4 | | | |
| hum_LT-m85-5 | | | |
| hum_LT-m85-6 | | | |
| hum_LT-m85-7 | | | |
| hum_LT-m85-8 | | | |
| hum_LT-m85-9 | | | |
| | | | |

| **Antibody Name** | **LC-CDR1** | **LC-CDR2** | **LC-CDR3** |
|---|---|---|---|
| LT-m85 | KASQSVDFDGDSYMN (SEQ ID NO: 29) | TASNLES (SEQ ID NO: 36) | QQSYEDPFT (SEQ ID NO: 44) |
| hum_LT-m85-1 | | | |
| hum_LT-m85-2 | | | |
| hum_LT-m85-3 | | | |
| hum_LT-m85-4 | | | |
| hum_LT-m85-5 | | | |
| hum_LT-m85-6 | | | |
| hum_LT-m85-7 | | | |
| hum_LT-m85-8 | | | |
| hum_LT-m85-9 | | | |

**Table 3. Exemplary sequences**

| **SEQ ID NO** | **Description** | **Sequence** |
|---|---|---|
| 46 | LT-m37 V_{H} | QVQLKQSGAELVRPGTSVKVSCKASGYSFTGYFINWVKQSHG KSLEWIGRIYPYNVNTFYNQNFKGRATLTADKSSSTAHMELLS LTSEDSAVYYCGRGTHYYGSSGAMDYWGQGTSVTVSS |
| 47 | hum_LT-m37-1 V_{H} | QVQLVQSGAEVKKPGASVKVSCKASGYSFTGYFINWVRQAP GQSLEWIGRIYPYNVNTFYNQNFKGRATLTADKSTSTAYMEL |
| | hum_LT-m37-5 V_{H} | |
| | hum_LT-m37-9 V_{H} | SSLRSEDTAVYYCGRGTHYYGSSGAMDYWGQGTLVTVSS |
| 48 | hum_LT-m37-2 V_{H} | QVQLVQSGAEVKKPGASVKVSCKASGYSFTGYFINWVRQAP GQGLEWIGRIYPYNVNTFYNQNFKGRATLTADKSTSTAYMEL SSLRSEDTAVYYCGRGTHYYGSSGAMDYWGQGTLVTVSS |
| | hum_LT-m37-6 V_{H} | |
| | hum_LT-m37-10 V_{H} | |
| 49 | hum_LT-m37-3 V_{H} | QVQLVQSGAEVKKPGASVKVSCKASGYSFTGYFINWVRQAP GQGLEWIGRIYPYNVNTFYNQNFKGRATMTADKSTSTAYME LSSLRSEDTAVYYCGRGTHYYGSSGAMDYWGQGTLVTVSS |
| | hum_LT-m37-7 V_{H} | |
| | hum_LT-m37-11 V_{H} | |
| 50 | hum_LT-m37-4 V_{H} | QVQLVQSGAEVKKPGASVKVSCKASGYSFTGYFINWVRQAP GQGLEWIGRIYPYNVNTFYNQNFKGRVTMTADKSTSTAYME LSSLRSEDTAVYYCGRGTHYYGSSGAMDYWGQGTLVTVSS |
| | hum_LT-m37-8 V_{H} | |
| | hum_LT-m37-12 V_{H} | |
| 51 | hum_LT-m37-13 V_{H} | QVQLVQSGAEVKKPGASVKVSCKASGYSFTGYFINWVRQAP GQGLEWIGRIYPYNVDTFYNQNFKGRVTMTADKSTSTAYME LSSLRSEDTAVYYCGRGTHYYGSSGAMDYWGQGTLVTVSS |
| | hum_LT-m37-16 V_{H} | |
| 52 | hum_LT-m37-14 V_{H} | QVQLVQSGAEVKKPGASVKVSCKASGYSFTGYFINWVRQAP GQSLEWIGRIYPYNVDTFYNQNFKGRATLTADKSTSTAYMEL SSLRSEDTAVYYCGRGTHYYGSSGAMDYWGQGTLVTVSS |
| 53 | hum_LT-m37-15 V_{H} | QVQLVQSGAEVKKPGASVKVSCKASGYSFTGYFINWVRQAP GQGLEWIGRIYPYNVDTFYNQNFKGRATMTADKSTSTAYME LSSLRSEDTAVYYCGRGTHYYGSSGAMDYWGQGTLVTVSS |
| 54 | LT-m17 V_{H} | QVQLQQSGPELVKPGASVKISCKASGYSFTGYFMNWVKQSH GKSLEWIGRIYPYSGDTFYNQKFNNKATLTVDKSSSTAHLELL SLTSEDSAVYYCGRSEHFGRNYGTGAVDYWGQGTSVTVSS |
| 55 | LT-m18 V_{H} | EVKLLESGPELVKPGASVKISCKASGYSFTGYFMNWVKQSHG KSLEWIGRIYPYSGDTFYNQKFNNKATLTVDKSSSTAHLELLS LTSEDSAVYYCGRSEHFGRNYGTGAVDYWGQGTSVTVSS |
| 56 | LT-m33 V_{H} | QVQLKQSGAELVKPGASVRLSCKTSGYTFTNYYVYWVKQRP GQGLEWIGEINPTNGDPNFNEKFKSKATLTVDKSSSTAYMHLS SLTSEDSAVYYCTRSNWDYGNAMDFWGQGTSVTVSS |
| 57 | LT-m64 V_{H} | EVKLEESGAELVKPGASVRLSCKTSGYTFTNYYVYWVKQRPG QGLEWIGEINPTNGDPNFNEKFKSKATLTVDKSSSTAYMHLSS LTSEDSAVYYCTRSNWDYGNAMDFWGQGTSVTVSS |
| 58 | LT-m31 V_{H} | QIQLLQSGPELEKPGETVKISCKASGYTFTNYGMNWVKQAPG KGLKWMGWINTYTGEPTYADDFKGRFAFSLETSASTAYLQIN NLKNEDTATYFCARDNWDGKGMDYWGQGTSVTVSS |
| 59 | LT-m55 V_{H} | EVQLQQSGAELVKPGASVKLSCKASGYTFATYYMYWVKQRP GQGLEWIGEINPTNGGTIFNEKFKTKATLTVDKSSSTAYMQLS SLTSEDSAVYYCARGNWPYWSFDVWGAGTSVTVSS |
| 60 | LT-m58 V_{H} | QVQLQQSGAEMASPGASVTLSCKASGYTFIDHIMNWVKKRPG QGLEWIGRIYPVSGETNYNQKFMGKATFSVDRSSSTVYMVLN SLTSEDPAVYYCGRGSYYWNAMDYWGQGTTLTVSS |
| 61 | LT-m85 V_{H} | EVQLQQSGAELVKPGASVKMSCKASGYTFTSYNVHWVKQTP GQGLEWIGAVYPGNGDTSYNQKFKGKATLTADKSSNTAYMQ LSSLTSEDSAVYYCARGSYYYTSSYFDHWGQGTTLTVSS |
| | | |
| 62 | LT-m37 V_{L} | DILLTQSHKFMSTSVGDRVSITCKASQNVGTAVAWYQQKPGQ SPKLLIYSASNRYTGVPDRFTGSGSGTDFTLTISNMQSEDLADY FCQQYSSYPYTFGGGTKLEIK |
| 63 | hum_LT-m37-1 V_{L} | EIVLTQSPATLSVSPGERATLSCKASQNVGTAVAWYQQKPGQ SPRLLIYSASNRYTGIPARFSGSGSGTEFTLTISSLQSEDFAVYF |
| | hum_LT-m37-2 V_{L} | |
| | hum_LT-m37-3 V_{L} | CQQYSSYPYTFGGGTKVEIK |
| | hum_LT-m37-4 V_{L} | |
| | hum_LT-m37-13 V_{L} | |
| 64 | hum_LT-m37-5 V_{L} | EIVMTQSPATLSVSPGERATLSCKASQNVGTAVAWYQQKPGQ SPRLLIYSASNRYTGIPARFSGSGSGTEFTLTISSLQSEDFAVYF CQQYSSYPYTFGGGTKVEIK |
| | hum_LT-m37-6 V_{L} | |
| | hum_LT-m37-7 V_{L} | |
| | hum_LT-m37-8 V_{L} | |
| 65 | hum_LT-m37-9 V_{L} | EIVLTQSPATLSVSPGERATLSCKASQNVGTAVAWYQQKPGQ SPRLLIYSASNRYTGIPARFSGSGSGTEFTLTISSLQSEDFAVYY CQQYSSYPYTFGGGTKVEIK |
| | hum_LT-m37-10 V_{L} | |
| | hum_LT-m37-11 V_{L} | |
| | hum_LT-m37-12 V_{L} | |
| | hum_LT-m37-14 V_{L} | |
| | hum_LT-m37-15 V_{L} | |
| | hum_LT-m37-16 V_{L} | |
| 66 | LT-m17 V_{L} | DVVMTQTPLSLPVSLGDQASISCRSSQGLVHSNGNTYLHWYL QKPGQSPKLLIYKVSNRFSGVPDRFSGSGSGTDFTLKISRVEAE DLGIYFCSQSTHVPYTFGGGTKLEIK |
| 67 | LT-m18 V_{L} | DIVLTQSPLSLPVSLGDQASISCRSSQSLVHSNGNTYLHWYLQ KPGQSPKLLIYKVSNRFSGVPDRFSGSGSGTDFTLKISRVEAED LGVYFCSQSTHVPLTFGDGTRLEIK |
| 68 | LT-m33 V_{L} | DIVMTQSPASLAVSLGQRATISCKASQSVDFDGDGYMNWYQ QKPGQPPKLLIYKASHLDSGIPARFSGSGSGTDFTLNIHPVEEE DAATYYCQQSIEDPLTFGDGTRLEIK |
| 69 | LT-m64 V_{L} | DIVMTQSPASLAVSLGQRATISCKASQSVDFDGDGYMNWYQ QKPGQPPKLLIYKASHLDSGIPARFSASGSGTDFTLNIHPVEEE DAATYYCQQSIEDPFTFGSGTKLEIK |
| 70 | LT-m31 V_{L} | DIVLTQSPKFMSTSVGDRVSITCKASQDVDTAVAWYQQKPGH SPKLLLYWASTRHTGVPDRFTGSGSGTDFTLTISNVQSEDLAD YFCHQYGSYPLTFGAGTKLELK |
| 71 | LT-m55 V_{L} | DIVLTQSPASLAVSLGQRATISCKASQSVDFDGDNYMNWYQQ KPGQPPKLLIYAASNLDSGIPARFSASGSGTDFTLNIHPVEEED AATYYCQQSIEDPWTFGGGTKLEIK |
| 72 | LT-m58 V_{L} | DIVMTQSPASLAVSLGQRATISCKASQSVDFDGDSYMNWYQQ KPGQPPKLLIYSASNLESGIPARFSGSGSGTDFTLNIHPVEEEDA ATYYCQQSIEDPWTFGGGTKLEIK |
| 73 | LT-m85 V_{L} | DIVLTQSPASLAVSLGQRATISCKASQSVDFDGDSYMNWYQQ KPGQPPKLLIYTASNLESGIPARFTGSGSGTDFTLNIHPVEEEDA ATYYCQQSYEDPFTFGSGTKLEIK |
| 74 | IgG1 heavy chain constant region | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSREEMTKNQVSLTCLVKGF YPSDIAVEWESNGQPENNYKTTPPVLDSDGSFFLYSKLTVDKS RWQQGNVFSCSVMHEALHNHYTQKSLSLSPGK |
| 75 | IgG4 heavy chain constant region | ASTKGPSVFPLAPCSRSTSESTAALGCLVKDYFPEPVTVSWNS GALTSGVHTFPAVLQSSGLYSLSSVVTVPSSSLGTKTYTCNVD HKPSNTKVDKRVESKYGPPCPSCPAPEFLGGPSVFLFPPKPKDT |
| | | LMISRTPEVTCVVVDVSQEDPEVQFNWYVDGVEVHNAKTKP REEQFNSTYRVVSVLTVLHQDWLNGKEYKCKVSNKGLPSSIE KTISKAKGQPREPQVYTLPPSQEEMTKNQVSLTCLVKGFYPSD IAVEWESNGQPENNYKTTPPVLDSDGSFFLYSRLTVDKSRWQ EGNVFSCSVMHEALHNHYTQKSLSLSLGK |
| 76 | Light chain constant region (kappa) | |
| 77 | Light chain constant region (lambda) | GQPKAAPSVTLFPPSSEELQANKATLVCLISDFYPGAVTVAWK ADSSPVKAGVETTTPSKQSNNKYAASSYLSLTPEQWKSHRSY SCQVTHEGSTVEKTVAPTECS |
| 78 | Native human LIGHT | RDGPAGSWEQLIQERRSHEVNPAAHLTGANSSLTGSGGPLLW ETQLGLAFLRGLSYHDGALVVTKAGYYYIYSKVQLGGVGCPL GLASTITHGLYKRTPRYPEELELLVSQQSPCGRATSSSRVWWD SSFLGGVVHLEAGEEVVVRVLDERLVRLRDGTRSYFGAFMV |
| 79 | His-huLIGHT | HHHHHHHHHHGGGGSGGGGSGGGGSIEGRDGPAGSWEQLIQ ERRSHEVNPAAHLTGANSSLTGSGGPLLWETQLGLAFLRGLS YHDGALVVTKAGYYYIYSKVQLGGVGCPLGLASTITHGLYKR TPRYPEELELLVSQQSPCGRATSSSRVWWDSSFLGGVVHLEA GEEVVVRVLDERLVRLRDGTRSYFGAFMV |
| 80 | hFc-huLIGHT | DKTHTCPPCPAPELLGGPSVFLFPPKPKDTLMISRTPEVTCVVV DVSHEDPEVKFNWYVDGVEVHNAKTKPREEQYGSTYRVVSV LTVLHQDWLNGKEYKCKVSNKALPAPIEKTISKAKGQPREPQ VYTLPPSREEMTKNQVSLTCLVKGFYPSDIAVEWESNGQPEN NYKTTPPVLDSDGSFFLYSKLTVDKSRWQQGNVFSCSVMHEA LHNHYTQKSLSLSPGGGGGSGGGGSGGGGSIEGRDGPAGSWE QLIQERRSHEVNPAAHLTGANSSLTGSGGPLLWETQLGLAFLR GLSYHDGALVVTKAGYYYIYSKVQLGGVGCPLGLASTITHGL YKRTPRYPEELELLVSQQSPCGRATSSSRVWWDSSFLGGVVH LEAGEEVVVRVLDERLVRLRDGTRSYFGAFMV |
| 81 | His-cynoLIGHT | HHHHHHHHHHGGGGSGGGGSGGGGSIEGRDGHGGSWEQLIQ ERRSHQNNPAAHLTGANSSLTGSGGPLLWETQLGLAFLRGLD YHDGALVANKAGYYYIYSKVQLGGVGCPLGLASTITHGLYK RTPRYPEELELLVSQQSPCGRATSNSRVWWDSSFLGGVVHLE VGEEVVVRVLDERLVRLRDGTRSYFGAFMV |
| 82 | His-musLIGHT | HHHHHHHHHGGGGSGGGGSGGGGSIEGRDGGKGSWEKLIQ DQRSHQANPAAHLTGANASLIGIGGPLLWETRLGLAFLRGLT YHDGALVTMEPGYYYVYSKVQLSGVGCPQGLANGLPITHGL YKRTSRYPKELELLVSRRSPCGRANSSRVWWDSSFLGGVVHL EAGEEVVVRVPGNRLVRPRDGTRSYFGAFMV |

**Table 3A. Exemplary sequences**

| **SEQ ID NO** | **Description** | **Sequence** |
|---|---|---|
| 60 | LT-m58 V_{H} | QVQLQQSGAEMASPGASVTLSCKASGYTFIDHIMNWVKKRP GQGLEWIGRIYPVSGETNYNQKFMGKATFSVDRSSSTVYMV LNSLTSEDPAVYYCGRGSYYWNAMDYWGQGTTLTVSS |
| 84 | hum_LT-m58-1 V_{H} | QVQLVQSGAEVKKPGASVKVSCKASGYTFIDHIMNWVRKAP GQGLEWMGRIYPVSGETNYNQKFMGRATFTVDRSTSTVYM |
| | hum_LT-m58-3 V_{H} | |
| | hum_LT-m58-4 V_{H} | ELSSLRSEDTAVYYCGRGSYYWNAMDYWGQGTLVTVSS |
| | hum_LT-m58-6 V_{H} | |
| 85 | hum_LT-m58-2 V_{H} | ELSSLRSEDTAVYYCGRGSYYWNAMDYWGQGTLVTVSS QVQLVQSGAEVKKPGASVKVSCKASGYTFIDHIMNWVRKAP GQGLEWIGRIYPVSGETNYNQKFMGRVTFTVDRSTSTVYME LSSLRSEDTAVYYCGRGSYYWNAMDYWGQGTLVTVSS |
| | hum_LT-m58-5 V_{H} | |
| | | |
| 72 | LT-m58 V_{L} | DIVMTQSPASLAVSLGQRATISCKASQSVDFDGDSYMNWYQ QKPGQPPKLLIYSASNLESGIPARFSGSGSGTDFTLNIHPVEEE DAATYYCQQSIEDPWTFGGGTKLEIK |
| 86 | hum_LT-m58-1 V_{L} | EIVMTQSPATLSVSPGERATLSCKASQSVDFDGDSYMNWYQ QKPGQPPRLLIYSASNLESGIPARFSGSGSGTEFTLTISSLQSED FAVYYCQQSIEDPWTFGQGTKLEIK |
| 87 | hum_LT-m58-2 V_{L} | EIVMTQSPATLSVSPGERATLSCKASQSVDFDGDSYMNWYQ QKPGQAPRLLIYSASNLESGIPARFSGSGSGTEFTLTISSLQSE DFAVYYCQQSIEDPWTFGQGTKVEIK |
| 88 | hum_LT-m58-3 V_{L} | DIVMTQSPASLAVSLGQRATISCKASQSVDFDGDSYMNWYQ QKPGQPPKLLIYSASNLESGIPARFSGSGSGTDFTLNIHPVEEE DAATYYCQQSIEDPWTFGGGTKLEIK EIVMTQSPATLSVSPGERATLSCKASQSVDFDGDSYMNWYQ QKPGQPPRLLIYSASNLESGIPARFSGSGSGTEFTLTISSLQSED FAVYYCQQSIEDPWTFGQGTKLEIK EIVMTQSPATLSVSPGERATLSCKASQSVDFDGDSYMNWYQ QKPGQAPRLLIYSASNLESGIPARFSGSGSGTEFTLTISSLQSE DFAVYYCQQSIEDPWTFGQGTKVEIK DIVMTQSPDSLAVSLGERATINCKASQSVDFDGDSYMNWYQ QKPGQPPKLLIYSASNLESGVPDRFSGSGSGTDFTLTISSLQAE DVAVYYCQQSIEDPWTFGQGTKVEIK EIVMTQSPATLSVSPGERATLSCKASQSVDFDGESYMNWYQ QKPGQPPRLLIYSASNLESGIPARFSGSGSGTEFTLTISSLQSED FAVYYCQQSIEDPWTFGQGTKLEIK EIVMTQSPATLSVSPGERATLSCKASQSVDFDGESYMNWYQ QKPGQAPRLLIYSASNLESGIPARFSGSGSGTEFTLTISSLQSE DFAVYYCQQSIEDPWTFGQGTKVEIK DIVMTQSPDSLAVSLGERATINCKASQSVDFDGESYMNWYQ QKPGQPPKLLIYSASNLESGVPDRFSGSGSGTDFTLTISSLQAE DVAVYYCQQSIEDPWTFGQGTKVEIK |
| 89 | hum_LT-m58-4 V_{L} | EIVMTQSPATLSVSPGERATLSCKASQSVDFDGESYMNWYQ QKPGQPPRLLIYSASNLESGIPARFSGSGSGTEFTLTISSLQSED FAVYYCQQSIEDPWTFGQGTKLEIK |
| 90 | hum_LT-m58-5 V_{L} | EIVMTQSPATLSVSPGERATLSCKASQSVDFDGESYMNWYQ QKPGQAPRLLIYSASNLESGIPARFSGSGSGTEFTLTISSLQSE DFAVYYCQQSIEDPWTFGQGTKVEIK |
| 91 | hum_LT-m58-6 V_{L} | DIVMTQSPDSLAVSLGERATINCKASQSVDFDGESYMNWYQ QKPGQPPKLLIYSASNLESGVPDRFSGSGSGTDFTLTISSLQAE DVAVYYCQQSIEDPWTFGQGTKVEIK |

**Table 3B. Exemplary sequences**

| **SEQ ID NO** | **Description** | **Sequence** |
|---|---|---|
| 61 | LT-m85 V_{H} | EVQLQQSGAELVKPGASVKMSCKASGYTFTSYNVHWVKQT PGQGLEWIGAVYPGNGDTSYNQKFKGKATLTADKSSNTAY MQLSSLTSEDSAVYYCARGSYYYTSSYFDHWGQGTTLTVSS |
| 92 | hum_LT-m85-1 V_{H} | QVQLVQSGAEVKKPGASVKVSCKASGYTFTSYNVHWVRQA PGQGLEWIGAVYPGNGDTSYNQKFKGRATLTADKSTSTVY MELSSLRSEDTAVYYCARGSYYYTSSYFDHWGQGTLVTVSS |
| | hum_LT-m85-3 V_{H} | |
| | hum_LT-m85-7 V_{H} | |
| | hum_LT-m85-9 V_{H} | |
| 93 | hum_LT-m85-2 V_{H} | QVQLVQSGAEVKKPGASVKVSCKASGYTFTSYNVHWVRQA PGQGLEWMGAVYPGNGDTSYNQKFKGRATLTADKSTSTAY MELSSLRSEDTAVYYCARGSYYYTSSYFDHWGQGTLVTVSS |
| | hum_LT-m85-6 V_{H} | |
| 94 | hum_LT-m85-4 V_{H} | QVQLVQSGAEVKKPGASVKVSCKASGYTFTSYNVHWVRQA PGQGLEWIGAVYPGNGDTSYNQKFKGRATLTADKSTSTAY MELSSLRSEDTAVYYCARGSYYYTSSYFDHWGQGTLVTVSS |
| | hum_LT-m85-5 V_{H} | |
| | hum_LT-m85-8 V_{H} | |
| | | |
| 73 | LT-m85 V_{L} | DIVLTQSPASLAVSLGQRATISCKASQSVDFDGDSYMNWYQ QKPGQPPKLLIYTASNLESGIPARFTGSGSGTDFTLNIHPVEEE DAATYYCQQSYEDPFTFGSGTKLEIK |
| 95 | hum_LT-m85-1 V_{L} | EIVLTQSPATLSVSPGERATLSCKASQSVDFDGDSYMNWYQ |
| | | QKPGQPPRLLIYTASNLESGIPARFSGSGSGTEFTLTISSLQSED FAVYYCQQSYEDPFTFGQGTKLEIK |
| 96 | hum_LT-m85-2 V_{L} | EIVMTQSPATLSVSPGERATLSCKASQSVDFDGDSYMNWYQ QKPGQPPRLLIYTASNLESGIPARFSGSGSGTEFTLTISSLQSED FAVYYCQQSYEDPFTFGQGTKLEIK |
| | hum_LT-m85-3 V_{L} | |
| 97 | hum_LT-m85-4 V_{L} | EIVMTQSPATLSVSPGERATLSCKASQSVDFDGDSYMNWYQ QKPGQAPRLLIYTASNLESGIPARFSGSGSGTEFTLTISSLQSE DFAVYYCQQSYEDPFTFGQGTKLEIK |
| 98 | hum_LT-m85-5 V_{L} | DIVLTQSPDSLAVSLGERATINCKASQSVDFDGDSYMNWYQ QKPGQPPKLLIYTASNLESGIPDRFSGSGSGTDFTLTISSLQAE DVAVYYCQQSYEDPFTFGQGTKVEIK |
| | hum_LT-m85-6 V_{L} | |
| | hum_LT-m85-7 V_{L} | |
| 99 | hum_LT-m85-8 V_{L} | DIVMTQSPDSLAVSLGERATINCKASQSVDFDGDSYMNWYQ QKPGQPPKLLIYTASNLESGVPDRFSGSGSGTDFTLTISSLQAE DVAVYYCQQSYEDPFTFGQGTKVEIK |
| | hum_LT-m85-9 V_{L} | |

### LIGHT

LIGHT (lymphotoxin-like, exhibits inducible expression, and competes with herpes simplex virus glycoprotein D for HVEM, a receptor expressed by T lymphocytes), as a member of the tumor necrosis factor superfamily (TNFSF), is a type II transmembrane protein containing 240 amino acids, forming a homotrimer on the cell surface. LIGHT is also known as tumor necrosis factor superfamily member 14 (TNFSF14), TL5, LTg, HVEML, or CD258. LIGHT is a T cell co-stimulate molecule that can induce T cell proliferation and cytokine production (Tamada et al., 2000, Nat Med). LIGHT can also induce inflammatory responses in monocytes and endothelial cells (Otterda et al., 2006, Blood*;* Chang et al., 2005, J Biomed Sci)*.* LIGHT binds to three different receptors expressed on different cell types: the herpesvirus entry regulator HVEM expressed on T and B cells, the lymphotoxin beta receptor LTβR and the decoy receptor 3 expressed on stromal cells and non-hematopoietic cells. LIGHT on dendritic cells and T cells enhances T cell proliferation and cytokine production. LIGHT can directly synergistically stimulate T cell responses. The role of LIGHT in various inflammatory diseases and conditions has been demonstrated through the use of LIGHT deficient models and various models of LIGHT overexpressing transgenic animals. Overexpression of LIGHT in mice leads to excessive production of activated peripheral T cell populations and spontaneous development of severe autoimmune diseases (Wang et al., 2001, J Clin Invest.)*.*

### LIGHT receptor (LIGHTR)

LIGHT mediates the biological effects by binding to three TNF superfamily receptors, including lymphotoxin β receptor (LTβR) (Crowe et al., 1994, Science 264, 707-10, Browning et al., 1997, J Immunol 159, 3288-98), herpes simplex virus entry mediator (HVEM) (Montgomery et al., 1996, Cell 87 (3) 427-36), and decoy receptor 3 (DcR3) (Yu et al., 1999, J Biol Chem 274, 13733-6).

The lymphotoxin beta receptor (LTβR) signal coordinates lymphocyte neogenesis and subsequent tertiary lymphoid structure (TLS), and is associated with severe chronic inflammatory diseases in multiple organ systems. Conlon TM et al. demonstrated that inhibiting the LTβR signaling pathway can activate Wnt induced lung regeneration (Conlon et al., 2020, Nature 588 (7836): 151-156). This study demonstrated that ligands of LTβR, including LTα, LTβ, and LIGHT, were upregulated on adaptive and innate immune cells in lung epithelial cells of smoking related chronic obstructive pulmonary disease (COPD) patients and mice exposed to chronic cigarette smoke. The atypical NF-κB signaling pathway was enhanced, and the expression of LTβR target genes was enriched. Therapeutic inhibition of the LTβR signaling pathway through LTβR-Ig peptide in young and old mice can disrupt smoking induced bronchial associated lymphoid tissue (iBALT), induce lung tissue regeneration, and restore airway fibrosis and systemic muscle atrophy. Mechanistically, blockade of LTβR signal inhibits the activation of atypical NF-κB in the epithelium, reduces TGFβ signal transduction in the airway, and induces regeneration by preventing epithelial cell death and activating Wnt/β-catenin signal transduction in alveolar epithelial precursor cells.

LIGHT on activated T cells can directly transmit signals to other T cells expressing HVEM, or indirectly stimulate these helper cells to secrete pro-inflammatory cytokines through HVEM and/or LTβR expressed on immature DC or SC cells. An increasing amount of experimental evidence suggests that LIGHT expressed on DCs or T cells can co-stimulate and enhance T cell proliferation and cytokine secretion (Tamada. K et al., 2000, J. Immunol. 164, 4105-4110). Consistent with these reports, the addition of LIGHT Ig recombinant fusion protein to T cells can activate NF-κB and AP-1, co-stimulate T cell proliferation, and secretion of IFN-γ (Granger et al., 2003, Cytokine Growth Factor Rev. 14, 289-296). This enhanced T cell activation is mediated by HVEM signaling, as LTβR is not expressed on mature T cells. The interaction between LIGHT and HVEM expressed in other cell types can also trigger various functional activities. For example, soluble or membrane-bound LIGHT expressed on activated T cells, when in contact with HVEM expressed on immature DC cells, can induce DC cell maturation and enhance allogeneic stimulatory activity when CD40L-Ig is added (Morel et al., 2001, J. Immunol. 167, 2479-2486).

Studies have shown that transgenic mice expressing constitutive expression of LIGHT are detected to have a hyperactive T cell population, which increases the risk of autoimmunity in mice. This is characterized by severe infiltration of effector cells in the peripheral tissues of mice (Wang et al., 2005, J Immunol 174 8173-82; Shaikh et al., 2001, J Immunol 167 6330-7; Wang et al., 2001, J Immunal 1675099-105; Wang et al., 2004, J Clin Invest 113 826-35). The interaction between HVEM-LIGHT can promote the pathogenesis of graft-versus-host disease (GVHD). Transplanting allogeneic T cells from hvem^{-/-} or light^{-/-} mice into recipient mice can induce weakened anti host CTL responses, thereby prolonging the survival of recipient mice. After transferring HVEM^{-/-} and Light^{-/-} donor T cells into recipient mice, apoptosis occurs. Therefore, the administration of mAbs against HVEM can inhibit the interaction between HVEM-LIGHT, improve GVHD response, and prolong the survival of recipient mice (Xu et al., 2007, Blood 109:4097-4104). When mesenteric lymph node cells from LIGHT transgenic animals are transferred to RAG^{-/-}, lymphocytes expressing LIGHT can induce IBD like symptoms, such as cytokine features of human Crohn's disease, peptic ulcer, ileitis, and increased colon IFN-γ and TNF (Wang et al., 2005, J Immunol 174 8173-82). In the T-cell dependent allergic pneumonia model, the interaction between HVEM-LIGHT plays a dominant role in the survival of pathogenic memory Th2 cells. Interrupting the interaction between HVEM-LIGHT inhibits the accumulation of inflammatory memory Th2 cells in the lungs. Although light^{-/-} donor CD4+T cells cannot be sustained after exposure to antigens in vivo, co transfer of wild-type and light^{-/-} donor T cells can rescue the survival defects of light^{-/-} donor T cells, indicating that HVEM induces cell survival signals into T cells by interacting with LIGHT expressed on adjacent antigen-specific T cells. This study suggests that HVEM expressed by CD4+T cells can induce cell survival signals and control T cell-driven allergic pneumonia (Soroosh et al., 2011, J Exp Med 208:797-809).

DcR3, also known as the tumor necrosis factor receptor superfamily member 6B (TNFRSF6B), is a functional attenuator for the LIGHT signal, found in the human genome but not present in mice and rats. It is a molecule lacking membrane anchoring, acting as a soluble inhibitory factor by binding to LIGHT, Fas ligand, and TL1A. In humans, DcR3 binds to LIGHT and inhibits the interaction between HVEM and LIGHT (Jiang M. 2016, Med SciMonit. 22:1850-7).

### Full-length anti-LIGHT antibody

The anti-LIGHT antibody in some embodiments is a full-length anti-LIGHT antibody. In some embodiments, the full-length anti-LIGHT antibody is an IgA, IgD, IgE, IgG, or IgM. In some embodiments, the full-length anti-LIGHT antibody comprises IgG constant domains, such as constant domains of any one of IgG1, IgG2, IgG3, and IgG4 including variants thereof. In some embodiments, the full-length anti-LIGHT antibody comprises a lambda light chain constant region. In some embodiments, the full-length anti-LIGHT antibody comprises a kappa light chain constant region. In some embodiments, the full-length anti-LIGHT antibody is a full-length human anti-LIGHT antibody. In some embodiments, the full-length anti-LIGHT antibody comprises an Fc sequence of a mouse immunoglobulin. In some embodiments, the full-length anti-LIGHT antibody comprises an Fc sequence that has been altered or otherwise changed so that it has enhanced antibody dependent cellular cytotoxicity (ADCC) or complement dependent cytotoxicity (CDC) effector function.

Thus, for example, in some embodiments, there is provided a full-length anti-LIGHT antibody comprising IgG1 constant domains, wherein the anti-LIGHT antibody specifically binds to LIGHT. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG2 constant domains, wherein the anti-LIGHT antibody specifically binds to LIGHT. In some embodiments, the IgG2 is human IgG2. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a full-length anti-LIGHT antibody comprising IgG3 constant domains, wherein the anti-LIGHT antibody specifically binds to LIGHT. In some embodiments, the IgG3 is human IgG3. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody specifically binds to LIGHT. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of any one of SEQ ID NOs: 1-7, or a variant thereof comprising up to about 3 (such as about any of 1, 2, or 3) amino acid substitutions, an HC-CDR2 comprising the amino acid sequence of any one of SEQ ID NOs: 8-15, or a variant thereof comprising up to about 3 (such as about any of 1, 2, or 3) amino acid substitutions, and an HC-CDR3 comprising the amino acid sequence of any one of SEQ ID NOs: 16-22, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 23-29, 83 or a variant thereof comprising up to about 3 (such as about any of 1, 2, or 3) amino acid substitutions, an LC-CDR2 comprising the amino acid sequence of any one of SEQ ID NOs: 30-36, or a variant thereof comprising up to about 3 (such as about any of 1, 2, or 3) amino acid substitutions, and an LC-CDR3 comprising the amino acid sequence of any one of SEQ ID NOs: 37-44, or a variant thereof comprising up to about 3 (such as about any of 1, 2, or 3) amino acid substitutions. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG2 constant domains, wherein the anti-LIGHT antibody comprises a) a heavy chain variable domain comprising an HC-CDR1 comprising the amino acid sequence of any one of SEQ ID NOs: 1-7, or a variant thereof comprising up to about 3 (such as about any of 1, 2, or 3) amino acid substitutions, an HC-CDR2 comprising the amino acid sequence of any one of SEQ ID NOs: 8-15, or a variant thereof comprising up to about 3 (such as about any of 1, 2, or 3) amino acid substitutions, and an HC-CDR3 comprising the amino acid sequence of any one of SEQ ID NOs: 16-22, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 23-29, 83 or a variant thereof comprising up to about 3 (such as about any of 1, 2, or 3) amino acid substitutions, an LC-CDR2 comprising the amino acid sequence of any one of SEQ ID NOs: 30-36, or a variant thereof comprising up to about 3 (such as about any of 1, 2, or 3) amino acid substitutions, and an LC-CDR3 comprising the amino acid sequence of any one of SEQ ID NOs: 37-44, or a variant thereof comprising up to about 3 (such as about any of 1, 2, or 3) amino acid substitutions. In some embodiments, the IgG2 is human IgG2. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a full-length anti-LIGHT antibody comprising IgG3 constant domains, wherein the anti-LIGHT antibody comprises a) a heavy chain variable domain comprising an HC-CDR1 comprising the amino acid sequence of any one of SEQ ID NOs: 1-7, or a variant thereof comprising up to about 3 (such as about any of 1, 2, or 3) amino acid substitutions, an HC-CDR2 comprising the amino acid sequence of any one of SEQ ID NOs: 8-15, or a variant thereof comprising up to about 3 (such as about any of 1, 2, or 3) amino acid substitutions, and an HC-CDR3 comprising the amino acid sequence of any one of SEQ ID NOs: 16-22, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 23-29, 83 or a variant thereof comprising up to about 3 (such as about any of 1, 2, or 3) amino acid substitutions, an LC-CDR2 comprising the amino acid sequence of any one of SEQ ID NOs: 30-36, or a variant thereof comprising up to about 3 (such as about any of 1, 2, or 3) amino acid substitutions, and an LC-CDR3 comprising the amino acid sequence of any one of SEQ ID NOs: 37-44, or a variant thereof comprising up to about 3 (such as about any of 1, 2, or 3) amino acid substitutions. In some embodiments, the IgG3 is human IgG3. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of any one of SEQ ID NOs: 1-7, or a variant thereof comprising up to about 3 (such as about any of 1, 2, or 3) amino acid substitutions, an HC-CDR2 comprising the amino acid sequence of any one of SEQ ID NOs: 8-15, or a variant thereof comprising up to about 3 (such as about any of 1, 2, or 3) amino acid substitutions, and an HC-CDR3 comprising the amino acid sequence of any one of SEQ ID NOs: 16-22, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 23-29, 83 or a variant thereof comprising up to about 3 (such as about any of 1, 2, or 3) amino acid substitutions, an LC-CDR2 comprising the amino acid sequence of any one of SEQ ID NOs: 30-36, or a variant thereof comprising up to about 3 (such as about any of 1, 2, or 3) amino acid substitutions, and an LC-CDR3 comprising the amino acid sequence of any one of SEQ ID NOs: 37-44, or a variant thereof comprising up to about 3 (such as about any of 1, 2, or 3) amino acid substitutions. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of any one of SEQ ID NOs: 1-7, an HC-CDR2 comprising the amino acid sequence of any one of SEQ ID NOs: 8-15, and an HC-CDR3 comprising the amino acid sequence of any one of SEQ ID NOs: 16-22, or a variant thereof comprising up to about 5 (such as about any of 1, 2, 3, 4, or 5) amino acid substitutions in the HC-CDR sequences; and b) a light chain variable domain comprising an LC-CDR1 comprising the amino acid sequence of any one of SEQ ID NOs: 23-29, 83, an LC-CDR2 comprising the amino acid sequence of any one of SEQ ID NOs: 30-36, and an LC-CDR3 comprising the amino acid sequence of any one of SEQ ID NOs: 37-44, or a variant thereof comprising up to about 5 (such as about any of 1, 2, 3, 4, or 5) amino acid substitutions in the LC-CDR sequences. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of any one of SEQ ID NOs: 1-7, an HC-CDR2 comprising the amino acid sequence of any one of SEQ ID NOs: 8-15, and an HC-CDR3 comprising the amino acid sequence of any one of SEQ ID NOs: 16-22, or a variant thereof comprising up to about 5 (such as about any of 1, 2, 3, 4, or 5) amino acid substitutions in the HC-CDR sequences; and b) a light chain variable domain comprising an LC-CDR1 comprising the amino acid sequence of any one of SEQ ID NOs: 23-29, 83, an LC-CDR2 comprising the amino acid sequence of any one of SEQ ID NOs: 30-36, and an LC-CDR3 comprising the amino acid sequence of any one of SEQ ID NOs: 37-44, or a variant thereof comprising up to about 5 (such as about any of 1, 2, 3, 4, or 5) amino acid substitutions in the LC-CDR sequences. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of any one of SEQ ID NOs: 1-7, an HC-CDR2 comprising the amino acid sequence of any one of SEQ ID NOs: 8-15, and an HC-CDR3 comprising the amino acid sequence of any one of SEQ ID NOs: 16-22; and b) a light chain variable domain comprising an LC-CDR1 comprising the amino acid sequence of any one of SEQ ID NOs: 23-29, 83, an LC-CDR2 comprising the amino acid sequence of any one of SEQ ID NOs: 30-36, and an LC-CDR3 comprising the amino acid sequence of any one of SEQ ID NOs: 37-44. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of any one of SEQ ID NOs: 1-7, an HC-CDR2 comprising the amino acid sequence of any one of SEQ ID NOs: 8-15, and an HC-CDR3 comprising the amino acid sequence of any one of SEQ ID NOs: 16-22; and b) a light chain variable domain comprising an LC-CDR1 comprising the amino acid sequence of any one of SEQ ID NOs: 23-29, 83, an LC-CDR2 comprising the amino acid sequence of any one of SEQ ID NOs: 30-36, and an LC-CDR3 comprising the amino acid sequence of any one of SEQ ID NOs: 37-44. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 1, an HC-CDR2 comprising the amino acid sequence of SEQ ID NO: 8, and an HC-CDR3 comprising the amino acid sequence of SEQ ID NO: 16; and b) a light chain variable domain comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 16; and b) a light chain variable domain comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and b) a light chain variable domain comprising an LC-CDR1 comprising the amino acid sequence of SEQ ID NO: 24, an LC-CDR2 comprising the amino acid sequence of SEQ ID NO: 31, and an LC-CDR3 comprising the amino acid sequence of SEQ ID NO: 38. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and b) a light chain variable domain comprising an LC-CDR1 comprising the amino acid sequence of SEQ ID NO: 25, an LC-CDR2 comprising the amino acid sequence of SEQ ID NO: 31, and an LC-CDR3 comprising the amino acid sequence of SEQ ID NO: 39. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and b) a light chain variable domain comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 40. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and b) a light chain variable domain comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 41. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 4, an HC-CDR2 comprising the amino acid sequence of SEQ ID NO: 12, and an HC-CDR3 comprising the amino acid sequence of SEQ ID NO: 19; and b) a light chain variable domain comprising an LC-CDR1 comprising the amino acid sequence of SEQ ID NO: 27, an LC-CDR2 comprising the amino acid sequence of SEQ ID NO: 33, and an LC-CDR3 comprising the amino acid sequence of SEQ ID NO: 42. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 5, an HC-CDR2 comprising the amino acid sequence of SEQ ID NO: 13, and an HC-CDR3 comprising the amino acid sequence of SEQ ID NO: 20; and b) a light chain variable domain comprising an LC-CDR1 comprising the amino acid sequence of SEQ ID NO: 28, an LC-CDR2 comprising the amino acid sequence of SEQ ID NO: 34, and an LC-CDR3 comprising the amino acid sequence of SEQ ID NO: 43. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and b) a light chain variable domain comprising an LC-CDR1 comprising the amino acid sequence of SEQ ID NO: 29, an LC-CDR2 comprising the amino acid sequence of SEQ ID NO: 35, and an LC-CDR3 comprising the amino acid sequence of SEQ ID NO: 43. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and b) a light chain variable domain comprising an LC-CDR1 comprising the amino acid sequence of SEQ ID NO: 83, an LC-CDR2 comprising the amino acid sequence of SEQ ID NO: 35, and an LC-CDR3 comprising the amino acid sequence of SEQ ID NO: 43. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a) a heavy chain variable domain comprising an HC-CDR1 comprising the amino acid sequence of SEQ ID NO: 7, an HC-CDR2 comprising the amino acid sequence of SEQ ID NO: 15, and an HC-CDR3 comprising the amino acid sequence of SEQ ID NO: 22; and b) a light chain variable domain comprising an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 1, an HC-CDR2 comprising the amino acid sequence of SEQ ID NO: 8, and an HC-CDR3 comprising the amino acid sequence of SEQ ID NO: 16; and b) a light chain variable domain comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 16; and b) a light chain variable domain comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and b) a light chain variable domain comprising an LC-CDR1 comprising the amino acid sequence of SEQ ID NO: 24, an LC-CDR2 comprising the amino acid sequence of SEQ ID NO: 31, and an LC-CDR3 comprising the amino acid sequence of SEQ ID NO: 38. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and b) a light chain variable domain comprising an LC-CDR1 comprising the amino acid sequence of SEQ ID NO: 25, an LC-CDR2 comprising the amino acid sequence of SEQ ID NO: 31, and an LC-CDR3 comprising the amino acid sequence of SEQ ID NO: 39. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and b) a light chain variable domain comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 40. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and b) a light chain variable domain comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 41. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 4, an HC-CDR2 comprising the amino acid sequence of SEQ ID NO: 12, and an HC-CDR3 comprising the amino acid sequence of SEQ ID NO: 19; and b) a light chain variable domain comprising an LC-CDR1 comprising the amino acid sequence of SEQ ID NO: 27, an LC-CDR2 comprising the amino acid sequence of SEQ ID NO: 33, and an LC-CDR3 comprising the amino acid sequence of SEQ ID NO: 42. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 5, an HC-CDR2 comprising the amino acid sequence of SEQ ID NO: 13, and an HC-CDR3 comprising the amino acid sequence of SEQ ID NO: 20; and b) a light chain variable domain comprising an LC-CDR1 comprising the amino acid sequence of SEQ ID NO: 28, an LC-CDR2 comprising the amino acid sequence of SEQ ID NO: 34, and an LC-CDR3 comprising the amino acid sequence of SEQ ID NO: 43. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and b) a light chain variable domain comprising an LC-CDR1 comprising the amino acid sequence of SEQ ID NO: 29, an LC-CDR2 comprising the amino acid sequence of SEQ ID NO: 35, and an LC-CDR3 comprising the amino acid sequence of SEQ ID NO: 43. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and b) a light chain variable domain comprising an LC-CDR1 comprising the amino acid sequence of SEQ ID NO: 83, an LC-CDR2 comprising the amino acid sequence of SEQ ID NO: 35, and an LC-CDR3 comprising the amino acid sequence of SEQ ID NO: 43. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a) a heavy chain variable domain comprising an HC-CDR1 comprising the amino acid sequence of SEQ ID NO: 7, an HC-CDR2 comprising the amino acid sequence of SEQ ID NO: 15, and an HC-CDR3 comprising the amino acid sequence of SEQ ID NO: 22; and b) a light chain variable domain comprising an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of any one of SEQ ID NOs: 46-61, 84-85, 92-94, or a variant thereof having at least about 80% (such as at least about any of 80%, 85%, 90%, 95%, 96%, 97%, 98%, or 99%) sequence identity to the amino acid sequence of any one of SEQ ID NOs: 46-61, 84-85, 92-94, and a light chain variable domain comprising the amino acid sequence of any one of SEQ ID NOs: 62-73, 86-91, 95-99, or a variant thereof having at least about 80% (such as at least about any of 80%, 85%, 90%, 95%, 96%, 97%, 98%, or 99%) sequence identity to the amino acid sequence of any one of SEQ ID NOs: 62-73, 86-91, 95-99. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG2 constant domains, wherein the anti-LIGHT antibody comprises a heavy chain variable domain comprising the amino acid sequence of any one of SEQ ID NOs: 46-61, 84-85, 92-94, or a variant thereof having at least about 80% (such as at least about any of 80%, 85%, 90%, 95%, 96%, 97%, 98%, or 99%) sequence identity to the amino acid sequence of any one of SEQ ID NOs: 46-61, 84-85, 92-94, and a light chain variable domain comprising the amino acid sequence of any one of SEQ ID NOs: 62-73, 86-91, 95-99, or a variant thereof having at least about 80% (such as at least about any of 80%, 85%, 90%, 95%, 96%, 97%, 98%, or 99%) sequence identity to the amino acid sequence of any one of SEQ ID NOs: 62-73, 86-91, 95-99. In some embodiments, the IgG2 is human IgG2. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a full-length anti-LIGHT antibody comprising IgG3 constant domains, wherein the anti-LIGHT antibody comprises a heavy chain variable domain comprising the amino acid sequence of any one of SEQ ID NOs: 46-61, 84-85, 92-94, or a variant thereof having at least about 80% (such as at least about any of 80%, 85%, 90%, 95%, 96%, 97%, 98%, or 99%) sequence identity to the amino acid sequence of any one of SEQ ID NOs: 46-61, 84-85, 92-94, and a light chain variable domain comprising the amino acid sequence of any one of SEQ ID NOs: 62-73, 86-91, 95-99, or a variant thereof having at least about 80% (such as at least about any of 80%, 85%, 90%, 95%, 96%, 97%, 98%, or 99%) sequence identity to the amino acid sequence of any one of SEQ ID NOs: 62-73, 86-91, 95-99. In some embodiments, the IgG3 is human IgG3. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of any one of SEQ ID NOs: 46-61, 84-85, 92-94, or a variant thereof having at least about 80% (such as at least about any of 80%, 85%, 90%, 95%, 96%, 97%, 98%, or 99%) sequence identity to the amino acid sequence of any one of SEQ ID NOs: 46-61, 84-85, 92-94, and a light chain variable domain comprising the amino acid sequence of any one of SEQ ID NOs: 62-73, 86-91, 95-99, or a variant thereof having at least about 80% (such as at least about any of 80%, 85%, 90%, 95%, 96%, 97%, 98%, or 99%) sequence identity to the amino acid sequence of any one of SEQ ID NOs: 62-73, 86-91, 95-99. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of any one of SEQ ID NOs: 46-61, 84-85, 92-94, and a light chain variable domain comprising the amino acid sequence of any one of SEQ ID NOs: 62-73, 86-91, 95-99. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of any one of SEQ ID NOs: 46-61, 84-85, 92-94, and a light chain variable domain comprising the amino acid sequence of any one of SEQ ID NOs: 62-73, 86-91, 95-99. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 46, or a variant thereof having at least about 80% sequence identity to the amino acid sequence of SEQ ID NO: 46; and a light chain variable domain comprising the amino acid sequence of SEQ ID NO: 62, or a variant thereof having at least about 80% sequence identity to the amino acid sequence of SEQ ID NO: 62. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 47, or a variant thereof having at least about 80% sequence identity to the amino acid sequence of SEQ ID NO: 47; and a light chain variable domain comprising the amino acid sequence of SEQ ID NO: 63, or a variant thereof having at least about 80% sequence identity to the amino acid sequence of SEQ ID NO: 63. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 48, or a variant thereof having at least about 80% sequence identity to the amino acid sequence of SEQ ID NO: 48; and a light chain variable domain comprising the amino acid sequence of SEQ ID NO: 63, or a variant thereof having at least about 80% sequence identity to the amino acid sequence of SEQ ID NO: 63. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 49, or a variant thereof having at least about 80% sequence identity to the amino acid sequence of SEQ ID NO: 49; and a light chain variable domain comprising the amino acid sequence of SEQ ID NO: 63, or a variant thereof having at least about 80% sequence identity to the amino acid sequence of SEQ ID NO: 63. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 50, or a variant thereof having at least about 80% sequence identity to the amino acid sequence of SEQ ID NO: 50; and a light chain variable domain comprising the amino acid sequence of SEQ ID NO: 63, or a variant thereof having at least about 80% sequence identity to the amino acid sequence of SEQ ID NO: 63. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 47, or a variant thereof having at least about 80% sequence identity to the amino acid sequence of SEQ ID NO: 47; and a light chain variable domain comprising the amino acid sequence of SEQ ID NO: 64, or a variant thereof having at least about 80% sequence identity to the amino acid sequence of SEQ ID NO: 64. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 48, or a variant thereof having at least about 80% sequence identity to the amino acid sequence of SEQ ID NO: 48; and a light chain variable domain comprising the amino acid sequence of SEQ ID NO: 64, or a variant thereof having at least about 80% sequence identity to the amino acid sequence of SEQ ID NO: 64. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 49, or a variant thereof having at least about 80% sequence identity to the amino acid sequence of SEQ ID NO: 49; and a light chain variable domain comprising the amino acid sequence of SEQ ID NO: 64, or a variant thereof having at least about 80% sequence identity to the amino acid sequence of SEQ ID NO: 64. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 50, or a variant thereof having at least about 80% sequence identity to the amino acid sequence of SEQ ID NO: 50; and a light chain variable domain comprising the amino acid sequence of SEQ ID NO: 64, or a variant thereof having at least about 80% sequence identity to the amino acid sequence of SEQ ID NO: 64. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 47, or a variant thereof having at least about 80% sequence identity to the amino acid sequence of SEQ ID NO: 47; and a light chain variable domain comprising the amino acid sequence of SEQ ID NO: 65, or a variant thereof having at least about 80% sequence identity to the amino acid sequence of SEQ ID NO: 65. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 48, or a variant thereof having at least about 80% sequence identity to the amino acid sequence of SEQ ID NO: 48; and a light chain variable domain comprising the amino acid sequence of SEQ ID NO: 65, or a variant thereof having at least about 80% sequence identity to the amino acid sequence of SEQ ID NO: 65. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 49, or a variant thereof having at least about 80% sequence identity to the amino acid sequence of SEQ ID NO: 49; and a light chain variable domain comprising the amino acid sequence of SEQ ID NO: 65, or a variant thereof having at least about 80% sequence identity to the amino acid sequence of SEQ ID NO: 65. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 50, or a variant thereof having at least about 80% sequence identity to the amino acid sequence of SEQ ID NO: 50; and a light chain variable domain comprising the amino acid sequence of SEQ ID NO: 65, or a variant thereof having at least about 80% sequence identity to the amino acid sequence of SEQ ID NO: 65. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 51, or a variant thereof having at least about 80% sequence identity to the amino acid sequence of SEQ ID NO: 51; and a light chain variable domain comprising the amino acid sequence of SEQ ID NO: 63, or a variant thereof having at least about 80% sequence identity to the amino acid sequence of SEQ ID NO: 63. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 52, or a variant thereof having at least about 80% sequence identity to the amino acid sequence of SEQ ID NO: 52; and a light chain variable domain comprising the amino acid sequence of SEQ ID NO: 65, or a variant thereof having at least about 80% sequence identity to the amino acid sequence of SEQ ID NO: 65. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 53, or a variant thereof having at least about 80% sequence identity to the amino acid sequence of SEQ ID NO: 53; and a light chain variable domain comprising the amino acid sequence of SEQ ID NO: 65, or a variant thereof having at least about 80% sequence identity to the amino acid sequence of SEQ ID NO: 65. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 51, or a variant thereof having at least about 80% sequence identity to the amino acid sequence of SEQ ID NO: 51; and a light chain variable domain comprising the amino acid sequence of SEQ ID NO: 65, or a variant thereof having at least about 80% sequence identity to the amino acid sequence of SEQ ID NO: 65. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 54, or a variant thereof having at least about 80% sequence identity to the amino acid sequence of SEQ ID NO: 54; and a light chain variable domain comprising the amino acid sequence of SEQ ID NO: 66, or a variant thereof having at least about 80% sequence identity to the amino acid sequence of SEQ ID NO: 66. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 55, or a variant thereof having at least about 80% sequence identity to the amino acid sequence of SEQ ID NO: 55; and a light chain variable domain comprising the amino acid sequence of SEQ ID NO: 67, or a variant thereof having at least about 80% sequence identity to the amino acid sequence of SEQ ID NO: 67. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 56, or a variant thereof having at least about 80% sequence identity to the amino acid sequence of SEQ ID NO: 56; and a light chain variable domain comprising the amino acid sequence of SEQ ID NO: 68, or a variant thereof having at least about 80% sequence identity to the amino acid sequence of SEQ ID NO: 68. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 57, or a variant thereof having at least about 80% sequence identity to the amino acid sequence of SEQ ID NO: 57; and a light chain variable domain comprising the amino acid sequence of SEQ ID NO: 69, or a variant thereof having at least about 80% sequence identity to the amino acid sequence of SEQ ID NO: 69. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 58, or a variant thereof having at least about 80% sequence identity to the amino acid sequence of SEQ ID NO: 58; and a light chain variable domain comprising the amino acid sequence of SEQ ID NO: 70, or a variant thereof having at least about 80% sequence identity to the amino acid sequence of SEQ ID NO: 70. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 59, or a variant thereof having at least about 80% sequence identity to the amino acid sequence of SEQ ID NO: 59; and a light chain variable domain comprising the amino acid sequence of SEQ ID NO: 71, or a variant thereof having at least about 80% sequence identity to the amino acid sequence of SEQ ID NO: 71. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 60, or a variant thereof having at least about 80% sequence identity to the amino acid sequence of SEQ ID NO: 60; and a light chain variable domain comprising the amino acid sequence of SEQ ID NO: 72, or a variant thereof having at least about 80% sequence identity to the amino acid sequence of SEQ ID NO: 72. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; and a light chain variable domain comprising the amino acid sequence of SEQ ID NO: 86, or a variant thereof having at least about 80% sequence identity to the amino acid sequence of SEQ ID NO: 86. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 85, or a variant thereof having at least about 80% sequence identity to the amino acid sequence of SEQ ID NO: 85; and a light chain variable domain comprising the amino acid sequence of SEQ ID NO: 87, or a variant thereof having at least about 80% sequence identity to the amino acid sequence of SEQ ID NO: 87. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; and a light chain variable domain comprising the amino acid sequence of SEQ ID NO: 88, or a variant thereof having at least about 80% sequence identity to the amino acid sequence of SEQ ID NO: 88. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; and a light chain variable domain comprising the amino acid sequence of SEQ ID NO: 89, or a variant thereof having at least about 80% sequence identity to the amino acid sequence of SEQ ID NO: 89. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 85, or a variant thereof having at least about 80% sequence identity to the amino acid sequence of SEQ ID NO: 85; and a light chain variable domain comprising the amino acid sequence of SEQ ID NO: 90, or a variant thereof having at least about 80% sequence identity to the amino acid sequence of SEQ ID NO: 90. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; and a light chain variable domain comprising the amino acid sequence of SEQ ID NO: 91, or a variant thereof having at least about 80% sequence identity to the amino acid sequence of SEQ ID NO: 91. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 61, or a variant thereof having at least about 80% sequence identity to the amino acid sequence of SEQ ID NO: 61; and a light chain variable domain comprising the amino acid sequence of SEQ ID NO: 73, or a variant thereof having at least about 80% sequence identity to the amino acid sequence of SEQ ID NO: 73. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; and a light chain variable domain comprising the amino acid sequence of SEQ ID NO: 95, or a variant thereof having at least about 80% sequence identity to the amino acid sequence of SEQ ID NO: 95. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 93, or a variant thereof having at least about 80% sequence identity to the amino acid sequence of SEQ ID NO: 93; and a light chain variable domain comprising the amino acid sequence of SEQ ID NO: 96, or a variant thereof having at least about 80% sequence identity to the amino acid sequence of SEQ ID NO: 96. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; and a light chain variable domain comprising the amino acid sequence of SEQ ID NO: 96, or a variant thereof having at least about 80% sequence identity to the amino acid sequence of SEQ ID NO: 96. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 94, or a variant thereof having at least about 80% sequence identity to the amino acid sequence of SEQ ID NO: 94; and a light chain variable domain comprising the amino acid sequence of SEQ ID NO: 97, or a variant thereof having at least about 80% sequence identity to the amino acid sequence of SEQ ID NO: 97. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 94, or a variant thereof having at least about 80% sequence identity to the amino acid sequence of SEQ ID NO: 94; and a light chain variable domain comprising the amino acid sequence of SEQ ID NO: 98, or a variant thereof having at least about 80% sequence identity to the amino acid sequence of SEQ ID NO: 98. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 93, or a variant thereof having at least about 80% sequence identity to the amino acid sequence of SEQ ID NO: 93; and a light chain variable domain comprising the amino acid sequence of SEQ ID NO: 98, or a variant thereof having at least about 80% sequence identity to the amino acid sequence of SEQ ID NO: 98. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; and a light chain variable domain comprising the amino acid sequence of SEQ ID NO: 98, or a variant thereof having at least about 80% sequence identity to the amino acid sequence of SEQ ID NO: 98. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 94, or a variant thereof having at least about 80% sequence identity to the amino acid sequence of SEQ ID NO: 94; and a light chain variable domain comprising the amino acid sequence of SEQ ID NO: 99, or a variant thereof having at least about 80% sequence identity to the amino acid sequence of SEQ ID NO: 99. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG1 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; and a light chain variable domain comprising the amino acid sequence of SEQ ID NO: 99, or a variant thereof having at least about 80% sequence identity to the amino acid sequence of SEQ ID NO: 99. In some embodiments, the IgG1 is human IgG1. In some embodiments, the heavy chain constant region comprises or consists of the amino acid sequence of SEQ ID NO: 74. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 74 and the light chain constant region comprises or consists of the amino acid sequence of SEQ ID NO: 76. In some embodiments, the heavy chain constant region comprises or consists of the amino acid sequence of SEQ ID NO: 74 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 46, or a variant thereof having at least about 80% sequence identity to the amino acid sequence of SEQ ID NO: 46; a light chain variable domain comprising the amino acid sequence of SEQ ID NO: 62, or a variant thereof having at least about 80% sequence identity to the amino acid sequence of SEQ ID NO: 62. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 47, or a variant thereof having at least about 80% sequence identity to the amino acid sequence of SEQ ID NO: 47; a light chain variable domain comprising the amino acid sequence of SEQ ID NO: 63, or a variant thereof having at least about 80% sequence identity to the amino acid sequence of SEQ ID NO: 63. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 48, or a variant thereof having at least about 80% sequence identity to the amino acid sequence of SEQ ID NO: 48; a light chain variable domain comprising the amino acid sequence of SEQ ID NO: 63, or a variant thereof having at least about 80% sequence identity to the amino acid sequence of SEQ ID NO: 63. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 49, or a variant thereof having at least about 80% sequence identity to the amino acid sequence of SEQ ID NO: 49; a light chain variable domain comprising the amino acid sequence of SEQ ID NO: 63, or a variant thereof having at least about 80% sequence identity to the amino acid sequence of SEQ ID NO: 63. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 50, or a variant thereof having at least about 80% sequence identity to the amino acid sequence of SEQ ID NO: 50; a light chain variable domain comprising the amino acid sequence of SEQ ID NO: 63, or a variant thereof having at least about 80% sequence identity to the amino acid sequence of SEQ ID NO: 63. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 47, or a variant thereof having at least about 80% sequence identity to the amino acid sequence of SEQ ID NO: 47; a light chain variable domain comprising the amino acid sequence of SEQ ID NO: 64, or a variant thereof having at least about 80% sequence identity to the amino acid sequence of SEQ ID NO: 64. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 48, or a variant thereof having at least about 80% sequence identity to the amino acid sequence of SEQ ID NO: 48; a light chain variable domain comprising the amino acid sequence of SEQ ID NO: 64, or a variant thereof having at least about 80% sequence identity to the amino acid sequence of SEQ ID NO: 64. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 49, or a variant thereof having at least about 80% sequence identity to the amino acid sequence of SEQ ID NO: 49; a light chain variable domain comprising the amino acid sequence of SEQ ID NO: 64, or a variant thereof having at least about 80% sequence identity to the amino acid sequence of SEQ ID NO: 64. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 50, or a variant thereof having at least about 80% sequence identity to the amino acid sequence of SEQ ID NO: 50; a light chain variable domain comprising the amino acid sequence of SEQ ID NO: 64, or a variant thereof having at least about 80% sequence identity to the amino acid sequence of SEQ ID NO: 64. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 47, or a variant thereof having at least about 80% sequence identity to the amino acid sequence of SEQ ID NO: 47; a light chain variable domain comprising the amino acid sequence of SEQ ID NO: 65, or a variant thereof having at least about 80% sequence identity to the amino acid sequence of SEQ ID NO: 65. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 48, or a variant thereof having at least about 80% sequence identity to the amino acid sequence of SEQ ID NO: 48; a light chain variable domain comprising the amino acid sequence of SEQ ID NO: 65, or a variant thereof having at least about 80% sequence identity to the amino acid sequence of SEQ ID NO: 65. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 49, or a variant thereof having at least about 80% sequence identity to the amino acid sequence of SEQ ID NO: 49; a light chain variable domain comprising the amino acid sequence of SEQ ID NO: 65, or a variant thereof having at least about 80% sequence identity to the amino acid sequence of SEQ ID NO: 65. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 50, or a variant thereof having at least about 80% sequence identity to the amino acid sequence of SEQ ID NO: 50; a light chain variable domain comprising the amino acid sequence of SEQ ID NO: 65, or a variant thereof having at least about 80% sequence identity to the amino acid sequence of SEQ ID NO: 65. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 51, or a variant thereof having at least about 80% sequence identity to the amino acid sequence of SEQ ID NO: 51; a light chain variable domain comprising the amino acid sequence of SEQ ID NO: 63, or a variant thereof having at least about 80% sequence identity to the amino acid sequence of SEQ ID NO: 63. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 52, or a variant thereof having at least about 80% sequence identity to the amino acid sequence of SEQ ID NO: 52; a light chain variable domain comprising the amino acid sequence of SEQ ID NO: 65, or a variant thereof having at least about 80% sequence identity to the amino acid sequence of SEQ ID NO: 65. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 53, or a variant thereof having at least about 80% sequence identity to the amino acid sequence of SEQ ID NO: 53; a light chain variable domain comprising the amino acid sequence of SEQ ID NO: 65, or a variant thereof having at least about 80% sequence identity to the amino acid sequence of SEQ ID NO: 65. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 51, or a variant thereof having at least about 80% sequence identity to the amino acid sequence of SEQ ID NO: 51; a light chain variable domain comprising the amino acid sequence of SEQ ID NO: 65, or a variant thereof having at least about 80% sequence identity to the amino acid sequence of SEQ ID NO: 65. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 54, or a variant thereof having at least about 80% sequence identity to the amino acid sequence of SEQ ID NO: 54; a light chain variable domain comprising the amino acid sequence of SEQ ID NO: 66, or a variant thereof having at least about 80% sequence identity to the amino acid sequence of SEQ ID NO: 66. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 55, or a variant thereof having at least about 80% sequence identity to the amino acid sequence of SEQ ID NO: 55; a light chain variable domain comprising the amino acid sequence of SEQ ID NO: 67, or a variant thereof having at least about 80% sequence identity to the amino acid sequence of SEQ ID NO: 67. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 56, or a variant thereof having at least about 80% sequence identity to the amino acid sequence of SEQ ID NO: 56; a light chain variable domain comprising the amino acid sequence of SEQ ID NO: 68, or a variant thereof having at least about 80% sequence identity to the amino acid sequence of SEQ ID NO: 68. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 57, or a variant thereof having at least about 80% sequence identity to the amino acid sequence of SEQ ID NO: 57; a light chain variable domain comprising the amino acid sequence of SEQ ID NO: 69, or a variant thereof having at least about 80% sequence identity to the amino acid sequence of SEQ ID NO: 69. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 58, or a variant thereof having at least about 80% sequence identity to the amino acid sequence of SEQ ID NO: 58; a light chain variable domain comprising the amino acid sequence of SEQ ID NO: 70, or a variant thereof having at least about 80% sequence identity to the amino acid sequence of SEQ ID NO: 70. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 59, or a variant thereof having at least about 80% sequence identity to the amino acid sequence of SEQ ID NO: 59; a light chain variable domain comprising the amino acid sequence of SEQ ID NO: 71, or a variant thereof having at least about 80% sequence identity to the amino acid sequence of SEQ ID NO: 71. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 60, or a variant thereof having at least about 80% sequence identity to the amino acid sequence of SEQ ID NO: 60; a light chain variable domain comprising the amino acid sequence of SEQ ID NO: 72, or a variant thereof having at least about 80% sequence identity to the amino acid sequence of SEQ ID NO: 72. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; a light chain variable domain comprising the amino acid sequence of SEQ ID NO: 86, or a variant thereof having at least about 80% sequence identity to the amino acid sequence of SEQ ID NO: 86. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 85, or a variant thereof having at least about 80% sequence identity to the amino acid sequence of SEQ ID NO: 85; a light chain variable domain comprising the amino acid sequence of SEQ ID NO: 87, or a variant thereof having at least about 80% sequence identity to the amino acid sequence of SEQ ID NO: 87. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; a light chain variable domain comprising the amino acid sequence of SEQ ID NO: 88, or a variant thereof having at least about 80% sequence identity to the amino acid sequence of SEQ ID NO: 88. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; a light chain variable domain comprising the amino acid sequence of SEQ ID NO: 89, or a variant thereof having at least about 80% sequence identity to the amino acid sequence of SEQ ID NO: 89. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 85, or a variant thereof having at least about 80% sequence identity to the amino acid sequence of SEQ ID NO: 85; a light chain variable domain comprising the amino acid sequence of SEQ ID NO: 90, or a variant thereof having at least about 80% sequence identity to the amino acid sequence of SEQ ID NO: 90. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 84, or a variant thereof having at least about 80% sequence identity to the amino acid sequence of SEQ ID NO: 84; a light chain variable domain comprising the amino acid sequence of SEQ ID NO: 91, or a variant thereof having at least about 80% sequence identity to the amino acid sequence of SEQ ID NO: 91. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 61, or a variant thereof having at least about 80% sequence identity to the amino acid sequence of SEQ ID NO: 61; a light chain variable domain comprising the amino acid sequence of SEQ ID NO: 73, or a variant thereof having at least about 80% sequence identity to the amino acid sequence of SEQ ID NO: 73. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; a light chain variable domain comprising the amino acid sequence of SEQ ID NO: 95, or a variant thereof having at least about 80% sequence identity to the amino acid sequence of SEQ ID NO: 95. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 93, or a variant thereof having at least about 80% sequence identity to the amino acid sequence of SEQ ID NO: 93; a light chain variable domain comprising the amino acid sequence of SEQ ID NO: 96, or a variant thereof having at least about 80% sequence identity to the amino acid sequence of SEQ ID NO: 96. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; a light chain variable domain comprising the amino acid sequence of SEQ ID NO: 96, or a variant thereof having at least about 80% sequence identity to the amino acid sequence of SEQ ID NO: 96. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 94, or a variant thereof having at least about 80% sequence identity to the amino acid sequence of SEQ ID NO: 94; a light chain variable domain comprising the amino acid sequence of SEQ ID NO: 97, or a variant thereof having at least about 80% sequence identity to the amino acid sequence of SEQ ID NO: 97. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 94, or a variant thereof having at least about 80% sequence identity to the amino acid sequence of SEQ ID NO: 94; a light chain variable domain comprising the amino acid sequence of SEQ ID NO: 98, or a variant thereof having at least about 80% sequence identity to the amino acid sequence of SEQ ID NO: 98. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 93, or a variant thereof having at least about 80% sequence identity to the amino acid sequence of SEQ ID NO: 93; a light chain variable domain comprising the amino acid sequence of SEQ ID NO: 98, or a variant thereof having at least about 80% sequence identity to the amino acid sequence of SEQ ID NO: 98. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; a light chain variable domain comprising the amino acid sequence of SEQ ID NO: 98, or a variant thereof having at least about 80% sequence identity to the amino acid sequence of SEQ ID NO: 98. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 94, or a variant thereof having at least about 80% sequence identity to the amino acid sequence of SEQ ID NO: 94; a light chain variable domain comprising the amino acid sequence of SEQ ID NO: 99, or a variant thereof having at least about 80% sequence identity to the amino acid sequence of SEQ ID NO: 99. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

In some embodiments, there is provided a full-length anti-LIGHT antibody comprising IgG4 constant domains, wherein the anti-LIGHT antibody comprises a heavy chain variable domain comprising the amino acid sequence of SEQ ID NO: 92, or a variant thereof having at least about 80% sequence identity to the amino acid sequence of SEQ ID NO: 92; a light chain variable domain comprising the amino acid sequence of SEQ ID NO: 99, or a variant thereof having at least about 80% sequence identity to the amino acid sequence of SEQ ID NO: 99. In some embodiments, the IgG4 is human IgG4. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77. In some embodiments, the heavy chain constant region comprises or consists of the amino acid sequence of SEQ ID NO: 75 and the light chain constant region comprises or consists of the amino acid sequence of SEQ ID NO: 76. In some embodiments, the heavy chain constant region comprises or consists of the amino acid sequence of SEQ ID NO: 75 and the light chain constant region comprises or consists of the amino acid sequence of SEQ ID NO: 77.

### Binding affinity

Binding affinity can be indicated by Kd, Koff, Kon, or Ka. The term "Koff", as used herein, is intended to refer to the off-rate constant for dissociation of an antibody from the antibody /antigen complex, as determined from a kinetic selection set up. The term "Kon", as used herein, is intended to refer to the on-rate constant for association of an antibody to the antigen to form the antibody/antigen complex. The term dissociation constant "Kd", as used herein, refers to the dissociation constant of a particular antibody-antigen interaction, and describes the concentration of antigen required to occupy one half of all of the antibody-binding domains present in a solution of antibody molecules at equilibrium, and is equal to Koff/Kon. The measurement of Kd presupposes that all binding agents are in solution. In the case where the antibody is tethered to a cell wall, *e.g.,* in a yeast expression system, the corresponding equilibrium rate constant is expressed as EC50, which gives a good approximation of Kd. The affinity constant, Ka, is the inverse of the dissociation constant, Kd.

The dissociation constant (Kd) is used as an indicator showing affinity of antibody moieties to antigens. For example, easy analysis is possible by the Scatchard method using antibodies marked with a variety of marker agents, as well as by using Biacore (made by Amersham Biosciences), analysis of biomolecular interactions by surface plasmon resonance, according to the user's manual and attached kit. The Kd value that can be derived using these methods is expressed in units of M. An antibody that specifically binds to a target may have a Kd of, for example, ≤ 10⁻⁷ M, ≤ 10⁻⁸ M, ≤ 10⁻⁹ M, ≤ 10⁻¹⁰ M, ≤ 10⁻¹¹ M, ≤ 10⁻¹² M, or ≤ 10⁻¹³ M.

Binding specificity of the antibody can be determined experimentally by methods known in the art. Such methods comprise, but are not limited to, Western blots, ELISA-, RIA-, ECL-, IRMA-, EIA-, BIAcore-tests and peptide scans.

In some embodiments, the anti-LIGHT antibody specifically binds to a target LIGHT with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the anti-LIGHT antibody and LIGHT, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹ M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the anti-LIGHT antibody and a LIGHT is about 10⁻⁷ M to about 10⁻¹³ M.

In some embodiments, the Kd of the binding between the anti-LIGHT antibody and a non-target is more than the Kd of the binding between the anti-LIGHT antibody and the target, and is herein referred to in some embodiments as the binding affinity, of the anti-LIGHT antibody to the target (e.g., LIGHT) is higher than that to a non-target. In some embodiments, the non-target is an antigen that is not LIGHT. In some embodiments, the Kd of the binding between the anti-LIGHT antibody (against LIGHT) and a non-LIGHT target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the anti-LIGHT antibody and a target LIGHT.

In some embodiments, the anti-LIGHT antibody binds to a non-target with a Kd of about 10⁻¹ M to about 10⁻⁶ M (such as about 10⁻¹ M to about 10⁻⁶ M, about 10⁻¹ M to about 10⁻⁵ M, or about 10⁻² M to about 10⁻⁴ M). In some embodiments, the non-target is an antigen that is not LIGHT. Thus in some embodiments, the Kd of the binding between the anti-LIGHT antibody and a non-LIGHT target is about 10⁻¹ M to about 10⁻⁶ M, about 1×10⁻¹ M to about 5×10⁻⁶ M, about 10⁻¹ M to about 10⁻⁵ M, about 1×10⁻¹ M to about 5×10⁻⁵ M, about 10⁻¹ M to about 10⁻⁴ M, about 1×10⁻¹ M to about 5×10⁻⁴ M, about 10⁻¹ M to about 10⁻³ M, about 1×10⁻¹ M to about 5×10⁻³ M, about 10⁻¹ M to about 10⁻² M, about 10⁻² M to about 10⁻⁶ M, about 1×10⁻² M to about 5×10⁻⁶ M, about 10⁻² M to about 10⁻⁵ M, about 1×10⁻² M to about 5×10⁻⁵ M, about 10⁻² M to about 10⁻⁴ M, about 1×10⁻² M to about 5×10⁻⁴ M, about 10⁻² M to about 10⁻³ M, about 10⁻³ M to about 10⁻⁶ M, about 1×10⁻³ M to about 5×10⁻⁶ M, about 10⁻³ M to about 10⁻⁵ M, about 1×10⁻³ M to about 5×10⁻⁵ M, about 10⁻³ M to about 10⁻⁴ M, about 10⁻⁴ M to about 10⁻⁶ M, about 1×10⁻⁴ M to about 5×10⁻⁶ M, about 10⁻⁴ M to about 10⁻⁵ M, or about 10⁻⁵ M to about 10⁻⁶ M.

In some embodiments, when referring to that the anti-LIGHT antibody specifically recognizes a target LIGHT at a high binding affinity, and binds to a non-target at a low binding affinity, the anti-LIGHT antibody will bind to the target LIGHT with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M), and will bind to the non-target with a Kd of about 10⁻¹ M to about 10⁻⁶ M (such as about 10⁻¹ M to about 10⁻⁶ M, about 10⁻¹ M to about 10⁻⁵ M, or about 10⁻³ M to about 10⁻⁴ M).

In some embodiments, when referring to that the anti-LIGHT antibody specifically recognizes LIGHT, the binding affinity of the anti-LIGHT antibody is compared to that of a control anti-LIGHT antibody (such as CERC-002). In some embodiments, the Kd of the binding between the control anti-LIGHT antibody and LIGHT can be at least about 2 times, such as about 2 times, about 3 times, about 4 times, about 5 times, about 6 times, about 7 times, about 8 times, about 9 times, about 10 times, about 10-100 times, about 100-1000 times, about 10³-10⁴ times of the Kd of the binding between the anti-LIGHT antibody described herein and LIGHT.

### Nucleic Acids

Nucleic acid molecules encoding the anti-LIGHT antibodies are also contemplated. In some embodiments, there is provided a nucleic acid (or a set of nucleic acids) encoding a full-length anti-LIGHT antibody, including any of the full-length anti-LIGHT antibodies described herein. In some embodiments, the nucleic acid (or a set of nucleic acids) encoding the anti-LIGHT antibody described herein may further comprises a nucleic acid sequence encoding a peptide tag (such as protein purification tag, *e.g.*, His-tag, HA tag).

Also contemplated here are isolated host cells comprising an anti-LIGHT antibody, an isolated nucleic acid encoding the polypeptide components of the anti-LIGHT antibody, or a vector comprising a nucleic acid encoding the polypeptide components of the anti-LIGHT antibody described herein.

The present application also includes variants to these nucleic acid sequences. For example, the variants include nucleotide sequences that hybridize to the nucleic acid sequences encoding the anti-LIGHT antibodies of the present application under at least moderately stringent hybridization conditions.

The present application also provides vectors in which a nucleic acid of the present application is inserted.

In brief summary, the expression of an anti-LIGHT antibody (*e.g.,* full-length anti-LIGHT antibody) by a natural or synthetic nucleic acid encoding the anti-LIGHT antibody can be achieved by inserting the nucleic acid into an appropriate expression vector, such that the nucleic acid is operably linked to 5' and 3' regulatory elements, including for example a promoter (*e.g.,* a lymphocyte-specific promoter) and a 3' untranslated region (UTR). The vectors can be suitable for replication and integration in eukaryotic host cells. Typical cloning and expression vectors contain transcription and translation terminators, initiation sequences, and promoters useful for regulation of the expression of the desired nucleic acid sequences.

The nucleic acids of the present application may also be used for nucleic acid immunization and gene therapy, using standard gene delivery protocols. Methods for gene delivery are known in the art. See, *e.g.,* U.S. Pat. Nos. 5,399,346, 5,580,859, 5,589,466, incorporated by reference herein in their entireties. In some embodiments, the application provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Green and Sambrook (2013, Molecular Cloning: A Laboratory Manual, Cold Spring Harbor Laboratory, New York), and in other virology and molecular biology manuals. Viruses which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers (*see, e.g.,* WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In some embodiments, lentivirus vectors are used. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

Additional promoter elements, *e.g.*, enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the application should not be limited to the use of constitutive promoters. Inducible promoters are also contemplated as part of the application. The use of an inducible promoter provides a molecular switch capable of turning on expression of the polynucleotide sequence to which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

In some embodiments, the expression of the anti-LIGHT antibody is inducible. In some embodiments, a nucleic acid sequence encoding the anti-LIGHT antibody is operably linked to an inducible promoter, including any inducible promoter described herein.

### Inducible promoters

The use of an inducible promoter provides a molecular switch capable of turning on expression of the polynucleotide sequence to which it is operatively linked when such expression is desired, or turning off the expression when expression is not desired. Exemplary inducible promoter systems for use in eukaryotic cells include, but are not limited to, hormone-regulated elements (*e.g., see* Mader, S. and White, J. H. (1993) Proc. Natl. Acad. Sci. USA 90:5603-5607), synthetic ligand-regulated elements (*see, e.g.,* Spencer, D. M. et al. (1993) Science 262: 1019-1024) and ionizing radiation-regulated elements (*e.g.,* see Manome, Y. et al. (1993) Biochemistry 32: 10607-10613; Datta, R. et al. (1992) Proc. Natl. Acad. Sci. USA 89: 1014- 10153). Further exemplary inducible promoter systems for use in in vitro or in vivo mammalian systems are reviewed in Gingrich et al. (1998) Annual Rev. Neurosci 21:377-405. In some embodiments, the inducible promoter system for use to express the anti-LIGHT antibody is the Tet system. In some embodiments, the inducible promoter system for use to express the anti-LIGHT antibody is the lac repressor system from *E. coli.*

An exemplary inducible promoter system for use in the present application is the Tet system. Such systems are based on the Tet system described by Gossen *et al.* (1993). In an exemplary embodiment, a polynucleotide of interest is under the control of a promoter that comprises one or more Tet operator (TetO) sites. In the inactive state, Tet repressor (TetR) will bind to the TetO sites and repress transcription from the promoter. In the active state, *e.g.,* in the presence of an inducing agent such as tetracycline (Tc), anhydrotetracycline, doxycycline (Dox), or an active analog thereof, the inducing agent causes release of TetR from TetO, thereby allowing transcription to take place. Doxycycline is a member of the tetracycline family of antibiotics having the chemical name of 1-dimethylamino-2,4a,5,7,12-pentahydroxy-11-methyl-4,6-dioxo-1,4a,11,11a,12,12a-hexahydrotetracene-3-carboxamide.

In one embodiment, a TetR is codon-optimized for expression in mammalian cells, *e.g.,* murine or human cells. Most amino acids are encoded by more than one codon due to the degeneracy of the genetic code, allowing for substantial variations in the nucleotide sequence of a given nucleic acid without any alteration in the amino acid sequence encoded by the nucleic acid. However, many organisms display differences in codon usage, also known as "codon bias" (*i.e.,* bias for use of a particular codon(s) for a given amino acid). Codon bias often correlates with the presence of a predominant species of tRNA for a particular codon, which in turn increases efficiency of mRNA translation. Accordingly, a coding sequence derived from a particular organism (*e.g.,* a prokaryote) may be tailored for improved expression in a different organism (*e.g.,* a eukaryote) through codon optimization.

Other specific variations of the Tet system include the following "Tet-Off" and "Tet-On" systems. In the Tet-Off system, transcription is inactive in the presence of Tc or Dox. In that system, a tetracycline-controlled transactivator protein (tTA), which is composed of TetR fused to the strong transactivating domain of VP16 from Herpes simplex virus, regulates expression of a target nucleic acid that is under transcriptional control of a tetracycline-responsive promoter element (TRE). The TRE is made up of TetO sequence concatamers fused to a promoter (commonly the minimal promoter sequence derived from the human cytomegalovirus (hCMV) immediate-early promoter). In the absence of Tc or Dox, tTA binds to the TRE and activates transcription of the target gene. In the presence of Tc or Dox, tTA cannot bind to the TRE, and expression from the target gene remains inactive.

Conversely, in the Tet-On system, transcription is active in the presence of Tc or Dox. The Tet-On system is based on a reverse tetracycline-controlled transactivator, rtTA. Like tTA, rtTA is a fusion protein comprised of the TetR repressor and the VP16 transactivation domain. However, a four amino acid change in the TetR DNA binding moiety alters rtTA's binding characteristics such that it can only recognize the tetO sequences in the TRE of the target transgene in the presence of Dox. Thus, in the Tet-On system, transcription of the TRE-regulated target gene is stimulated by rtTA only in the presence of Dox.

Another inducible promoter system is the lac repressor system from *E. coli* (*See* Brown et al., Cell 49:603-612 (1987)). The lac repressor system functions by regulating transcription of a polynucleotide of interest operably linked to a promoter comprising the lac operator (lacO). The lac repressor (lacR) binds to LacO, thus preventing transcription of the polynucleotide of interest. Expression of the polynucleotide of interest is induced by a suitable inducing agent, *e.g.,* isopropyl-β-D-thiogalactopyranoside (IPTG).

In order to assess the expression of a polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, *e.g.,* enzymatic activity. Expression of the reporter gene is assayed at a suitable time after the DNA has been introduced into the recipient cells. Suitable reporter genes may include genes encoding luciferase, β-galactosidase, chloramphenicol acetyl transferase, secreted alkaline phosphatase, or the green fluorescent protein gene (*e.g.,* Ui-Tel et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In some embodiments, there is provided nucleic acid encoding a full-length anti-LIGHT antibody according to any of the full-length anti-LIGHT antibodies described herein. In some embodiments, the nucleic acid comprises one or more nucleic acid sequences encoding the heavy and light chains of the full-length anti-LIGHT antibody. In some embodiments, each of the one or more nucleic acid sequences are contained in separate vectors. In some embodiments, at least some of the nucleic acid sequences are contained in the same vector. In some embodiments, all of the nucleic acid sequences are contained in the same vector. Vectors may be selected, for example, from the group consisting of mammalian expression vectors and viral vectors (such as those derived from retroviruses, adenoviruses, adeno-associated viruses, herpes viruses, and lentiviruses).

Methods of introducing and expressing genes into a cell are known in the art. In the context of an expression vector, the vector can be readily introduced into a host cell, *e.g.,* mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Green and Sambrook (2013, Molecular Cloning: A Laboratory Manual, Cold Spring Harbor Laboratory, New York). In some embodiments, the introduction of a polynucleotide into a host cell is carried out by calcium phosphate transfection.

Biological methods for introducing a polynucleotide of interest into a host cell include the use of DNA and RNA vectors. Viral vectors, and especially retroviral vectors, have become the most widely used method of inserting genes into mammalian, *e.g.*, human cells. Other viral vectors can be derived from lentivirus, poxviruses, herpes simplex virus 1, adenoviruses and adeno-associated viruses, and the like. *See,* for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (*e.g.,* an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Regardless of the method used to introduce exogenous nucleic acids into a host cell or otherwise expose a cell to the inhibitor of the present application, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.,* by immunological means (ELISAs and Western blots) or by assays described herein to identify agents falling within the scope of the application.

### Preparation of anti-LIGHT antibodies

In some embodiments, the anti-LIGHT antibody is a monoclonal antibody or derived from a monoclonal antibody. In some embodiments, the anti-LIGHT antibody comprises V_{H} and V_{L} domains, or variants thereof, from the monoclonal antibody. In some embodiments, the anti-LIGHT antibody further comprises C_{H}1 and C_{L} domains, or variants thereof, from the monoclonal antibody. Monoclonal antibodies can be prepared, *e.g.,* using known methods in the art, including hybridoma methods, phage display methods, or using recombinant DNA methods. Additionally, exemplary phage display methods are described herein and in the Examples below.

In a hybridoma method, a hamster, mouse, or other appropriate host animal is typically immunized with an immunizing agent to elicit lymphocytes that produce or are capable of producing antibodies that will specifically bind to the immunizing agent. Alternatively, the lymphocytes can be immunized *in vitro.* The immunizing agent can include a polypeptide or a fusion protein of the protein of interest. Generally, peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine, and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

In some embodiments, the immortalized cell lines fuse efficiently, support stable high-level expression of antibody by the selected antibody-producing cells, and are sensitive to a medium such as HAT medium. In some embodiments, the immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies.

The culture medium in which the hybridoma cells are cultured can then be assayed for the presence of monoclonal antibodies directed against the polypeptide. The binding specificity of monoclonal antibodies produced by the hybridoma cells can be determined by immunoprecipitation or by an *in vitro* binding assay, such as radioimmunoassay (RIA) or enzyme-linked immunosorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones can be sub-cloned by limiting dilution procedures and grown by standard methods. Goding, *supra.* Suitable culture media for this purpose include, for example, Dulbecco's Modified Eagle's Medium and RPMI-1640 medium. Alternatively, the hybridoma cells can be grown *in vivo* as ascites in a mammal.

The monoclonal antibodies secreted by the sub-clones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

In some embodiments, according to any of the anti-LIGHT antibodies described herein, the anti-LIGHT antibody comprises sequences from a clone selected from an antibody library (such as a phage library presenting scFv or Fab fragments). The clone may be identified by screening combinatorial libraries for antibody fragments with the desired activity or activities. For example, a variety of methods are known in the art for generating phage display libraries and screening such libraries for antibodies possessing the desired binding characteristics. Such methods are reviewed, *e.g.,* in Hoogenboom et al., Methods in Molecular Biology 178:1-37 (O'Brien et al., ed., Human Press, Totowa, N.J., 2001) and further described, *e.g.,* in McCafferty et al., Nature 348:552-554; Clackson et al., Nature 352: 624-628 (1991); Marks et al., J. Mol. Biol. 222: 581-597 (1992); Marks and Bradbury, Methods in Molecular Biology 248:161-175 (Lo, ed., Human Press, Totowa, N.J., 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as scFv fragments or as Fab fragments. Libraries from immunized sources provide high-affinity antibodies to the immunogen without the requirement of constructing hybridomas. Alternatively, the naive repertoire can be cloned (*e.g.,* from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: U.S. Pat. No. 5,750,373, and US Patent Publication Nos. 2005/0079574, 2005/0119455, 2005/0266000, 2007/0117126, 2007/0160598, 2007/0237764, 2007/0292936, and 2009/0002360.

The anti-LIGHT antibodies can be prepared using phage display to screen libraries for anti-LIGHT antibody moieties specific to the target LIGHT. The library can be a human scFv phage display library having a diversity of at least 1 × 10⁹ (such as at least about any of 1 × 10⁹, 2.5 × 10⁹, 5 × 10⁹, 7.5 × 10⁹, 1 × 10¹⁰, 2.5 × 10¹⁰, 5 × 10¹⁰, 7.5 × 10¹⁰, or 1 × 10¹¹) unique human antibody fragments. In some embodiments, the library is a naïve human library constructed from DNA extracted from human PMBCs and spleens from healthy donors, encompassing all human heavy and light chain subfamilies. In some embodiments, the library is a naïve human library constructed from DNA extracted from PBMCs isolated from patients with various diseases, such as patients with autoimmune diseases, cancer patients, and patients with infectious diseases. In some embodiments, the library is a semi-synthetic human library, wherein heavy chain CDR3 is completely randomized, with all amino acids (with the exception of cysteine) equally likely to be present at any given position (*see, e.g.,* Hoet, R.M. et al., Nat. Biotechnol. 23(3):344-348, 2005). In some embodiments, the heavy chain CDR3 of the semi-synthetic human library has a length from about 5 to about 24 (such as about any of 5, 6, 7, 8, 9, 10, 11, 12, 13, 14, 15, 16, 17, 18, 19, 20, 21, 22, 23, or 24) amino acids. In some embodiments, the library is a fully-synthetic phage display library. In some embodiments, the library is a non-human phage display library.

Phage clones that bind to the target LIGHT with high affinity can be selected by iterative binding of phage to the target LIGHT, which is bound to a solid support (such as, for example, beads for solution panning or mammalian cells for cell panning), followed by removal of non-bound phage and by elution of specifically bound phage. The bound phage clones are then eluted and used to infect an appropriate host cell, such as *E. coli* XL1-Blue, for expression and purification. The panning can be performed for multiple (such as about any of 2, 3, 4, 5, 6 or more) rounds with solution panning, cell panning, or a combination of both, to enrich for phage clones binding specifically to the target LIGHT. Enriched phage clones can be tested for specific binding to the target LIGHT by any methods known in the art, including for example ELISA and FACS.

An alternative method for screening antibody libraries is to display the protein on the surface of yeast cells. Wittrup et al. (US Patent Nos. 6,699,658 and 6,696,25 1) have developed a method for a yeast cell display library. In this yeast display system, a component involves the yeast agglutinin protein (Aga1), which is anchored to the yeast cell wall. Another component involves a second subunit of the agglutinin protein Aga2, which can display on the surface yeast cells through disulfide bonds to Aga1 protein. The protein Aga1 is expressed from a yeast chromosome after the Aga1 gene integration. A library of single chain variable fragments (scFv) is fused genetically to Aga2 sequence in the yeast display plasmid, which, after transformation, is maintained in yeast episomally with a nutritional marker. Both of the Aga1 and Aga2 proteins were expressed under the control of the galactose-inducible promoter.

Human antibody V gene repertoire (V_{H} and V_{L} fragments) are obtained by PCR method using a pool of degenerate primers (Sblattero, D. & Bradbury, A. Immunotechnology 3, 271-278 1998). The PCR templates are from the commercially available RNAs or cDNAs, including PBMC, spleen, lymph nodes, bone marrow and tonsils. Separate V_{H} and V_{K} PCR libraries were combined, then assembled together in the scFv format by overlap extension PCR ( Sheets, M.D. et al., Proc. Natl. Acad. Sci. USA 95, 6157-6162 1998.). To construct the yeast scFv display library, the resultant scFv PCR products are cloned into the yeast display plasmid in the yeasts by homologous recombination. (Chao, G, et al., Nat Protoc. 2006;1(2):755-68. Miller KD, et al., Current Protocols in Cytometry 4.7.1-4.7.30, 2008).

The anti-LIGHT antibodies can be discovered using mammalian cell display systems in which antibody moieties are displayed on the cell surface and those specific to the target LIGHT are isolated by the antigen-guided screening method, as described in U.S. patent No. 7,732,195B2. A Chinese hamster ovary (CHO) cell library representing a large set of human IgG antibody genes can be established and used to discover the clones expressing high-affinity antibody genes. Another display system has been developed to enable simultaneous high-level cell surface display and secretion of the same protein through alternate splicing, where the displayed protein phenotype remains linked to genotype, allowing soluble secreted antibody to be simultaneously characterized in biophysical and cell-based functional assays. This approach overcomes many limitations of previous mammalian cell display, enabling direct selection and maturation of antibodies in the form of full-length, glycosylated IgGs (Peter M. Bowers, et al., Methods 2014,65:44-56). Transient expression systems are suitable for a single round of antigen selection before recovery of the antibody genes and therefore most useful for the selection of antibodies from smaller libraries. Stable episomal vectors offer an attractive alternative. Episomal vectors can be transfected at high efficiency and stably maintained at low copy number, permitting multiple rounds of panning and the resolution of more complex antibody libraries.

The IgG library is based on germline sequence V-gene segments joined to rearranged (D)J regions isolated from a panel of human donors. RNA collected from 2000 human blood samples was reverse-transcribed into cDNA, and the V_{H} and V_{K} fragments were amplified using V_{H}- and V_{L}-specific primers and purified by gel extraction. IgG libraries were generated by sub-cloning the V_{H} and V_{K} fragments into the display vectors containing IgG1 or K constant regions respectively and then electroporating into or transducing 293T cells. To generate the scFv antibody display library, scFvs were generated by linking V_{H} and V_{K}, and then sub-cloned into the display vector, which were then electroporated into or transduce 293T cells. As we known, the IgG library is based on germline sequence V-gene segments joined to rearranged (D)J regions isolated from a panel of donors, the donor can be a mouse, rat, rabbit, or monkey.

Monoclonal antibodies can also be made by recombinant DNA methods, such as those described in U.S. Patent No. 4,816,567. DNA encoding the monoclonal antibodies of the application can be readily isolated and sequenced using conventional procedures (*e.g.,* by using oligonucleotide probes that are capable of binding specifically to genes encoding the heavy and light chains of murine antibodies). Hybridoma cells as described above or LIGHT-specific phage clones of the application can serve as a source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy- and light-chain constant domains and/or framework regions in place of the homologous non-human sequences (U.S. Patent No. 4,816,567; Morrison *et al., supra*) or by covalently joining to the immunoglobulin coding sequence all or part of the coding sequence for a non-immunoglobulin polypeptide. Such a non-immunoglobulin polypeptide can be substituted for the constant domains of an antibody of the application, or can be substituted for the variable domains of one antigen-combining site of an antibody of the application to create a chimeric bivalent antibody.

The antibodies can be monovalent antibodies. Methods for preparing monovalent antibodies are known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using any method known in the art.

Antibody variable domains with the desired binding specificities (antibody-antigen combining sites) can be fused to immunoglobulin constant-domain sequences. The fusion preferably is with an immunoglobulin heavy-chain constant domain, comprising at least part of the hinge, CH2, and CH3 regions. In some embodiments, the first heavy-chain constant region (CH1) containing the site necessary for light-chain binding is present in at least one of the fusions. DNAs encoding the immunoglobulin heavy-chain fusions and, if desired, the immunoglobulin light chain, are inserted into separate expression vectors, and are co-transfected into a suitable host organism.

### Human and Humanized Antibodies

The anti-LIGHT antibodies (*e.g.,* full-length anti-LIGHT antibodies) can be humanized antibodies or human antibodies. Humanized forms of non-human (*e.g.,* murine) antibody moieties are chimeric immunoglobulins, immunoglobulin chains, or fragments thereof (such as Fv, Fab, Fab', F(ab')₂, scFv, or other antigen-binding subsequences of antibodies) that typically contain minimal sequence derived from non-human immunoglobulin. Humanized antibody moieties include human immunoglobulins, immunoglobulin chains, or fragments thereof (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibody moieties can also comprise residues that are found neither in the recipient antibody nor in the imported CDR or framework sequences. In general, the humanized antibody can comprise substantially at least one, and typically two, variable domains, in which all or substantially all of the CDR regions correspond to those of a non-human immunoglobulin, and all or substantially all of the FR regions are those of a human immunoglobulin consensus sequence.

Generally, a humanized antibody has one or more amino acid residues introduced into it from a source that is non-human. These non-human amino acid residues are often referred to as "import" residues, which are typically taken from an "import" variable domain. According to some embodiments, humanization can be essentially performed following the method of Winter and co-workers (Jones et al., Nature, 321: 522-525 (1986); Riechmann et al., Nature, 332: 323-327 (1988); Verhoeyen et al., Science, 239: 1534-1536 (1988)), by substituting rodent CDRs or CDR sequences for the corresponding sequences of a human antibody. Accordingly, such "humanized" antibody moieties are antibody moieties (U.S. Patent No. 4,816,567), wherein substantially less than an intact human variable domain has been substituted by the corresponding sequence from a non-human species. In practice, humanized antibody moieties are typically human antibody moieties in which some CDR residues and possibly some FR residues are substituted by residues from analogous sites in rodent antibodies.

As an alternative to humanization, human antibody moieties can be generated. For example, it is now possible to produce transgenic animals (*e.g.,* mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., PNAS USA, 90:2551 (1993); Jakobovits et al., Nature, 362:255-258 (1993); Bruggemann et al., Year in Immunol., 7:33 (1993); U.S. Patent Nos. 5,545,806, 5,569,825, 5,591,669; 5,545,807; and WO 97/17852. Alternatively, human antibodies can be made by introducing human immunoglobulin loci into transgenic animals, *e.g.,* mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016, and Marks et al., Bio/Technology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

Human antibodies may also be generated by *in vitro* activated B cells (see U.S. Patents 5,567,610 and 5,229,275) or by using various techniques known in the art, including phage display libraries. Hoogenboom and Winter, J. Mol. Biol., 227:381 (1991); Marks et al., J. Mol. Biol., 222:581 (1991). The techniques of Cole *et al.* and Boerner *et al.* are also available for the preparation of human monoclonal antibodies. Cole et al., Monoclonal Antibodies and Cancer Therapy, Alan R. Liss, p.77 (1985) and Boerner et al., J. Immunol., 147(1): 86-95 (1991).

### Anti-LIGHT antibody variants

In some embodiments, amino acid sequences of the anti-LIGHT antibody variants (*e.g.,* full-length anti-LIGHT antibody) provided herein are contemplated. For example, it may be desirable to improve the binding affinity and/or other biological properties of the antibody. Amino acid sequences of an antibody variant may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, *e.g.,* antigen-binding.

In some embodiments, anti-LIGHT antibody variants having one or more amino acid substitutions are provided. Sites of interest for substitutional mutagenesis include the HVRs and FRs. Amino acid substitutions may be introduced into an antibody of interest and the products screened for a desired activity, *e.g.,* improved bioactivity, retained/improved antigen binding, decreased immunogenicity, or improved ADCC or CDC.

Conservative substitutions are shown in Table 4 below.

**TABLE 4: CONSERVATIVE SUBSTITUTIONS**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tvr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped into different classes according to common side-chain properties:
a. hydrophobic: Norleucine, Met, Ala, Val, Leu, Ile;
b. neutral hydrophilic: Cys, Ser, Thr, Asn, Gln;
c. acidic: Asp, Glu;
d. basic: His, Lys, Arg;
e. residues that influence chain orientation: Gly, Pro;
f. aromatic: Trp, Tyr, Phe.

Non-conservative substitutions will entail exchanging a member of one of these classes for another class.

An exemplary substitutional variant is an affinity matured antibody, which may be conveniently generated, *e.g.,* using phage display-based affinity maturation techniques. Briefly, one or more CDR residues are mutated and the variant antibody moieties displayed on phage and screened for a particular biological activity (*e.g.,* bioactivity based on inhibition of primary T cell proliferation co-stimulated by CD3 agonist and LIGHT or binding affinity). Alterations (*e.g.,* substitutions) may be made in HVRs, *e.g.,* to improve bioactivity based on inhibition of primary T cell proliferation co-stimulated by CD3 agonist and LIGHT or binding affinity. Such alterations may be made in HVR "hotspots", *e.g.,* residues encoded by codons that undergo mutation at high frequency during the somatic maturation process (see, *e.g.,* Chowdhury, Methods Mol. Biol. 207:179-196 (2008)), and/or specificity determining residues (SDRs), with the resulting variant V_{H} and V_{L} being tested for binding affinity. Affinity maturation by constructing and reselecting from secondary libraries has been described, *e.g.,* in Hoogenboom et al., in Methods in Molecular Biology 178:1-37 (O'Brien et al., ed., Human Press, Totowa, NJ, (2001*)).*

In some embodiments of affinity maturation, diversity is introduced into the variable genes chosen for maturation by any of a variety of methods (*e.g.,* error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g.,* 4-6 residues at a time) are randomized. HVR residues involved in antigen binding may be specifically identified, *e.g.,* using alanine scanning mutagenesis or modeling. CDR-H3 and CDR-L3 in particular are often targeted.

In some embodiments, substitutions, insertions, or deletions may occur within one or more HVRs so long as such alterations do not substantially reduce the ability of the antibody to bind antigen. For example, conservative alterations (*e.g.,* conservative substitutions as provided herein) that do not substantially reduce binding affinity may be made in HVRs. Such alterations may be outside of HVR "hotspots" or SDRs. In some embodiments of the variant V_{H} and V_{L} sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e.g.,* charged residues such as Arg, Asp, His, Lys, and Glu) are identified and replaced by a neutral or negatively charged amino acid (*e.g.,* Ala or Glu) to determine whether the interaction of the antibody with antigen is affected. Further substitutions may be introduced at the amino acid locations to demonstrate functional sensitivity to the initial substitutions. Alternatively, or additionally, a crystal structure of an antigen-antibody complex can be determined to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.,* for ADEPT) or a polypeptide which increases the serum half-life of the antibody.

### Fc Region Variants

In some embodiments, one or more amino acid modifications may be introduced into the Fc region of an antibody (*e.g.,* a full-length anti-LIGHT antibody or anti-LIGHT Fc fusion protein) provided herein, thereby generating an Fc region variant. In some embodiments, the Fc region variant has enhanced ADCC effector function, often related to binding to Fc receptors (FcRs). In some embodiments, the Fc region variant has decreased ADCC effector function. There are many examples of changes or mutations to Fc sequences that can alter effector function. For example, WO 00/42072 and Shields et al., J Biol. Chem. 9(2): 6591-6604 (2001) describe antibody variants with improved or diminished binding to FcRs. The contents of those publications are specifically incorporated herein by reference.

Antibody-Dependent Cell-Mediated Cytotoxicity (ADCC) is a mechanism of action of therapeutic antibodies against tumor cells. ADCC is a cell-mediated immune defense whereby an effector cell of the immune system actively lyses a target cell (*e.g.,* a cancer cell), whose membrane-surface antigens have been bound by specific antibodies (*e.g.,* an anti-LIGHT antibody). The typical ADCC involves activation of NK cells by antibodies. An NK cell expresses CD16 which is an Fc receptor. This receptor recognizes, and binds to, the Fc portion of an antibody bound to the surface of a target cell. The most common Fc receptor on the surface of an NK cell is called CD16 or FcγRIII. Binding of the Fc receptor to the Fc region of an antibody results in NK cell activation, release of cytolytic granules and consequent target cell apoptosis. The contribution of ADCC to tumor cell killing can be measured with a specific test that uses NK-92 cells that have been transfected with a high-affinity FcR. Results are compared to wild-type NK-92 cells that do not express the FcR.

In some embodiments, the application contemplates an anti-LIGHT antibody variant (such as a full-length anti-LIGHT antibody variant) comprising an Fc region that possesses some but not all effector functions, which makes it a desirable candidate for applications in which the half-life of the anti-LIGHT antibody *in vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Pat. No. 5,500,362 (see, *e.g.,* Hellstrom, I. et al., Proc. Nat'l Acad. Sci. USA 83:7059-7063 (1986)) and Hellstrom, I. et al., Proc. Nat'l Acad. Sci. USA 82:1499-1502 (1985); U.S. Pat. No. 5,821,337 (see Bruggemann, M. et al., J. Exp. Med. 166:1351-1361 (1987)). Alternatively, non-radioactive assay methods may be employed (see, for example, ACTI^{™} non-radioactive cytotoxicity assay for flow cytometry (CellTechnology, Inc. Mountain View, Calif.; and CYTOTOX 96^{™} non-radioactive cytotoxicity assay (Promega, Madison, Wis.). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al., Proc. Nat'l Acad. Sci. USA 95:652-656 (1998). C1q binding assays may also be carried out to confirm that the antibody is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M. S. et al., Blood 101:1045-1052 (2003); and Cragg, M. S. and M. J. Glennie, Blood 103:2738-2743 (2004)). FcRn binding and *in vivo* clearance/half-life determinations can also be performed using methods known in the art (see, *e.g.,* Petkova, S. B. et al., Int'l. Immunol. 18(12):1759-1769 (2006)).

Antibodies with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

Certain antibody variants with improved or diminished binding to FcRs are described. (See, *e.g.,* U.S. Pat. No. 6,737,056; WO 2004/056312, and Shields et al., J. Biol. Chem. 9(2): 6591-6604 (2001).)

In some embodiments, there is provided an anti-LIGHT antibody (such as a full-length anti-LIGHT antibody) variant comprising a variant Fc region comprising one or more amino acid substitutions which improve ADCC. In some embodiments, the variant Fc region comprises one or more amino acid substitutions which improve ADCC, wherein the substitutions are at positions 298, 333, and/or 334 of the variant Fc region (EU numbering of residues). In some embodiments, the anti-LIGHT antibody (*e.g.,* full-length anti-LIGHT antibody) variant comprises the following amino acid substitution in its variant Fc region: S298A, E333A, and K334A.

In some embodiments, alterations are made in the Fc region that result in altered (*i.e.,* either improved or diminished) C1q binding and/or Complement Dependent Cytotoxicity (CDC), *e.g.,* as described in U.S. Pat. No. 6,194,551, WO 99/51642, and Idusogie et al., J. Immunol. 164: 4178-4184 (2000).

In some embodiments, there is provided an anti-LIGHT antibody (such as a full-length anti-LIGHT antibody) variant comprising a variant Fc region comprising one or more amino acid substitutions which increase half-life and/or improve binding to the neonatal Fc receptor (FcRn). Antibodies with increased half-lives and improved binding to FcRn are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g.,* substitution of Fc region residue 434 (U.S. Pat. No. 7,371,826).

*See also* Duncan & Winter, Nature 322:738-40 (1988); U.S. Pat. No. 5,648,260; U.S. Pat. No. 5,624,821; and WO 94/29351 concerning other examples of Fc region variants.

Anti-LIGHT antibodies (such as full-length anti-LIGHT antibodies) comprising any of the Fc variants described herein, or combinations thereof, are contemplated.

### Glycosylation Variants

In some embodiments, an anti-LIGHT antibody (such as a full-length anti-LIGHT antibody) provided herein is altered to increase or decrease the extent to which the anti-LIGHT antibody is glycosylated. Addition or deletion of glycosylation sites to an anti-LIGHT antibody may be conveniently accomplished by altering the amino acid sequence of the anti-LIGHT antibody or polypeptide portion thereof such that one or more glycosylation sites are created or removed.

Wherein the anti-LIGHT antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.,* Wright et al., TIBTECH 15:26-32 (1997). The oligosaccharide may include various carbohydrates, *e.g.,* mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an anti-LIGHT antibody of the application may be made in order to create anti-LIGHT antibody variants with certain improved properties.

The N-glycans attached to the CH2 domain of Fc is heterogeneous. Antibodies or Fc fusion proteins generated in CHO cells are fucosylated by fucosyltransferase activity. See Shoji-Hosaka et al., J. Biochem. 2006, 140:777- 83. Normally, a small percentage of naturally occurring afucosylated IgGs may be detected in human serum. N-glycosylation of the Fc is important for binding to FcγR; and afucosylation of the N-glycan increases Fc's binding capacity to FcγRIIIa. Increased FcγRIIIa binding can enhance ADCC, which can be advantageous in certain antibody therapeutic applications in which cytotoxicity is desirable.

In some embodiments, an enhanced effector function can be detrimental when Fc-mediated cytotoxicity is undesirable. In some embodiments, the Fc fragment or CH2 domain is not glycosylated. In some embodiments, the N-glycosylation site in the CH2 domain is mutated to prevent from glycosylation.

In some embodiments, anti-LIGHT antibody (such as a full-length anti-LIGHT antibody) variants are provided comprising an Fc region wherein a carbohydrate structure attached to the Fc region has reduced fucose or lacks fucose, which may improve ADCC function. Specifically, anti-LIGHT antibodies are contemplated herein that have reduced fucose relative to the amount of fucose on the same anti-LIGHT antibody produced in a wild-type CHO cell. That is, they are characterized by having a lower amount of fucose than they would otherwise have if produced by native CHO cells (*e.g.,* a CHO cell that produce a native glycosylation pattern, such as, a CHO cell containing a native FUT8 gene). In some embodiments, the anti-LIGHT antibody is one wherein less than about 50%, 40%, 30%, 20%, 10%, or 5% of the N-linked glycans thereon comprise fucose. For example, the amount of fucose in such an anti-LIGHT antibody may be from 1% to 80%, from 1% to 65%, from 5% to 65% or from 20% to 40%. In some embodiments, the anti-LIGHT antibody is one wherein none of the N-linked glycans thereon comprise fucose, *i.e.,* wherein the anti-LIGHT antibody is completely without fucose, or has no fucose or is afucosylated. The amount of fucose is determined by calculating the average amount of fucose within the sugar chain at Asn297, relative to the sum of all glycostructures attached to Asn 297 (*e.g.,* complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ±3 amino acids upstream or downstream of position 297, *i.e.,* between positions 294 and 300, due to minor sequence variations in antibodies. Such fucosylation variants may have improved ADCC function. See, *e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al., J. Mol. Biol. 336:1239-1249 (2004); Yamane-Ohnuki et al., Biotech. Bioeng. 87: 614 (2004). Examples of cell lines capable of producing defucosylated antibodies include Lec13 CHO cells deficient in protein fucosylation (Ripka et al., Arch. Biochem. Biophys. 249:533-545 (1986); US Pat Appl No US 2003/0157108 A1, Presta, L; and WO 2004/056312 A1, Adams et al*.,* especially at Example 11), and knockout cell lines, such asα-1,6-fucosyltransferase gene, FUT8, knockout CHO cells (see, *e.g.,* Yamane-Ohnuki et al., Biotech. Bioeng. 87: 614 (2004); Kanda, Y. et al., Biotechnol. Bioeng. 94(4):680-688 (2006); and WO2003/085107).

Anti-LIGHT antibody (such as a full-length anti-LIGHT antibody) variants are further provided with bisected oligosaccharides, *e.g.,* in which a biantennary oligosaccharide attached to the Fc region of the anti-LIGHT antibody is bisected by GlcNAc. Such anti-LIGHT antibody (such as a full-length anti-LIGHT antibody) variants may have reduced fucosylation and/or improved ADCC function. Examples of such antibody variants are described, *e.g.,* in WO 2003/011878 (Jean-Mairet et al.); U.S. Pat. No. 6,602,684 (Umana et al.); US 2005/0123546 (Umana et al.), and Ferrara et al., Biotechnology and Bioengineering, 93(5): 851-861 (2006). Anti-LIGHT antibody (such as full-length anti-LIGHT antibody) variants with at least one galactose residue in the oligosaccharide attached to the Fc region are also provided. Such anti-LIGHT antibody variants may have improved CDC function. Such antibody variants are described, *e.g.,* in WO 1997/30087 (Patel et al.); WO 1998/58964 (Raju, S.); and WO 1999/22764 (Raju, S.).

In some embodiments, the anti-LIGHT antibody (such as a full-length anti-LIGHT antibody) variants comprising an Fc region are capable of binding to an FcyRIII. In some embodiments, the anti-LIGHT antibody (such as a full-length anti-LIGHT antibody) variants comprising an Fc region have ADCC activity in the presence of human effector cells (*e.g.,* T cell) or have increased ADCC activity in the presence of human effector cells compared to the otherwise same anti-LIGHT antibody (such as a full-length anti-LIGHT antibody) comprising a human wild-type IgG1Fc region.

### Cysteine Engineered Variants

In some embodiments, it may be desirable to create cysteine engineered anti-LIGHT antibodies (such as a full-length anti-LIGHT antibody) in which one or more amino acid residues are substituted with cysteine residues. In some embodiments, the substituted residues occur at accessible sites of the anti-LIGHT antibody. By substituting those residues with cysteine, reactive thiol groups are thereby positioned at accessible sites of the anti-LIGHT antibody and may be used to conjugate the anti-LIGHT antibody to other moieties, such as drug moieties or linker-drug moieties, to create an anti-LIGHT immunoconjugate, as described further herein. Cysteine engineered anti-LIGHT antibodies (*e.g.,* full-length anti-LIGHT antibodies) may be generated as described, e.g., in U.S. Pat. No. 7,521,541.

### Derivatives

In some embodiments, an anti-LIGHT antibody (such as a full-length anti-LIGHT antibody) provided herein may be further modified to contain additional non-proteinaceous moieties that are known in the art and readily available. The moieties suitable for derivatization of the anti-LIGHT antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (*e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the anti-LIGHT antibody may vary, and if more than one polymer are attached, they can be the same or different molecules. In general, the number and/or type of polymers used for derivatization can be determined based on considerations including, but not limited to, the particular properties or functions of anti-LIGHT antibody to be improved, whether the anti-LIGHT antibody derivative will be used in a therapy under defined conditions, *etc.*

### Pharmaceutical Compositions

Also provided herein are compositions (such as pharmaceutical compositions, also referred to herein as formulations) comprising any of the anti-LIGHT antibodies (such as a full-length anti-LIGHT antibody), nucleic acids encoding the antibodies, vectors comprising the nucleic acids encoding the antibodies, or host cells comprising the nucleic acids or vectors described herein. In some embodiments, there is provided a pharmaceutical composition comprising any one of the anti-LIGHT antibodies described herein and a pharmaceutically acceptable carrier.

Suitable formulations of the anti-LIGHT antibodies are obtained by mixing an anti-LIGHT antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.,* Zn-protein complexes); and/or non-ionic surfactants such as TWEEN^{™}, PLURONICS^{™} or polyethylene glycol (PEG). Exemplary formulations are described in WO98/56418, expressly incorporated herein by reference. Lyophilized formulations adapted for subcutaneous administration are described in WO97/04801. Such lyophilized formulations may be reconstituted with a suitable diluent to a high protein concentration and the reconstituted formulation may be administered subcutaneously to the individual to be treated herein. Lipofectins or liposomes can be used to deliver the anti-LIGHT antibodies of this application into cells.

The formulation herein may also contain one or more active compounds in addition to the anti-LIGHT antibody (such as a full-length anti-LIGHT antibody) as necessary for the particular indication being treated, preferably those with complementary activities that do not adversely affect each other. For example, it may be desirable to further provide an antineoplastic agent, a growth inhibitory agent, a cytotoxic agent, or a chemotherapeutic agent in addition to the anti-LIGHT antibody. Such molecules are suitably present in combination in amounts that are effective for the purpose intended. The effective amount of such other agents depends on the amount of anti-LIGHT antibody present in the formulation, the type of disease or disorder or treatment, and other factors discussed above. These are generally used in the same dosages and with administration routes as described herein or about from 1 to 99% of the heretofore employed dosages.

The anti-LIGHT antibodies (*e.g.,* full-length anti-LIGHT antibodies) may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Sustained-release preparations may be prepared.

Sustained-release preparations of the anti-LIGHT antibodies (*e.g.,* full-length anti-LIGHT antibodies) can be prepared. Suitable examples of sustained-release preparations include semipermeable matrices of solid hydrophobic polymers containing the antibody (or fragment thereof), which matrices are in the form of shaped articles, *e.g.,* films, or microcapsules. Examples of sustained-release matrices include polyesters, hydrogels (for example, poly(2-hydroxyethyl-methacrylate ), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D (-)-3-hydroxybutyric acid. While polymers such as ethylene-vinyl acetate and lactic acid-glycolic acid enable release of molecules for over 100 days, certain hydro gels release proteins for shorter time periods. When encapsulated antibody remain in the body for a long time, they can denature or aggregate as a result of exposure to moisture at 37°C, resulting in a loss of biological activity and possible changes in immunogenicity. Rational strategies can be devised for stabilization of anti-LIGHT antibodies depending on the mechanism involved. For example, if the aggregation mechanism is discovered to be intermolecular S-S bond formation through thio-disulfide interchange, stabilization can be achieved by modifying sulfhydryl residues, lyophilizing from acidic solutions, controlling moisture content, using appropriate additives, and developing specific polymer matrix compositions.

In some embodiments, the anti-LIGHT antibody (such as a full-length anti-LIGHT antibody) is formulated in a buffer comprising a citrate, NaCl, acetate, succinate, glycine, polysorbate 80 (Tween 80), or any combination of the foregoing.

The formulations to be used for in vivo administration must be sterile. This is readily accomplished by, *e.g.,* filtration through sterile filtration membranes.

### Methods of treatment using anti-LIGHT antibodies

The anti-LIGHT antibodies (*e.g.,* full-length anti-LIGHT antibodies) and/or compositions of the application can be administered to individuals (*e.g.,* mammals such as humans) to treat a disease and/or disorder associated with LIGHT signaling (*e.g.,* autoimmune disease and/or inflammatory disease). These diseases include, but are not limited to, inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. The present application thus in some embodiments provides a method of treating a disease or condition associated with LIGHT signaling (*e.g.,* autoimmune disease and/or inflammatory disease) in an individual comprising administering to the individual an effective amount of a composition (such as a pharmaceutical composition) comprising an anti-LIGHT antibody (*e.g.,* a full-length anti-LIGHT antibody), such as any one of the anti-LIGHT antibodies (*e.g.,* full-length anti-LIGHT antibodies) described herein. In some embodiments, the individual is human.

For example, in some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody (e.g., full-length anti-LIGHT antibody) specifically binding to an epitope on human LIGHT, wherein the epitope comprises amino acid residues of human LIGHT. In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a heavy chain variable domain (V_{H}) comprising a heavy chain complementarity determining region (HC-CDR) 1 comprising GYFIN (SEQ ID NO: 1); an HC-CDR2 comprising RIYPYNVX₁TFYNQNFKG (SEQ ID NO: 45), wherein X₁ is D or N; and an HC-CDR3 comprising GTHYYGSSGAMDY (SEQ ID NO: 16); and a light chain variable domain (V_{L}) comprising a light chain complementarity determining region (LC-CDR) 1 comprising KASQNVGTAVA (SEQ ID NO: 23); an LC-CDR2 comprising SASNRYT (SEQ ID NO: 30); and an LC-CDR3 comprising QQYSSYPYT (SEQ ID NO: 37). In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising: a V_{H} comprising an HC-CDR1 comprising the amino acid sequence of SEQ ID NO: 1, an HC-CDR2 comprising the amino acid sequence of any one of SEQ ID NOs: 8-9, and an HC-CDR3 comprising the amino acid sequence of SEQ ID NO: 16, or a variant thereof comprising up to 5 amino acid substitutions in the HC-CDRs; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37, or a variant thereof comprising up to 5 amino acid substitutions in the LC-CDRs.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of any one of SEQ ID NOs: 46-53 or a variant thereof having at least about 80% sequence identity to the amino acid sequence of any one of SEQ ID NOs: 46-53, and a V_{L} comprising the amino acid sequence of any one of SEQ ID NOs: 62-65, or a variant thereof having at least about 80% sequence identity to the amino acid sequence of any one of SEQ ID NOs: 62-65.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising: a V_{H} comprising an HC-CDR1 comprising the amino acid sequence of SEQ ID NO: 1, an HC-CDR2 comprising the amino acid sequence of SEQ ID NO: 8, and an HC-CDR3 comprising the amino acid sequence of SEQ ID NO: 16, or a variant thereof comprising up to 5 amino acid substitutions in the HC-CDRs; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37, or a variant thereof comprising up to 5 amino acid substitutions in the LC-CDRs.

In some embodiments, the anti-LIGHT antibody provided herein comprises a V_{H} comprising the amino acid sequence of SEQ ID NO: 46, or a variant thereof having at least about 80% sequence identity to the amino acid sequence of SEQ ID NO: 46; and a V_{L} comprising the amino acid sequence of SEQ ID NO: 62, or a variant thereof having at least about 80% sequence identity to the amino acid sequence of SEQ ID NO: 62. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 63, or a variant thereof having at least about 80% sequence identity to the amino acid sequence of SEQ ID NO: 63. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 63, or a variant thereof having at least about 80% sequence identity to the amino acid sequence of SEQ ID NO: 63. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 63, or a variant thereof having at least about 80% sequence identity to the amino acid sequence of SEQ ID NO: 63. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 63, or a variant thereof having at least about 80% sequence identity to the amino acid sequence of SEQ ID NO: 63. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 64, or a variant thereof having at least about 80% sequence identity to the amino acid sequence of SEQ ID NO: 64. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 64, or a variant thereof having at least about 80% sequence identity to the amino acid sequence of SEQ ID NO: 64. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 64, or a variant thereof having at least about 80% sequence identity to the amino acid sequence of SEQ ID NO: 64. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 64, or a variant thereof having at least about 80% sequence identity to the amino acid sequence of SEQ ID NO: 64. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 65, or a variant thereof having at least about 80% sequence identity to the amino acid sequence of SEQ ID NO: 65. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 65, or a variant thereof having at least about 80% sequence identity to the amino acid sequence of SEQ ID NO: 65. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 65, or a variant thereof having at least about 80% sequence identity to the amino acid sequence of SEQ ID NO: 65. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 65, or a variant thereof having at least about 80% sequence identity to the amino acid sequence of SEQ ID NO: 65. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising: a V_{H} comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 16, or a variant thereof comprising up to 5 amino acid substitutions in the HC-CDRs; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37, or a variant thereof comprising up to 5 amino acid substitutions in the LC-CDRs.

In some embodiments, the anti-LIGHT antibody provided herein comprises a V_{H} comprising the amino acid sequence of SEQ ID NO: 51, or a variant thereof having at least about 80% sequence identity to the amino acid sequence of SEQ ID NO: 51; and a V_{L} comprising the amino acid sequence of SEQ ID NO: 63, or a variant thereof having at least about 80% sequence identity to the amino acid sequence of SEQ ID NO: 63. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 52, or a variant thereof having at least about 80% sequence identity to the amino acid sequence of SEQ ID NO: 52; and a V_{L} comprising the amino acid sequence of SEQ ID NO: 65, or a variant thereof having at least about 80% sequence identity to the amino acid sequence of SEQ ID NO: 65. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 53, or a variant thereof having at least about 80% sequence identity to the amino acid sequence of SEQ ID NO: 53; and a V_{L} comprising the amino acid sequence of SEQ ID NO: 65, or a variant thereof having at least about 80% sequence identity to the amino acid sequence of SEQ ID NO: 65. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 51, or a variant thereof having at least about 80% sequence identity to the amino acid sequence of SEQ ID NO: 51; and a V_{L} comprising the amino acid sequence of SEQ ID NO: 65, or a variant thereof having at least about 80% sequence identity to the amino acid sequence of SEQ ID NO: 65. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17, or a variant thereof comprising up to 5 amino acid substitutions in the HC-CDRs; and a V_{L} comprising an LC-CDR1 comprising the amino acid sequence of SEQ ID NO: 24, an LC-CDR2 comprising the amino acid sequence of SEQ ID NO: 31, and an LC-CDR3 comprising the amino acid sequence of SEQ ID NO: 38, or a variant thereof comprising up to 5 amino acid substitutions in the LC-CDRs.In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of SEQ ID NO: 54 or a variant thereof having at least about 80% sequence identity to the amino acid sequence of SEQ ID NO: 54, and a V_{L} comprising the amino acid sequence of SEQ ID NO: 66, or a variant thereof having at least about 80% sequence identity to the amino acid sequence of SEQ ID NO: 66.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17, or a variant thereof comprising up to 5 amino acid substitutions in the HC-CDRs; and a V_{L} comprising an LC-CDR1 comprising the amino acid sequence of SEQ ID NO: 25, an LC-CDR2 comprising the amino acid sequence of SEQ ID NO: 31, and an LC-CDR3 comprising the amino acid sequence of SEQ ID NO: 39, or a variant thereof comprising up to 5 amino acid substitutions in the LC-CDRs.In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of SEQ ID NO: 55 or a variant thereof having at least about 80% sequence identity to the amino acid sequence of SEQ ID NO: 55, and a V_{L} comprising the amino acid sequence of SEQ ID NO: 67, or a variant thereof having at least about 80% sequence identity to the amino acid sequence of SEQ ID NO: 67.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18, or a variant thereof comprising up to 5 amino acid substitutions in the HC-CDRs; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 40, or a variant thereof comprising up to 5 amino acid substitutions in the LC-CDRs.In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of SEQ ID NO: 56 or a variant thereof having at least about 80% sequence identity to the amino acid sequence of SEQ ID NO: 56, and a V_{L} comprising the amino acid sequence of SEQ ID NO: 68, or a variant thereof having at least about 80% sequence identity to the amino acid sequence of SEQ ID NO: 68.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18, or a variant thereof comprising up to 5 amino acid substitutions in the HC-CDRs; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 41, or a variant thereof comprising up to 5 amino acid substitutions in the LC-CDRs. In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of SEQ ID NO: 57 or a variant thereof having at least about 80% sequence identity to the amino acid sequence of SEQ ID NO: 57, and a V_{L} comprising the amino acid sequence of SEQ ID NO: 69, or a variant thereof having at least about 80% sequence identity to the amino acid sequence of SEQ ID NO: 69.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising an HC-CDR1 comprising the amino acid sequence of SEQ ID NO: 4, an HC-CDR2 comprising the amino acid sequence of SEQ ID NO: 12, and an HC-CDR3 comprising the amino acid sequence of SEQ ID NO: 19, or a variant thereof comprising up to 5 amino acid substitutions in the HC-CDRs; and a V_{L} comprising an LC-CDR1 comprising the amino acid sequence of SEQ ID NO: 27, an LC-CDR2 comprising the amino acid sequence of SEQ ID NO: 33, and an LC-CDR3 comprising the amino acid sequence of SEQ ID NO: 42, or a variant thereof comprising up to 5 amino acid substitutions in the LC-CDRs.In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of SEQ ID NO: 58 or a variant thereof having at least about 80% sequence identity to the amino acid sequence of SEQ ID NO: 58, and a V_{L} comprising the amino acid sequence of SEQ ID NO: 70, or a variant thereof having at least about 80% sequence identity to the amino acid sequence of SEQ ID NO: 70.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising an HC-CDR1 comprising the amino acid sequence of SEQ ID NO: 5, an HC-CDR2 comprising the amino acid sequence of SEQ ID NO: 13, and an HC-CDR3 comprising the amino acid sequence of SEQ ID NO: 20, or a variant thereof comprising up to 5 amino acid substitutions in the HC-CDRs; and a V_{L} comprising an LC-CDR1 comprising the amino acid sequence of SEQ ID NO: 28, an LC-CDR2 comprising the amino acid sequence of SEQ ID NO: 34, and an LC-CDR3 comprising the amino acid sequence of SEQ ID NO: 43, or a variant thereof comprising up to 5 amino acid substitutions in the LC-CDRs.In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of SEQ ID NO: 59 or a variant thereof having at least about 80% sequence identity to the amino acid sequence of SEQ ID NO: 59, and a V_{L} comprising the amino acid sequence of SEQ ID NO: 71, or a variant thereof having at least about 80% sequence identity to the amino acid sequence of SEQ ID NO: 71.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a heavy chain variable domain (V_{H}) comprising a heavy chain complementarity determining region (HC-CDR) 1 comprising DHIMN (SEQ ID NO: 6); an HC-CDR2 comprising RIYPVSGETNYNQKFMG (SEQ ID NO: 14); and an HC-CDR3 comprising GSYYWNAMDY (SEQ ID NO: 21); and a light chain variable domain (V_{L}) comprising a light chain complementarity determining region (LC-CDR) 1 comprising KASQSVDFDGDSYMN (SEQ ID NO: 29) or KASQSVDFDGESYMN (SEQ ID NO: 83); an LC-CDR2 comprising SASNLES (SEQ ID NO: 35); and an LC-CDR3 comprising QQSIEDPWT (SEQ ID NO: 43). In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising: a V_{H} comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21, or a variant thereof comprising up to 5 amino acid substitutions in the HC-CDRs; and a V_{L} comprising an LC-CDR1 comprising the amino acid sequence of any one of SEQ ID NOs: 29 or 83, an LC-CDR2 comprising the amino acid sequence of SEQ ID NO: 35, and an LC-CDR3 comprising the amino acid sequence of SEQ ID NO: 43, or a variant thereof comprising up to 5 amino acid substitutions in the LC-CDRs. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of any one of SEQ ID NOs: 60, 84-85 or a variant thereof having at least about 80% sequence identity to the amino acid sequence of any one of SEQ ID NOs: 60, 84-85, and a V_{L} comprising the amino acid sequence of any one of SEQ ID NOs: 72, 86-91, or a variant thereof having at least about 80% sequence identity to the amino acid sequence of any one of SEQ ID NOs: 72, 86-91.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising: a V_{H} comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21, or a variant thereof comprising up to 5 amino acid substitutions in the HC-CDRs; and a V_{L} comprising an LC-CDR1 comprising the amino acid sequence of SEQ ID NOs: 29, an LC-CDR2 comprising the amino acid sequence of SEQ ID NO: 35, and an LC-CDR3 comprising the amino acid sequence of SEQ ID NO: 43, or a variant thereof comprising up to 5 amino acid substitutions in the LC-CDRs. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 60, or a variant thereof having at least about 80% sequence identity to the amino acid sequence of SEQ ID NO: 60; and a V_{L} comprising the amino acid sequence of SEQ ID NO: 72, or a variant thereof having at least about 80% sequence identity to the amino acid sequence of SEQ ID NO: 72. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 86, or a variant thereof having at least about 80% sequence identity to the amino acid sequence of SEQ ID NO: 86. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 85, or a variant thereof having at least about 80% sequence identity to the amino acid sequence of SEQ ID NO: 85; and a V_{L} comprising the amino acid sequence of SEQ ID NO: 87, or a variant thereof having at least about 80% sequence identity to the amino acid sequence of SEQ ID NO: 87. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 88, or a variant thereof having at least about 80% sequence identity to the amino acid sequence of SEQ ID NO: 88. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising: a V_{H} comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21, or a variant thereof comprising up to 5 amino acid substitutions in the HC-CDRs; and a V_{L} comprising an LC-CDR1 comprising the amino acid sequence of SEQ ID NO: 83, an LC-CDR2 comprising the amino acid sequence of SEQ ID NO: 35, and an LC-CDR3 comprising the amino acid sequence of SEQ ID NO: 43, or a variant thereof comprising up to 5 amino acid substitutions in the LC-CDRs.

In some embodiments, the anti-LIGHT antibody provided herein comprises a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 89, or a variant thereof having at least about 80% sequence identity to the amino acid sequence of SEQ ID NO: 89. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 85, or a variant thereof having at least about 80% sequence identity to the amino acid sequence of SEQ ID NO: 85; and a V_{L} comprising the amino acid sequence of SEQ ID NO: 90, or a variant thereof having at least about 80% sequence identity to the amino acid sequence of SEQ ID NO: 90. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the anti-LIGHT antibody provided herein comprises a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 91, or a variant thereof having at least about 80% sequence identity to the amino acid sequence of SEQ ID NO: 91. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

For example, in some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising an HC-CDR1 comprising the amino acid sequence of SEQ ID NO: 7, an HC-CDR2 comprising the amino acid sequence of SEQ ID NO: 15, and an HC-CDR3 comprising the amino acid sequence of SEQ ID NO: 22, or a variant thereof comprising up to 5 amino acid substitutions in the HC-CDRs; and a V_{L} comprising an LC-CDR1 comprising the amino acid sequence of SEQ ID NO: 29, an LC-CDR2 comprising the amino acid sequence of SEQ ID NO: 36, and an LC-CDR3 comprising the amino acid sequence of SEQ ID NO: 44, or a variant thereof comprising up to 5 amino acid substitutions in the LC-CDRs.In some embodiments, the anti-LIGHT antibody is a full-length antibody. In some embodiments, the full-length anti-LIGHT antibody is an IgG1 or IgG4 antibody. In some embodiments, the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19. In some embodiments, the individual is human.

In some embodiments, there is provided a method of treating an individual having a disease or condition associated with LIGHT signaling (*e.g.,* autoimmune disease and/or inflammatory disease) comprising administering to the individual an effective amount of a pharmaceutical composition comprising an anti-LIGHT antibody (*e.g.,* full-length anti-LIGHT antibody) comprising a V_{H} comprising the amino acid sequence of any one of SEQ ID NO: 61, 92-94 or a variant thereof having at least about 80% sequence identity to the amino acid sequence of SEQ ID NO: 61, 92-94, and a V_{L} comprising the amino acid sequence of any one of SEQ ID NO: 73, 95-99, or a variant thereof having at least about 80% sequence identity to the amino acid sequence of SEQ ID NO: 73, 95-99.

In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 61, or a variant thereof having at least about 80% sequence identity to the amino acid sequence of SEQ ID NO: 61; and a V_{L} comprising the amino acid sequence of SEQ ID NO: 73, or a variant thereof having at least about 80% sequence identity to the amino acid sequence of SEQ ID NO: 73. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 95, or a variant thereof having at least about 80% sequence identity to the amino acid sequence of SEQ ID NO: 95. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 96, or a variant thereof having at least about 80% sequence identity to the amino acid sequence of SEQ ID NO: 96. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 96, or a variant thereof having at least about 80% sequence identity to the amino acid sequence of SEQ ID NO: 96. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 97, or a variant thereof having at least about 80% sequence identity to the amino acid sequence of SEQ ID NO: 97. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 98, or a variant thereof having at least about 80% sequence identity to the amino acid sequence of SEQ ID NO: 98. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 98, or a variant thereof having at least about 80% sequence identity to the amino acid sequence of SEQ ID NO: 98. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 98, or a variant thereof having at least about 80% sequence identity to the amino acid sequence of SEQ ID NO: 98. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 99, or a variant thereof having at least about 80% sequence identity to the amino acid sequence of SEQ ID NO: 99. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, there is provided a method of treating an individual having a disease or condition associated with LIGHT signaling (e.g., autoimmune disease and/or inflammatory disease) comprising administering to the individual an effective amount of a pharmaceutical composition comprising an anti-LIGHT antibody comprising: a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 99, or a variant thereof having at least about 80% sequence identity to the amino acid sequence of SEQ ID NO: 99. In some embodiments, the anti-LIGHT antibody provided herein is a full-length anti-LIGHT antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 74. In some embodiments, the heavy chain constant region comprises or consists of the amino acid sequence of SEQ ID NO: 75. In some embodiments, the light chain constant region comprises or consists of the amino acid sequence of SEQ ID NO: 76. In some embodiments, the light chain constant region comprises or consists of the amino acid sequence of SEQ ID NO: 77.

In some embodiments, the individual is a mammal (*e.g.,* human, non-human primate, rat, mouse, cow, horse, pig, sheep, goat, dog, cat, *etc*.). In some embodiments, the individual is a human. In some embodiments, the individual is a clinical patient, a clinical trial volunteer, an experimental animal, *etc.* In some embodiments, the individual is younger than about 60 years old (including for example younger than about any of 50, 40, 30, 25, 20, 15, or 10 years old). In some embodiments, the individual is older than about 60 years old (including for example older than about any of 70, 80, 90, or 100 years old). In some embodiments, the individual is diagnosed with or genetically prone to one or more of the diseases or disorders described herein (such as autoimmune disease and/or inflammatory disease). In some embodiments, the individual has one or more risk factors associated with one or more diseases or disorders described herein.

The present application in some embodiments provides a method of delivering an anti-LIGHT antibody (such as any one of the anti-LIGHT antibodies described herein, e.g., an isolated anti-LIGHT antibody) to a cell producing LIGHT in an individual, the method comprising administering to the individual a composition comprising the anti-LIGHT antibody.

Many diagnostic methods for autoimmune disease and/or inflammatory disease or any other disease associated with LIGHT signaling and the clinical delineation of those diseases are known in the art. Such methods include, but are not limited to, *e.g.,* immunohistochemistry, PCR, and fluorescent in situ hybridization (FISH).

In some embodiments, the anti-LIGHT antibodies (*e.g.,* full-length anti-LIGHT antibodies) and/or compositions of the application are administered in combination with a second, third, or fourth agent (including, *e.g.*, steroids, non-steroidal anti-inflammatory drugs (NSAIDS) (e.g. salicylates, propionic acid derivatives, acetic acid derivatives, enolic acid derivatives, anthranilic acid derivatives, selective COX-2 inhibitors and sulfonanilides), glucocorticoids (such as cortisol, cortisone, prednisone, prednisolone, dexamethasone, betamethasone, triamcinolone), cytostatics (such as alkylating agents, antimetabolites, methotrexate, azathioprine, mercaptopurine or cytotoxic antibiotics), TNF inhibitors, IL-12 inhibitors, IL-23 inhibitors, α4β7 integrin inhibitors) to treat diseases or disorders associated with LIGHT signaling.

### Dosing and method of administering the anti-LIGHT antibodies

The dose of the anti-LIGHT antibody (such as isolated anti-LIGHT antibody) compositions administered to an individual (such as a human) may vary with the particular composition, the mode of administration, and the type of disease being treated. In some embodiments, the amount of the composition (such as composition comprising isolated anti-LIGHT antibody) is effective to result in an objective response (such as a partial response or a complete response) in the treatment of autoimmune disease and/or inflammatory disease. In some embodiments, the amount of the anti-LIGHT antibody composition is sufficient to result in a complete response in the individual. In some embodiments, the amount of the anti-LIGHT antibody composition is sufficient to result in a partial response in the individual. In some embodiments, the amount of the anti-LIGHT antibody composition administered (for example when administered alone) is sufficient to produce an overall response rate of more than about any of 20%, 25%, 30%, 35%, 40%, 45%, 50%, 55%, 60%, 64%, 65%, 70%, 75%, 80%, 85%, or 90% among a population of individuals treated with the anti-LIGHT antibody composition. Responses of an individual to the treatment of the methods described herein can be determined, for example, based on ACQ5 score.

In some embodiments, the amount of the composition (such as composition comprising isolated anti-LIGHT antibody) is sufficient to control symptoms and reduce the risk of exacerbations of the individual. In some embodiments, the amount of the composition is sufficient to control symptoms and reduce the risk of exacerbations of the individual. In some embodiments, the amount of the composition (for example when administered along) is sufficient to produce clinical benefit of more than about any of 50%, 60%, 70%, or 77% among a population of individuals treated with the anti-LIGHT antibody composition.

In some embodiments, the amount of the composition (such as composition comprising isolated anti-LIGHT antibody), alone or in combination with a second, third, and/or fourth agent, is an amount sufficient to control symptoms and reduce the risk of exacerbations in the same subject prior to treatment or compared to the corresponding activity in other subjects not receiving the treatment. Standard methods can be used to measure the magnitude of this effect, such as *in vitro* assays with purified enzyme, cell-based assays, animal models, or human testing.

In some embodiments, the amount of the anti-LIGHT antibody (such as a full-length anti-LIGHT antibody) in the composition is below the level that induces a toxicological effect (*i.e.,* an effect above a clinically acceptable level of toxicity) or is at a level where a potential side effect can be controlled or tolerated when the composition is administered to the individual.

In some embodiments, the amount of the composition is close to a maximum tolerated dose (MTD) of the composition following the same dosing regimen. In some embodiments, the amount of the composition is more than about any of 80%, 90%, 95%, or 98% of the MTD.

In some embodiments, the amount of an anti-LIGHT antibody (such as a full-length anti-LIGHT antibody) in the composition is included in a range of about 0.001 µg to about 1000 µg.

In some embodiments of any of the above aspects, the effective amount of anti-LIGHT antibody (such as a full-length anti-LIGHT antibody) in the composition is in the range of about 0.1 µg/kg to about 100 mg/kg of total body weight.

The anti-LIGHT antibody compositions can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal or transdermal. In some embodiments, sustained continuous release formulation of the composition may be used. In some embodiments, the composition is administered inhaled. In some embodiments, the composition is administered intravenously. In some embodiments, the composition is administered intraportally. In some embodiments, the composition is administered intraarterially. In some embodiments, the composition is administered intraperitoneally. In some embodiments, the composition is administered intrahepatically. In some embodiments, the composition is administered by hepatic arterial infusion. In some embodiments, the administration is to an injection site distal to a first disease site.

### Articles of Manufacture and Kits

In some embodiments of the application, there is provided an article of manufacture containing materials useful for the treatment of disease or condition associated with LIGHT signaling, (*e.g.*, autoimmune disease and/or inflammatory disease) or for delivering an anti-LIGHT antibody (such as a full-length anti-LIGHT antibody) to a cell producing LIGHT of the individual. The article of manufacture can comprise a container and a label or package insert on or associated with the container. Suitable containers include, for example, bottles, vials, syringes, *etc.* The containers may be formed from a variety of materials such as glass or plastic. Generally, the container holds a composition which is effective for treating a disease or disorder described herein, and may have a sterile access port (for example the container may be an intravenous solution bag or a vial having a stopper pierceable by a hypodermic injection needle). At least one active agent in the composition is an anti-LIGHT antibody of the application. The label or package insert indicates that the composition is used for treating the particular condition. The label or package insert will further comprise instructions for administering the anti-LIGHT antibody composition to the patient. Articles of manufacture and kits comprising combinatorial therapies described herein are also contemplated.

Package insert refers to instructions customarily included in commercial packages of therapeutic products that contain information about the indications, usage, dosage, administration, contraindications and/or warnings concerning the use of such therapeutic products. In some embodiments, the package insert indicates that the composition is used for treating disease or condition associated with LIGHT signaling (such as autoimmune disease and/or inflammatory disease). In some embodiments, the package insert indicates that the composition is used for treating disease or condition selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19.

Additionally, the article of manufacture may further comprise a second container comprising a pharmaceutically-acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

Kits are also provided that are useful for various purposes, *e.g.,* for treatment of disease or condition associated with LIGHT signaling (*e.g.,* autoimmune disease and/or inflammatory disease), or for delivering an anti-LIGHT antibody (such as a full-length anti-LIGHT antibody) to a cell producing LIGHT of the individual, optionally in combination with the articles of manufacture. Kits of the application include one or more containers comprising anti-LIGHT antibody composition (or unit dosage form and/or article of manufacture), and in some embodiments, further comprise another agent (such as the agents described herein) and/or instructions for use in accordance with any of the methods described herein. The kit may further comprise a description of selection of individuals suitable for treatment. Instructions supplied in the kits of the application are typically written instructions on a label or package insert (*e.g.,* a paper sheet included in the kit), but machine-readable instructions (*e.g.*, instructions carried on a magnetic or optical storage disk) are also acceptable.

For example, in some embodiments, the kit comprises a composition comprising an anti-LIGHT antibody (such as a full-length anti-LIGHT antibody). In some embodiments, the kit comprises a) a composition comprising any one of the anti-LIGHT antibodies described herein, and b) an effective amount of at least one other agent, wherein the other agent enhances the effects (*e.g.,* treatment effect, detecting effect) of the anti-LIGHT antibody. In some embodiments, the kit comprises a) a composition comprising any one of the anti-LIGHT antibodies described herein, and b) instructions for administering the anti-LIGHT antibody composition to an individual for treatment of a disease or condition associated with LIGHT signaling (*e.g.,* autoimmune disease and/or inflammatory disease). In some embodiments, the kit comprises a) a composition comprising any one of the anti-LIGHT antibodies described herein, b) an effective amount of at least one other agent, wherein the other agent enhances the effect (*e.g.,* treatment effect, detecting effect) of the anti-LIGHT antibody, and c) instructions for administering the anti-LIGHT antibody composition and the other agent(s) to an individual for treatment of a disease or condition associated with LIGHT signaling (*e.g.*, autoimmune disease and/or inflammatory disease. The anti-LIGHT antibody and the other agent(s) can be present in separate containers or in a single container. For example, the kit may comprise one distinct composition or two or more compositions wherein one composition comprises an anti-LIGHT antibody and another composition comprises another agent.

In some embodiments, the kit comprises a nucleic acid (or a set of nucleic acids) encoding an anti-LIGHT antibody (such as a full-length anti-LIGHT antibody). In some embodiments, the kit comprises a) a nucleic acid (or a set of nucleic acids) encoding an anti-LIGHT antibody, and b) a host cell for expressing the nucleic acid (or a set of nucleic acids). In some embodiments, the kit comprises a) a nucleic acid (or a set of nucleic acids) encoding an anti-LIGHT antibody, and b) instructions for i) expressing the anti-LIGHT antibody in a host cell, ii) preparing a composition comprising the anti-LIGHT antibody, and iii) administering the composition comprising the anti-LIGHT antibody to an individual for the treatment of a disease or condition associated with LIGHT signaling (*e.g.,* autoimmune disease and/or inflammatory disease. In some embodiments, the kit comprises a) a nucleic acid (or a set of nucleic acids) encoding an anti-LIGHT antibody, b) a host cell for expressing the nucleic acid (or a set of nucleic acids), and c) instructions for i) expressing the anti-LIGHT antibody in the host cell, ii) preparing a composition comprising the anti-LIGHT antibody, and iii) administering the composition comprising the anti-LIGHT antibody to an individual for the treatment of a disease or condition associated with LIGHT signaling (*e.g.,* autoimmune disease and/or inflammatory disease).

The kits of the application are in suitable packaging. Suitable packaging includes, but is not limited to, vials, bottles, jars, flexible packaging (*e.g.,* sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the anti-LIGHT antibody compositions generally include information as to dosage, dosing schedule, and route of administration for the intended treatment. The containers may be unit doses, bulk packages (*e.g.,* multi-dose packages) or sub-unit doses. For example, kits may be provided that contain sufficient dosages of an anti-LIGHT antibody (such as a full-length anti-LIGHT antibody) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the anti-LIGHT antibody and pharmaceutical compositions and instructions for use and packaged in quantities sufficient for storage and use in pharmacies, for example, hospital pharmacies and compounding pharmacies.

Those skilled in the art will recognize that several embodiments are possible within the scope and spirit of this application. The application will now be described in greater detail by reference to the following non-limiting examples. The following examples further illustrate the application but, of course, should not be construed as in any way limiting its scope.

### EXAMPLES

According to the following disclosed embodiments, the abbreviations apply: LIGHT (also known as TNFSF14, tumor necrosis factor superfamily member 14, tumor necrosis factor superfamily 14); HVEM (herpes simplex virus entry mediator, also known as TNFRSF14, CD270); LTβ R (lymphotoxin β receptor).

### Example 1: Generation of antigen recombinant protein

### Generation of recombinant LIGHT derived peptides

The cDNA encoding human, cynomolgus monkey, or mouse LIGHT was constructed into mammalian cell expression vectors through subcloning. His-tag and/or Fc-tag and/or other tags commonly used by those skilled in the art were added to the N-terminus of the cDNA mentioned above to construct and express fusion proteins containing human, cynomolgus monkey or mouse LIGHT, such as His-human LIGHT (His-huLIGHT, SEQ ID NO: 79), hFc-human LIGHT (hFc-huLIGHT, SEQ ID NO: 80), His- cynomolgus monkey LIGHT (His-cynoLIGHT, SEQ ID NO: 81), and His-mouse LIGHT (His-musLIGHT, SEQ ID NO: 82). Wherein, the "His" represents the His-tag, and the "hFc" represents the Fc segment of human IgG1 antibody.

The recombinant LIGHT proteins, including hFc-huLIGHT, His-huLIGHT, His-cynoLIGHT, and His-musLIGHT, were expressed and purified. In short, the expression vector plasmids containing the above-mentioned fusion protein genes were transfected into 293F cells, and the cells were cultured at 37 °C, 5% CO₂, and 120rpm for 5 days, followed by collection of cell culture medium.

According to the manufacturer's instruction, the recombinant protein with His-tag was purified using a nickel column (Ni). The specific operation was as follows: Immobilized metal affinity chromatography (IMAC) was performed using Ni-NTA (5ml) from Changzhou Smart-Lifesciences Biotechnology Co., Ltd.. Firstly, the nickel column was equilibrated with buffer A1 (1×PBS, pH 7.4) at a flow rate of 150cm/h. The pH of the supernatant was adjusted to 7.2-7.4, and the sample was loaded at room temperature with a flow rate of 5ml/min. Subsequently, the column was equilibrated again with 6 times the volume of A1 buffer at a flow rate of 5ml/min. Subsequently, the column was washed with 6-fold column volume of A2 buffer (1×PBS containing 30mM imidazole, pH 7.4) at a flow rate of 5ml/min. Finally, eluted with 10-fold column volume of B buffer (1×PBS containing 0.25M imidazole, pH 7.4). The eluate was collected, and the eluate was concentrated and replaced with PBS using a suitable molecular weight cut-off ultrafiltration tube.

According to the manufacturer's instruction, the recombinant protein with Fc-tag was purified by using Protein A affinity chromatography column. The specific operation was as follows: firstly, 6-fold column volume PBS buffer (containing 0.15M NaCl, pH 7.4) was used to equilibrate the protein A column at a flow rate of 150cm/h (flow rate 5ml/min). The supernatant of the culture medium (adjusted to pH 7.2-7.4) flowed through the column at a flow rate of 150cm/h (flow rate 5ml/min). After further equilibration of the column, 6 column volumes of 0.1M glycine buffer (containing 0.15M NaCl, pH 3.2) was used for elution, and the eluate was collected and the pH was adjusted to neutral. The target protein was replaced with PBS buffer using an ultrafiltration concentration tube, and the target protein concentration was determined using the BCA protein quantification kit after ultrafiltration concentration.

### Screening of anti-LIGHT single chain antibodies (scFv)

Construction of scFv antibody phage display library: His-huLIGHT was used as an antigen to immunize 6-8 week old BALB/c mice with equal (v/v) adjuvants. The serum of immunized mouse was collected, and the total IgG titer in the immunized mouse serum was detected by ELISA. After several rounds of immunization, the spleen of mice was used to establish a phage display library. In short, the spleen of immunized mice was taken, RNA was extracted, cDNA was obtained by reverse transcription, and V_{H} and V_{K} specific primers were used to amplify V_{H} and V_{L} fragments. After gel recovery and purification, V_{H} and V_{K} were ligated to construct scFv, which was cloned into the phage display vector pDAN3X. After ligation, TG1 phage display electroporation competent cells were transformed to obtain the scFv antibody phage display library.

Screening of anti-LIGHT single chain antibodies (scFv): Solid phase screening strategy was used to isolate phages that specifically bind to human LIGHT from the above phage display library. In short, His-huLIGHT or hFc-huLIGHT was coated on the ELISA plate, and 4×10¹¹ pfu phage display library was added to each well. The plate was incubated at 37 °C for 2 hours. After washing the plate, positive bacteriophages that specifically bind to human LIGHT were eluted using acidic elution buffer. After 3 rounds of repeated screening, the binding of a single positive clone to human LIGHT was further detected by ELISA. The positive scFv after multiple rounds of screening was sequenced to obtain candidate lead antibody sequences. The variable domain sequences of the heavy and light chains of the candidate lead antibody were shown in Tables 2 and 3.

### Example 2: Generation and characterization of full-length anti-LIGHT antibodies

### Generation of full-length anti-LIGHT antibodies

The sequenced V_{L} and V_{H} sequences of mouse anti-LIGHT antibodies were constructed into eukaryotic expression vectors pTTa1-L (containing the constant region of κ) and pTTa1-H4 (containing the constant region of human IgG4 heavy chain), respectively. The extracted plasmids expressing light or heavy chains were co-transfected into 293F cells and cultured at 37 °C, 8% CO₂, and 120rpm for 6 days.

The culture medium was purified using protein A affinity chromatography column. In short, the protein A column was first equilibrated using a 6-fold column volume of PBS buffer (containing 0.15M NaCl, pH 7.4) at a flow rate of 5ml/min. The supernatant of the culture medium (adjusted to pH 7.2-7.4) flows through the column at a flow rate of 5ml/min. After further equilibration of the column, 6-fold column volume of 0.1M glycine buffer (containing 0.15M NaCl, pH 3.2) was used for elution, the eluent was collected, and the pH was adjusted to neutral. The IgG solution was replaced with PBS buffer using a desalination column, and the IgG concentration was measured after ultrafiltration concentration to obtain full-length chimeric anti-LIGHT antibodies.

### Detection of the binding activity of anti-LIGHT antibodies by ELISA

ELISA binding assay: The purified chimeric antibody was subjected to binding assay with human LIGHT protein to identify the binding activity of candidate chimeric anti-LIGHT antibodies with human LIGHT. In short, the purified His-huLIGHT was dissolved in PBS solution, coated on a 96 well plate at a concentration of 0.1 µg/well, incubated overnight at 4 °C, and washed 6 times with PBST solution. Add 1% BSA (PBS dissolution) blocking solution, block at 37 °C for 1 hour, and wash with PBST solution 6 times. Subsequently, 100 µl/well gradient dilution of the tested anti-LIGHT antibody was added (starting concentration of 100 µg/ml, followed by a 1:3 gradient dilution), of which F19 was used as the positive control antibody, incubated at 37 °C for 1 hour, and washed 6 times with PBST solution. Subsequently, 100 µl of goat anti human IgG HRP secondary antibody (diluted 1:20000) was added to each well, incubated at 37 °C for 1 hour, and washed 6 times with PBST solution. 100 µl TMB was added to each well for color development, incubated at 37 °C in the dark for 5-10 minutes, and the reaction was terminated with 2M H₂SO₄. OD450nm was measured and the binding curves were generated using GraphPad Prism 5.0 to calculate the EC50 values of each candidate antibody binding to the antigen His huLIGHT.

As shown in Figure 1 and Table 5, the chimeric anti-LIGHT antibodies LT-m17, LT-m18 (Figure 1A), LT-m31, LT-m33, LT-m37 (Figure 1B), LT-m55, LT-m58, LT-m64, and LT-m85 (Figure 1C) can effectively bind to human LIGHT antigen, and their binding activity was superior to or comparable to the positive control antibody F19 (Cerecor).

**Table 5: The binding activity of anti-LIGHT antibodies to human LIGHT**

| **Antibody name** | **EC₅₀ value (µg/ml)** |
|---|---|
| LT-m17 | 0.08708 |
| LT-m18 | 0.08347 |
| LT-m31 | 0.02561 |
| LT-m33 | 0.02214 |
| LT-m37 | 0.07744 |
| LT-m55 | 0.03320 |
| LT-m58 | 0.02426 |
| LT-m64 | 0.01120 |
| LT-m85 | 0.01206 |
| F19 | 0.05888 |

### Detection the activity of anti-LIGHT antibodies on blocking the binding of LIGHT to LTβR by ELISA

The activity of anti-LIGHT antibodies on blocking the binding of LIGHT to its receptor LTβR was detected by ELISA, and the IC50 value was used as the standard to measure the neutralizing activity of candidate chimeric antibodies. In short, 100ng/well of human LTβR-His (from Staidson) was coated onto a 96 well plate, overnight at 4 °C, and washed 6 times with PBST solution. 1% BSA (PBS dissolution) blocking solution was added, blocked at 37 °C for 1 hour, and washed with PBST solution 6 times. Subsequently, 50 µl/well gradient dilution of the tested anti-LIGHT antibody was added (initial concentration of 100 µg/ml, 1:3 gradient dilution), of which F19 was used as the positive control antibody, and then 50 µl of purified hFc-huLIGHT with a concentration of 1 µg/ml was immediately added and incubated at 37 °C for 1 hour. After washing with PBST solution, 100 µl of goat anti human IgG-HRP secondary antibody (1:20000 dilution) was added to each well and incubated at 37 °C for 1 hour. Finally, 100 µl TMB was added to each well for color development, incubated at 37 °C in the dark for 5-10 minutes, and the reaction was terminated with 2M H₂SO₄. OD450nm was measured, and binding curves were generated using GraphPad Prism 5.0 to calculate IC50 values, and the ratio of IC50 (IC50 of each candidate antibody's IC50/ IC50 of F19 antibody) was calculated using F19 antibody as a benchmark.

As shown in Table 6, the chimeric anti-LIGHT antibodies LT-m17, LT-m18, LT-m31, LT-m33, LT-m37, LT-m55, LT-m58, LT-m64, and LT-m85 can effectively block the binding of LIGHT to its receptor LTβR, with higher neutralizing activity than the positive control antibody F19.

**Table 6: Anti-LIGHT antibody inhibits the activity of human LIGHT binding to human LTβR**

| **Antibody name** | **IC₅₀ ratio** |
|---|---|
| LT-m17 | 0.4210 |
| LT-m18 | 0.4330 |
| LT-m31 | 0.1850 |
| LT-m33 | 0.1410 |
| LT-m37 | 0.3010 |
| LT-m55 | 0.1675 |
| LT-m58 | 0.1727 |
| LT-m64 | 0.1738 |
| LT-m85 | 0.2410 |
| F19 | 1.0000 |

### Detection the activity of anti-LIGHT antibodies on blocking the binding of LIGHT and HVEM by ELISA

LIGHT not only binds to LTβR receptors, but also binds to HVEM receptor. The activity of candidate chimeric anti-LIGHT antibodies on blocking the binding of LIGHT to its receptor HVEM was detected by ELISA. The specific method was carried out according to the scheme of " Detection the activity of anti-LIGHT antibodies on blocking the binding of LIGHT to LTβR by ELISA" mentioned above, where the coating protein is HVEM-His (from Staidson).

As shown in Table 7, the exemplary chimeric anti-LIGHT antibodies LT-m17, LT-m18, LT-m31, LT-m33, and LT-m37 can effectively block the binding of LIGHT to its receptor HVEM, with higher neutralizing activity than the positive control antibody F19.

**Table 7: Anti-LIGHT antibodies inhibit human LIGHT binding to human HVEM activity**

| **Antibody name** | **IC₅₀ ratio** |
|---|---|
| LT-m17 | 0.516 |
| LT-m18 | 0.528 |
| LT-m31 | 0.208 |
| LT-m33 | 0.174 |
| LT-m37 | 0.435 |
| F19 | 1.0000 |

### Species cross activity detection of anti-LIGHT antibodies

The cross-binding activity of candidate chimeric anti-LIGHT antibodies with cynomolgus monkey LIGHT and mouse LIGHT was detected by ELISA. The specific method was carried out according to the scheme of " Detection of the binding activity of anti-LIGHT antibodies by ELISA" mentioned above, wherein the coated antigen was purified His-cynoLIGHT or His-musLIGHT protein.

The cross-binding activity results with cynomolgus monkey LIGHT were shown in Figure 2. Chimeric anti-LIGHT antibodies LT-m17, LT-m18 (Figure 2A), LT-m31, LT-m33, LT-m37 (Figure 2B), LT-m55, LT-m58, LT-m64, and LT-m85 (Figure 2C) all had strong cross-binding activity with cynomolgus monkey LIGHT.

The candidate chimeric anti-LIGHT antibodies LT-m17, LT-m18, LT-m31, LT-m33, and LT-m37 had weak cross-binding activity with mouse LIGHT, while other chimeric antibodies had no cross-binding activity with mouse LIGHT (data not shown).

### Determination the affinity of anti-LIGHT antibodies by BIAcore

Biacore 8K (GE) was used to characterize the binding affinity of anti-LIGHT antibodies to human LIGHT and cynomolgus monkey LIGHT. The anti-LIGHT antibodies were immobilized on the sensor chip CM5, and the affinity of antibodies of different concentrations to different species of LIGHT was detected, the binding and dissociation rates of antibodies were measured using SPR technology, and the binding affinity was determined.

The Kon, Koff, and Kd values of the anti-LIGHT antibodies were shown in Table 8, indicating that the exemplary chimeric anti-LIGHT antibodies LT-m31, LT-m33, LT-m55, LT-m58, LT-m64, and LT-m85 bind to human LIGHT antigen with high affinity.

Exemplary chimeric anti-LIGHT antibodies LT-m17, LT-m18, LT-m55, LT-m58, LT-m64, and LT-m85 also exhibited strong cross binding activity with cynomolgus monkey LIGHT.

**Table 8: Determination the affinity of anti-LIGHT antibodies by BIAcore**

| His-huLIGHT | | | |
|---|---|---|---|
| **Antibody name** | **Kon (1/Ms)** | **Koff (1/s)** | **Kd (M)** |
| LT-m31 | 1.02E+06 | 4.03E-05 | 3.95E-11 |
| LT-m33 | 1.73E+06 | 4.07E-05 | 2.35E-11 |
| LT-m55 | 8.768E+05 | 6.009E-05 | 6.854E-11 |
| LT-m58 | 3.846E+05 | 2.675E-05 | 6.956E-11 |
| LT-m64 | 1.007E+06 | 5.610E-05 | 5.572E-11 |
| LT-m85 | 7.830E+05 | 5.280E-05 | 6.743E-11 |

| His-cynoLIGHT | | | |
|---|---|---|---|
| LT-m17 | 7.27E+04 | 4.24E-06 | 5.82E-11 |
| LT-m18 | 7.91E+04 | 1.26E-05 | 1.60E-10 |
| LT-m55 | 9.557E+05 | 5.861E-05 | 6.1333E-11 |
| LT-m58 | 3.569E+05 | 1.237E-05 | 3.466E-11 |
| LT-m64 | 1.003E+06 | 4.888E-05 | 4.873E-11 |
| LT-m85 | 1.118E+06 | 6.721E-05 | 6.013E-11 |

### Activity detection of anti-LIGHT antibody inhibiting the activation of LTβR reporter gene by human LIGHT

After binding to LTβR receptors, LIGHT can activate the NF-κB pathway. The constructed LTβR receptor activation responsive luciferase reporter gene cell line (Hela-Rc32-NF-κB-Luc) was used to detect the ability of chimeric anti-LIGHT antibodies to inhibit the activation of LTβR receptors by LIGHT (including free LIGHT and cell surface LIGHT) and the initiation of luciferase signal, as a measure of the inhibitory effect of candidate chimeric anti-LIGHT antibodies on the LTβR-mediated signaling pathway activated by LIGHT.

### Assay on the activation of reporter gene by free LIGHT:

Firstly, a LTβR receptor activation responsive luciferase reporter gene cell line (Hela-Rc32-NF-κB-Luc) was constructed. In short, the cervical cancer cell line Hela-Rc32 expresses the LTβR receptor, and due to LIGHT binds to LTβR and activates the NF-κB pathway, the plasmid pGL4.32[luc2P/NF-κB-RE/Hygro] (Promega, Cat#E849A) was transfected into the Hela-Rc32 cell line. The luciferase gene of this plasmid is located downstream of the NF-κB response element, and luciferase can be activated when LIGHT binds to LTβR.

The construction method was as follows: According to the manufacturer's instruction, the plasmid vector pGL4.32[luc2P/NF-κB-RE/Hygro] (Promega, Cat#E849A) was transfected into Hela-Rc32 cells (ATCC, CRL-2972) using Lipofectamine^{®} 3000 reagent (Invitrogen, L3000-001), and cultured in complete medium containing antibiotics (DMEM, 10% FBS, 400 µg/ml Hygromycin B, 1% Penicillin Streptomycin abbreviated as PS) for monoclonal cell screening. Subsequently, monoclonal cells were selected and inoculated into a white transparent 96 well plate at a rate of 1×10⁴ cells/well. 50ng of purified His-huLIGHT protein and 200 µl complete culture medium were added, and incubated at 37 °C, 5% CO₂ for 24 hours. Then, 100 µl luciferase substrate was added to each well and incubated in dark for 5 minutes to detect fluorescence (RLU). Monoclonal cells with RLU> 1000 or RLU net increase rate (LIGHT stimulation: control) ≥ 4 were selected for expansion and further screening. After 3 rounds of repeated screening, a Hela-Rc32-NF-κB-Luc luciferase reporter gene cell line was obtained.

Then, the LTβR receptor activation responsive luciferase reporter gene cell line Hela-Rc32-NF-κB-Luc was seeded at a density of 1×10⁴ cells/well onto a white transparent 96 well plate and cultured overnight in 200 µl complete medium (DMEM, 10% FBS, 400 µg/ml Hygromycin B, 1% PS) at 37 °C and 5% CO₂. After removing the cell culture medium, 50 µl/well His-huLIGHT antigen at a concentration of 400ng/ml, as well as 50 µl/well gradient diluted chimeric anti-LIGHT antibody (starting concentration of 200 µg/ml, followed by a 1:3 gradient dilution) were added, of which F19 antibody was used as the positive control. After slight shaking and mixing, the cells were incubated at 37 °C and 5% CO₂ for 6 hours. Then, ONE-Glo^{™} Luciferase Assay System (Promega, E6120) was used, 50 µl ONE-Glo^{™} buffer solution was added to the reporter gene cells that have been equilibrated to room temperature. After shaking in dark for 10 minutes, the fluorescence reading (Luminescence 1000ms) was immediately detected using a microplate reader. GraphPad Prism 5.0 was used to generate signal intensity antibody concentration curves and the IC50 values of each candidate chimeric antibody for inhibiting the activation of LTβR-NF-κB signaling by free LIGHT was calculated.

As shown in Figure 3, the exemplary chimeric anti-LIGHT antibodies LT-m17, LT-m31, LT-m33, LT-m37 (Figure 3A), LT-m55, LT-m58, LT-m64, and LT-m85 (Figure 3B) can effectively inhibit the activation of LTβR initiated luciferase signal by free LIGHT, and the inhibitory activity was superior to or equivalent to the positive control antibody F19.

### Assay on the activation of reporter genes by cell surface LIGHT:

Firstly, a stable cell line expressing LIGHT (HEK293-LIGHT) was constructed. The construction method was as follows: According to the manufacturer's instruction, the plasmid expressing LIGHT (from Staidson) was transfected into HEK293 cells using Lipofectamine^{®} 2000 reagent (Invitrogen, 11668-027), and cultured in complete medium containing antibiotics (DMEM, 10% FBS, 300 µg/ml Zeocin, 1% PS), and monoclonal screening was performed and expanded.

About 10 monoclonal antibodies were selected for cell surface LIGHT labeling. First, 20 µg/mL anti-LIGHT antibody F23-hFc (Cerecor) was added to the cells, incubated at 4 °C for 1 hour, washed the cells three times with DPBS buffer, and goat-anti-human Fc-FITC secondary antibody (Southern Biotech, 2063-02, 1:1000 dilution) was added, incubated at 4 °C in dark for 1 hour, and washed the cells three times with DPBS. Subsequently, flow cytometry was used to detect fluorescence, and LIGHT positive cells were sorted and collected.

The selected LIGHT positive cell lines were then identified by ELISA. In short, 4 ×10⁴ cells/well of the above-mentioned LIGHT positive cells were seeded onto a 96 well plate and cultured overnight at 37 °C and 5% CO₂. After washing with 0.1% PBST buffer, 100 µl 1% BSA solution was added to each well and blocked at 37 °C for 30 minutes. Then, gradient concentration of anti-LIGHT antibody F23-IgG4 (Cerecor) was added and incubated at 37 °C for 1 hour. After washing, anti-human Fc-AP secondary antibody and color reagent PNPP were added. The OD405nm value was read by a microplate reader, and finally HEK293-LIGHT stable cell lines were obtained.

Then, the LTβR receptor activation responsive luciferase reporter gene cell line Hela-Rc32-NF-κB-Luc was seeded into a white transparent 96 well plate at a density of 1×10⁴ cells/well and cultured overnight at 37 °C and 5% CO₂. After removing the cell culture medium, 50 µl/well of HEK293-LIGHT stable cell suspension with a density of 2×10⁵ cells/ml, as well as 50 µl/well gradient diluted chimeric anti-LIGHT antibody (initial concentration of 1600 µg/ml, diluted in a 1:3 gradient) were added, and F19 antibody was used as the positive control. After slight shaking and mixing, the plate was incubated at 37 °C and 5% CO₂ for 6 hours. Then, the fluorescence reading (Luminescence 1000ms) was detected using ONE-Glo ^{™} Luciferase Assay System detection method, as described above.

As shown in Figure 4, the exemplary chimeric anti-LIGHT antibodies LT-m17, LT-m31, LT-m33, LT-m37 (Figure 4A), LT-m55, LT-m58, LT-m64 (Figure 4B), and LT-m85 (Figure 4C) can effectively inhibit the activation of LTβR initiated luciferase signal by the cell surface LIGHT, and the inhibitory activity was superior to the positive control antibody F19.

### Activity detection of anti-LIGHT antibody inhibiting the activation of HVEM reporter gene by human LIGHT

In addition to LTβR, LIGHT also binds to HVEM receptors. After binding with HVEM, LIGHT can also activate the NF-κB signaling pathway. By constructing a HVEM receptor activation responsive luciferase reporter gene cell line (293T-HVEM-NF-κB-Luc), which enables the activation of luciferase upon binding of LIGHT to HVEM, the inhibitory effect of anti-LIGHT antibodies on the activation of HVEM receptor initiated luciferase signaling by free LIGHT was detected as a measure of the activity of candidate chimeric antibodies in inhibiting the activation of HVEM mediated signaling pathway by LIGHT.

First, a HVEM receptor activation responsive luciferase reporter gene cell line (293T-HVEM-NF-κB-Luc) was constructed. In short, according to the manufacturer's instruction, the nucleic acid sequence encoding the HVEM-GFP-Blasticidin fusion protein was constructed into a lentiviral vector shuttle plasmid (from Staidson) using a lentiviral packaging system, and the recombinant lentivirus was packaged. Subsequently, 293T cells were seeded into a 24 well plate at 5×10⁴ cells/well. After discarding the supernatant, the recombinant lentivirus was added according to different infection ratios (1, 2.5, or 5 MOI), and DMEM medium containing 20% FBS was added to 1.2ml, and cultured at 37 °C and 5% CO₂ for 72 hours. Next, the culture medium containing 10 µg/ml Blasticidin was used for culture and screening. After 10-14 days, cells with positive GFP fluorescence signals (the top 20% of strong signals) were sorted and collected using flow cytometry. Subsequently, the plasmid pGL4.32[luc2P/NF-κB-RE/Hygro] (Promega, Cat#E849A) expressing luciferase was transfected into the GFP positive cells described above. GFP positive cells were then sorted and collected using flow cytometry. Mono clones were screened in complete medium containing antibiotics (DMEM, 300 µg/ml Hygromycin B, 10 µg/ml Blasticidin, 1% PS), and after expanded culture, the HVEM receptor activation responsive luciferase reporter gene cell line 293T-HVEM-NF-κB-Luc was obtained.

Then, the HVEM receptor activation responsive luciferase reporter gene cell line 293T-HVEM-NF-κB-Luc was seeded at a density of 1×10⁴ cells/well onto a white transparent 96 well plate and cultured overnight in 200 µl complete medium (DMEM, 10% FBS, 300 µg/ml Hygromycin B, 10 µg/ml Blasticidin, 1% PS) at 37 °C and 5% CO₂. After removing the cell culture medium, 50 µl/well of His-huLIGHT antigen at a concentration of 400ng/ml, as well as 50 µl/well gradient diluted chimeric anti-LIGHT antibody to be tested (starting concentration up to 200 µg/ml, followed by a 1:3 gradient dilution) were added, and F19 antibody was used as the positive control. After slight shaking and mixing, the plate was incubated at 37 °C and 5% CO₂ for 6 hours. The fluorescence value was detected by ONE-Glo^{™} Luciferase Assay System detection method as described above. The signal intensity antibody concentration curves were generated using GraphPad Prism 5.0 and the IC50 values of the inhibitory activity of each candidate chimeric antibody on the activation of HVEM-NF-κB signal by free LIGHT was calculated.

As shown in Figure 5 and Table 9, the exemplary chimeric anti-LIGHT antibodies LT-m17, LT-m31, LT-m33, LT-m37 (Figure 5A and Table 9), LT-m55, LT-m64, LT-m85 (Figure 5B and Table 9), and LT-m58 (Figure 5C) can effectively inhibit the activation of HVEM initiated luciferase signal by free LIGHT, and the inhibitory activity is comparable to the positive control antibody F19.

**Table 9: Anti-LIGHT antibody inhibits the activity of free LIGHT activated HVEM reporter genes**

| **Antibody name** | **IC₅₀ value (µg/ml)** |
|---|---|
| LT-m17 | 0.1844 |
| LT-m31 | 0.1050 |
| LT-m33 | 0.2071 |
| LT-m37 | 0.2638 |
| LT-m55 | 0.2644 |
| LT-m58 | 0.3181 |
| LT-m64 | 0.2641 |
| LT-m85 | 0.1241 |
| F19 | 0.3388 |

### Example 3: Humanization of anti-LIGHT antibodies

Based on the typical CDR structures of the V_{H}/V_{L} of the obtained chimeric antibody LT-m37, the variable region sequences of the heavy and light chains were compared with the sequences of the antibody germline database to obtain a high homology human germline template. The CDR regions of mouse antibodies was transplanted to the selected germline templates to generate humanized variable regions, which are then recombined with corresponding human IgG constant regions (preferably IgG4 heavy chain and κ light chain). Subsequently, based on the three-dimensional structure of mouse antibodies, recovery mutations were performed on the embedded residues, residues that directly interact with the CDR region, and residues that have a significant impact on the conformation of V_{L} and V_{H}. The chemically unstable amino acid residues in the CDR region were optimized to obtain the final humanized molecules hum_LT-m37-1-hum_LT-m37-16, respectively. The variable domain sequences of the heavy and light chains of humanized antibodies were shown in Tables 2 and 3.

According to the humanization method described above, the chimeric antibody LT-m58 was also humanized, and the risk points in the CDR region were further optimized to obtain the corresponding humanized molecules hum_LT-m58-1~hum_LT-m58-6. The variable domain sequences of the heavy and light chains of the humanized antibodies mentioned above were shown in Tables 2A and 3A.

According to the humanization method described above, the chimeric antibody LT-m85 was also humanized to obtain the corresponding humanized molecules hum_LT-m85-1-hum_LT-m85-9. The variable domain sequences of the heavy and light chains of the humanized antibodies mentioned above were shown in Tables 2B and 3B.

### Example 4: Characterization of humanized antibodies

The corresponding experimental protocol in Example 2 was used to detect the affinity and biological activity of humanized anti-LIGHT antibodies, including affinity detection, neutralization activity detection, and biological activity detection.

### Detection of the binding activity of humanized anti-LIGHT antibodies by ELISA

The protocol of "Detection the binding activity of anti-LIGHT antibodies by ELISA" described in Example 2 was used to test the binding ability of humanized antibodies (human IgG4 form) to human LIGHT.

The results of the binding activity of the humanized molecular derived from the chimeric antibody LT-m37 were shown in Figure 6 and Tables 10A-10B. Humanized anti-LIGHT antibodies can effectively bind to human LIGHT antigen, and their binding activity was superior to or comparable to the positive control antibody F19.

**Table 10A: Binding activity of humanized anti-LIGHT antibodies to human LIGHT**

| **Antibody name** | **EC₅₀ value(µg/ml)** |
|---|---|
| hum_LT-m37-1 | 0.06624 |
| hum_LT-m37-2 | 0.08102 |
| hum_LT-m37-3 | 0.08752 |
| hum_LT-m37-4 | 0.08592 |
| hum_LT-m37-5 | 0.08374 |
| hum_LT-m37-6 | 0.09392 |
| hum_LT-m37-7 | 0.08009 |
| hum_LT-m37-8 | 0.07955 |
| hum_LT-m37-9 | 0.08249 |
| hum_LT-m37-10 | 0.09178 |
| hum_LT-m37-11 | 0.09073 |
| hum_LT-m37-12 | 0.08785 |
| F19 | 0.08620 |

**Table 10B: Binding activity of humanized anti-LIGHT antibodies to human LIGHT**

| **Antibody name** | **EC₅₀ value(nM)** |
|---|---|
| hum_LT-m37-13 | 0.04218 |
| hum_LT-m37-14 | 0.04642 |
| hum_LT-m37-15 | 0.06580 |
| hum_LT-m37-16 | 0.03687 |
| F19 | 0.1675 |

The results of the binding activity of the humanized molecular derived from the chimeric antibody LT-m58 were shown in Table 10C. The humanized anti-LIGHT antibodies hum_LT-m58-1~hum_LT-m58-6 can effectively bind to human LIGHT antigen, and their binding activity was superior to or comparable to the positive control antibody F19.

**Table 10C: Binding activity of humanized anti-LIGHT antibody of LT-m58 with human LIGHT**

| **Antibody name** | **EC₅ value(nM)** |
|---|---|
| hum_LT-m58-1 | 0.01410 |
| hum_LT-m58-2 | 0.01543 |
| hum_LT-m58-3 | 0.01120 |
| hum_LT-m58-4 | 0.00617 |
| hum_LT-m58-5 | 0.03046 |
| hum_LT-m58-6 | 0.03486 |
| F19 | 0.05197 |

The results of the binding activity of the humanized molecular derived from the chimeric antibody LT-m85 were shown in Table 10D. The humanized anti-LIGHT antibodies hum_LT-m85-1-hum_LT-m85-9 can effectively bind to human LIGHT antigen, and their binding activity was better than the positive control antibody F19.

**Table 10D: Binding activity of humanized anti-LIGHT antibody of LT-m85 with human LIGHT**

| **Antibody name** | **EC₅₀ value(nM)** |
|---|---|
| hum_LT-m85-1 | 0.01228 |
| hum_LT-m85-2 | 0.01306 |
| hum_LT-m85-3 | 0.00869 |
| hum_LT-m85-4 | 0.01268 |
| hum_LT-m85-5 | 0.00610 |
| hum_LT-m85-6 | 0.00479 |
| hum_LT-m85-7 | 0.00542 |
| hum_LT-m85-8 | 0.00951 |
| hum_LT-m85-9 | 0.00200 |
| F19 | 0.05029 |

### Detection of the activity of humanized anti-LIGHT antibodies on blocking the binding of LIGHT to LTβR by ELISA

The protocol of " Detection the activity of anti-LIGHT antibodies on blocking the binding of LIGHT to LTβR by ELISA " described in Example 2 was used to detect the activity of humanized anti-LIGHT antibodies (in human IgG4 form) in blocking the binding of LIGHT to LTβR, to measure the inhibitory ability of humanized anti-LIGHT antibodies on the pro-inflammatory mechanism mediated by LIGHT.

As shown in Figure 7 and Tables 11A-11B, the humanized anti-LIGHT antibodies derived from the chimeric antibody LT-m37 can effectively block the binding of human LIGHT to LTβR, and their blocking activity was superior to the positive control antibody F19.

In addition, compared with the chimeric antibody LT-m37, the exemplary humanized anti-LIGHT antibodies hum_LT-m37-13, hum_LT-m37-14, hum_LT-m37-15, and hum_LT-m37-16 (Figure 7C and Table 11B) exhibited comparable blocking activity on the binding of human LIGHT to LTβR.

**Table 11A: Humanized anti-LIGHT antibodies inhibit the binding activity of human LIGHT to human LTβR**

| **Antibody name** | **IC₅₀ (nM)** |
|---|---|
| hum_LT-m37-1 | 0.07541 |
| hum_LT-m37-2 | 0.05984 |
| hum_LT-m37-4 | 0.07480 |
| hum_LT-m37-5 | 0.07711 |
| hum_LT-m37-6 | 0.07527 |
| hum_LT-m37-7 | 0.06904 |
| hum_LT-m37-8 | 0.06761 |
| hum_LT-m37-9 | 0.08304 |
| hum_LT-m37-11 | 0.08639 |
| hum_LT-m37-12 | 0.07728 |
| F19 | 0.2666 |

**Table 11B: Humanized anti-LIGHT antibodies inhibit the binding activity of human LIGHT to human LTβR**

| **Antibody name** | **IC₅₀ (nM)** |
|---|---|
| hum_LT-m37-13 | 0.8672 |
| hum_LT-m37-14 | 1.0170 |
| hum_LT-m37-15 | 0.8441 |
| hum_LT-m37-16 | 0.8081 |
| LT-m37 | 0.8662 |
| F19 | 2.2210 |

The results of the neutralizing activity of humanized molecules derived from the chimeric antibody LT-m58 were shown in Table 11C. Humanized anti-LIGHT antibodies hum_LT-m58-1~hum_LT-m58-6 can effectively block the binding of human LIGHT to LTβR, and their blocking activity was superior to the positive control antibody F19.

**Table 11C: Humanized anti-LIGHT antibody of LT-m58 inhibits human LIGHT binding to human LTβR activity**

| **Antibody name** | **IC₅₀ value(nM)** |
|---|---|
| hum_LT-m58-1 | 0.2908 |
| hum_LT-m58-2 | 0.2584 |
| hum_LT-m58-3 | 0.1692 |
| hum_LT-m58-4 | 0.2082 |
| hum_LT-m58-5 | 0.7458 |
| hum_LT-m58-6 | 0.6284 |
| F19 | 1.494 |

The results of the neutralizing activity of humanized molecules derived from the chimeric antibody LT-m85 were shown in Table 11D. Humanized anti-LIGHT antibodies hum_LT-m85-1~hum_LT-m85-9 can effectively block the binding of human LIGHT to LTβR, and their blocking activity was superior to the positive control antibody F19.

**Table 11D: Humanized anti-LIGHT antibodies of LT-m85 inhibit human LIGHT binding to human LTβR activity**

| **Antibody name** | **IC₅₀ value(nM)** |
|---|---|
| hum_LT-m85-1 | 0.2736 |
| hum_LT-m85-2 | 0.2654 |
| hum_LT-m85-3 | 0.2821 |
| hum_LT-m85-4 | 0.3085 |
| hum_LT-m85-5 | 0.1737 |
| hum_LT-m85-6 | 0.1951 |
| hum_LT-m85-7 | 0.2228 |
| hum_LT-m85-8 | 0.1893 |
| hum_LT-m85-9 | 0.2045 |
| F19 | 1.2190 |

### Species cross activity detection of humanized anti-LIGHT antibodies

The ELISA method described in Example 2 was used to detect the species cross binding activity of humanized antibodies (human IgG4 form) with cynomolgus monkey LIGHT and mouse LIGHT.

As shown in Table 12A, the humanized anti-LIGHT antibody derived from the chimeric antibody LT-m37 exhibited strong cross binding activity with cynomolgus monkey LIGHT.

Humanized anti-LIGHT antibodies had weak or no cross binding activity with mouse LIGHT (data not shown).

**Table 12A: Cross binding ability of anti-LIGHT antibodies with cynomolgus monkey LIGHT**

| **Antibody name** | **EC₅₀ (nM)** |
|---|---|
| hum_LT-m37-1 | 0.04683 |
| hum_LT-m37-2 | 0.05169 |
| hum_LT-m37-3 | 0.05543 |
| hum_LT-m37-4 | 0.05268 |
| hum_LT-m37-5 | 0.05135 |
| hum_LT-m37-6 | 0.05884 |
| hum_LT-m37-7 | 0.04743 |
| hum_LT-m37-8 | 0.04486 |
| hum_LT-m37-9 | 0.04783 |
| hum_LT-m37-10 | 0.04626 |
| hum_LT-m37-11 | 0.04937 |
| hum_LT-m37-12 | 0.05301 |
| hum_LT-m37-13 | 0.10376 |
| hum_LT-m37-14 | 0.10400 |
| hum_LT-m37-15 | 0.13013 |
| hum_LT-m37-16 | 0.10362 |
| LT-m37 | 0.1566 |
| F19 | 0.2235 |

The results of species cross binding activity of humanized molecules derived from chimeric antibody LT-m58 were shown in Table 12B. Exemplary anti-LIGHT antibodies hum_LT-m58-5 and hum_LT-m58-6 had a strong cross binding activity with cynomolgus monkey LIGHT, but no cross-binding activity with mouse LIGHT (data not shown).

**Table 12B: Cross binding ability of humanized anti-LIGHT antibody of LT-m58 with cynomolgus monkey LIGHT**

| **Antibody name** | **EC₅₀ value(nM)** |
|---|---|
| hum_LT-m58-5 | 0.00443 |
| hum_LT-m58-6 | 0.00258 |
| F19 | 0.03809 |

The results of species cross binding activity of humanized molecules derived from chimeric antibody LT-m85 were shown in Table 12C. Exemplary anti-LIGHT antibodies hum_LT-m85-1, hum_LT-m85-2, hum_LT-m85-3, and hum_LT-m85-4 had strong cross binding activity with cynomolgus monkey LIGHT, but no cross binding activity with mouse LIGHT (data not shown).

**Table 12C: Cross binding ability of humanized anti-LIGHT antibody LT-m85 with cynomolgus monkey LIGHT**

| **Antibody name** | **EC₅₀ value(nM)** |
|---|---|
| hum_LT-m85-1 | 0.0089 |
| hum_LT-m85-2 | 0.0142 |
| hum_LT-m85-3 | 0.0155 |
| hum_LT-m85-4 | 0.0033 |
| F19 | 0.0131 |

### Determination of the affinity of humanized anti-LIGHT antibodies by BIAcore

Biacore 8K (GE) was used to characterize the binding affinity of humanized anti-LIGHT antibodies to human LIGHT. The anti-LIGHT antibody was immobilized on the sensor chip CM5, and the affinity of different concentrations of humanized anti-LIGHT antibody (IgG4 form) to human LIGHT was detected. The binding rate and dissociation rate of the antibody were measured using SPR technology, and the binding affinity was determined.

The Kon, Koff, and Kd values of the anti-LIGHT antibodies were shown in Table 13. It can be seen that the exemplary humanized anti-LIGHT antibodies hum_LT-m37-4, hum_LT-m37-5, hum_LT-m37-6, hum_LT-m37-7, hum_LT-m37-8, hum_LT-m37-9, hum_LT-m37-11, and hum_LT-m37-12 bind to the human LIGHT antigen with high affinity.

**Table 13: Affinity of humanized anti-LIGHT antibodies detected by Biacore**

| **Antibody name** | **Kon(1/Ms)** | **Koff(1/s)** | **Kd (M)** |
|---|---|---|---|
| **Hum_LT-m37-4** | 2.948E+05 | 7.097E-05 | 2.41E-10 |
| **Hum_LT-m37-5** | 1.102E+06 | 6.337E-05 | 5.751E-11 |
| **Hum_LT-m37-6** | 1.287E+06 | 5.252E-05 | 4.080E-11 |
| **Hum_LT-m37-7** | 1.303E+06 | 5.340E-05 | 4.097E-11 |
| **Hum_LT-m37-8** | 1.279E+06 | 4.856E-05 | 3.797E-11 |
| **Hum_LT-m37-9** | 2.097E+05 | 8.026E-05 | 3.83E-10 |
| **Hum_LT-m37-11** | 1.241E+06 | 7.692E-05 | 6.20E-11 |
| **Hum_LT-m37-12** | 1.175E+06 | 6.754E-05 | 5.75E-11 |

### Activity detection of humanized anti-LIGHT antibody inhibiting the activation of LTβR reporter gene by free LIGHT

The experimental protocol of "Activity detection of anti-LIGHT antibody inhibiting the activation of LTβR reporter gene by human LIGHT" described in Example 2 was used to detect the inhibitory activity of humanized anti-LIGHT antibody on the activation of LTβR reporter gene by free LIGHT.

As shown in Figure 8 and Tables 14A-14B, humanized anti-LIGHT antibodies (human IgG4 form) derived from the chimeric antibody LT-m37 were able to effectively inhibit the activation of LTβR initiated luciferase signal by free LIGHT, and the inhibitory activity was comparable to the positive control antibody F19.

In addition, compared with the chimeric antibody LT-m37, the exemplary humanized anti-LIGHT antibodies hum_LT-m37-13, hum_LT-m37-14, hum_LT-m37-15, and hum_LT-m37-16 (Figure 8C and Table 14B) exhibited comparable inhibitory activity against the activation of LTβR initiated luciferase signal by free LIGHT.

**Table 14A: Humanized anti-LIGHT antibodies inhibit the activation of LTβR reporter genes by free LIGHT**

| **Antibody name** | **IC₅₀ (µg/ml)** |
|---|---|
| hum_LT-m37-1 | 0.2690 |
| hum_LT-m37-2 | 0.2931 |
| hum_LT-m37-3 | 0.3988 |
| hum_LT-m37-4 | 0.1289 |
| hum_LT-m37-5 | 0.1602 |
| hum_LT-m37-6 | 0.2239 |
| hum_LT-m37-7 | 0.1353 |
| hum_LT-m37-8 | 0.1699 |
| hum_LT-m37-9 | 0.2273 |
| hum_LT-m37-10 | 0.1532 |
| hum_LT-m37-11 | 0.2029 |
| hum_LT-m37-12 | 0.1439 |
| F19 | 0.4621 |

**Table 14B: Humanized anti-LIGHT antibodies inhibit the activation of LTβR reporter genes by free LIGHT**

| **Antibody name** | **IC₅₀ (nM)** |
|---|---|
| hum_LT-m37-13 | 0.2268 |
| hum_LT-m37-14 | 0.1820 |
| hum_LT-m37-15 | 0.2187 |
| hum_LT-m37-16 | 0.1575 |
| LT-m37 | 0.2567 |
| F19 | 0.3540 |

The LTβR inhibitory activity results of humanized molecules derived from chimeric antibody LT-m58 were shown in Table 14C. Exemplary anti-LIGHT antibodies hum_LT-m58-1 and hum_LT-m58-2 can effectively inhibit the activation of LTβR initiated luciferase signal by LIGHT, and the inhibitory activity was superior to the positive control antibody F19.

**Table 14C: Humanized anti-LIGHT antibody of LT-m58 inhibits the activation of LTβR reporter genes by free LIGHT**

| | |
|---|---|
| **Antibody name** | **IC₅₀ value(nM)** |
| hum_LT-m58-1 | 0.1826 |
| hum_LT-m58-2 | 0.1353 |
| F19 | 0.3988 |

The LTβR inhibitory activity results of humanized molecules derived from the chimeric antibody LT-m85 were shown in Table 14D. Exemplary anti-LIGHT antibodies hum_LT-m85-1, hum_LT-m85-2, hum_LT-m85-3, and hum_LT-m85-4 can effectively inhibit the activation of LTβR initiated luciferase signal by LIGHT, and this inhibitory activity was superior to the positive control antibody F19.

**Table 14D: Humanized anti-LIGHT antibodies of LT-m85 inhibit the activation of LTβR initiated luciferase signaling by LIGHT**

| **Antibody name** | **IC₅₀ value(nM)** |
|---|---|
| hum_LT-m85-1 | 0.6491 |
| hum_LT-m85-2 | 0.0613 |
| hum_LT-m85-3 | 0.1589 |
| hum_LT-m85-4 | 0.2525 |
| F19 | 1.8450 |

### Activity detection of humanized anti-LIGHT antibody inhibiting the activation of LTβR reporter gene by LIGHT on the cell surface

The experimental protocol of "Activity detection of anti-LIGHT antibody inhibiting the activation of LTβR reporter gene by human LIGHT" described in Example 2 was used to detect the inhibitory activity of humanized anti-LIGHT antibody on the activation of LTβR reporter gene by LIGHT on the cell surface.

As shown in Figure 9 and Tables 15A-15B, humanized anti-LIGHT antibodies (human IgG4 form) derived from the chimeric antibody LT-m37 were able to effectively inhibit the activation of LTβR initiated luciferase signal by LIGHT on the cell surface, and the inhibitory activity was superior to the positive control antibody F19.

In addition, compared with the chimeric antibody LT-m37, the exemplary humanized anti-LIGHT antibodies (human IgG4 form) hum_LT-m37-13, hum_LT-m37-14, hum_LT-m37-15, and hum_LT-m37-16 (Figure 9C and Table 15B) exhibited comparable inhibitory activity on the activation of LTβR initiated luciferase signal by LIGHT on the cell surface.

**Table 15A: Humanized anti-LIGHT antibodies inhibit the activation of LTβR reporter genes by LIGHT on the cell surface**

| **Antibody name** | **IC₅₀ (µg/ml)** |
|---|---|
| hum_LT-m37-1 | 5.388 |
| hum_LT-m37-2 | 3.540 |
| hum_LT-m37-3 | 1.791 |
| hum_LT-m37-4 | 2.564 |
| hum_LT-m37-5 | 2.220 |
| hum_LT-m37-6 | 1.857 |
| hum_LT-m37-7 | 3.716 |
| hum_LT-m37-8 | 3.224 |
| hum_LT-m37-9 | 3.861 |
| hum_LT-m37-10 | 4.456 |
| hum_LT-m37-11 | 2.930 |
| hum_LT-m37-12 | 2.574 |
| F19 | 76.70 |

**Table 15B: Humanized anti-LIGHT antibodies inhibit the activation of LTβR reporter genes by LIGHT on the cell surface**

| **Antibody name** | **IC₅₀ (nM)** |
|---|---|
| hum_LT-m37-13 | 0.8669 |
| hum_LT-m37-14 | 1.8640 |
| hum_LT-m37-15 | 1.2850 |
| hum_LT-m37-16 | 2.5510 |
| LT-m37 | 2.2810 |
| F19 | 6.0640 |

The LTβR inhibitory activity results of humanized molecules derived from chimeric antibody LT-m58 were shown in Table 15C. Exemplary anti-LIGHT antibodies hum_LT-m58-1 and hum_LT-m58-2 can effectively inhibit the activation of LTβR initiated luciferase signal by LIGHT on the cell surface, and the inhibitory activity is superior to the positive control antibody F19.

**Table 15C: Humanized anti-LIGHT antibody of LT-m58 inhibits the activation of LTβR reporter gene by LIGHT on the cell surface**

| **Antibody name** | **IC₅₀ value(nM)** |
|---|---|
| hum_LT-m58-1 | 8.060 |
| hum_LT-m58-2 | 5.914 |
| F19 | 41.09 |

The LTβR inhibitory activity results of humanized molecules derived from the chimeric antibody LT-m85 were shown in Table 15D. Exemplary anti-LIGHT antibodies hum_LT-m85-1, hum_LT-m85-2, hum_LT-m85-3, and hum_LT-m85-4 can effectively inhibit the activation of LTβR initiated luciferase signal by LIGHT on the cell surface, and the inhibitory activity was superior to the positive control antibody F19.

**Table 15D: Humanized anti-LIGHT antibodies of LT-m85 inhibit the activation of the LTβR reporter gene by LIGHT on the cell surface**

| **Antibody name** | **IC₅₀ value(nM)** |
|---|---|
| hum_LT-m85-1 | 4.241 |
| hum_LT-m85-2 | 6.017 |
| hum_LT-m85-3 | 4.683 |
| hum_LT-m85-4 | 2.564 |
| F19 | 44.81 |

### Activity detection of humanized anti-LIGHT antibodies inhibiting the activation of HVEM reporter gene by free LIGHT

The experimental protocol described in Example 2 was used to detect the activity of human LIGHT activated HVEM reporter genes inhibited by anti-LIGHT antibodies, the inhibitory activity of humanized anti-LIGHT antibodies on the free LIGHT activated HVEM mediated signaling pathway was detected.

As shown in Figure 10 and Tables 16A-16B, exemplary humanized anti-LIGHT antibodies (human IgG4 form) derived from chimeric antibody LT-m37, including hum_LT-m37-1, hum_LT-m37-2, hum_LT-m37-3, hum_LT-m37-4, hum_LT-m37-5, hum_LT-m37-6 (Figure 10A and Table 16A), hum_LT-m37-14, hum_LT-m37-15, and hum_LT-m37-16 (Figure 10B and Table 16B), were able to effectively inhibit the activation of HVEM initiated luciferase signal by free LIGHT, and the inhibitory activity was comparable to the positive control antibody F19.

**Table 16A: Humanized anti-LIGHT antibodies inhibit the activation of HVEM reporter genes by free LIGHT**

| **Antibody name** | **IC₅₀ (µg/ml)** |
|---|---|
| hum_LT-m37-1 | 0.11760 |
| hum_LT-m37-2 | 0.09183 |
| hum_LT-m37-3 | 0.07474 |
| hum_LT-m37-4 | 0.06772 |
| hum_LT-m37-5 | 0.06200 |
| hum_LT-m37-6 | 0.05180 |
| F19 | 0.10370 |

**Table 16B: Humanized anti-LIGHT antibodies inhibit the activation of HVEM reporter genes by free LIGHT**

| **Antibody name** | **IC₅₀ (nM)** |
|---|---|
| hum_LT-m37-14 | 0.1614 |
| hum_LT-m37-15 | 0.1199 |
| hum_LT-m37-16 | 0.1794 |
| LT-m37 | 0.4017 |
| F19 | 0.1824 |

The HVEM inhibitory activity results of humanized molecules derived from chimeric antibody LT-m58 were shown in Table 16C. Exemplary anti-LIGHT antibodies hum_LT-m58-1 and hum_LT-m58-2 can effectively inhibit the activation of HVEM initiated luciferase signal by free LIGHT, and the inhibitory activity was superior to the positive control antibody F19.

**Table 16C: Humanized anti-LIGHT antibodies of LT-m58 inhibit the activation of HVEM reporter genes by free LIGHT**

| **Antibody name** | **IC₅₀ value(nM)** |
|---|---|
| hum_LT-m58-1 | 0.0933 |
| hum_LT-m58-2 | 0.1990 |
| F19 | 1.3450 |

The HVEM inhibitory activity results of humanized molecules derived from chimeric antibody LT-m85 were shown in Table 16D. Exemplary anti-LIGHT antibodies hum_LT-m85-1, hum_LT-m85-2, hum_LT-m85-3, and hum_LT-m85-4 can effectively inhibit the activation of HVEM initiated luciferase signal by free LIGHT, and the inhibitory activity was superior to the positive control antibody F19.

**Table 16D: Humanized anti-LIGHT antibodies of LT-m85 inhibit the activation of HVEM reporter genes by free LIGHT**

| **Antibody name** | **IC₅₀ value(nM)** |
|---|---|
| hum_LT-m85-1 | 0.3304 |
| hum_LT-m85-2 | 0.2093 |
| hum_LT-m85-3 | 0.5642 |
| hum_LT-m85-4 | 0.4537 |
| F19 | 1.7140 |

### Activity detection of humanized anti-LIGHT antibody inhibiting human LIGHT induced MMP-9 production in BEAS-2B bronchial epithelial cells

BEAS-2B bronchial epithelial cells simultaneously express LTβR and HVEM receptors. When LIGHT binds to the receptors LTβR or HVEM, it can induce the secretion of MMP-9 in bronchial epithelial cells, leading to lung tissue damage. By detecting the level of MMP-9 produced by BEAS-2B bronchial epithelial cells, the inhibitory ability of anti-LIGHT antibodies on free LIGHT activated HVEM or LTβR receptors can be assessed as a measure of the mechanism by which the tested antibody inhibits LIGHT activation and promotes lung tissue remodeling.

BEAS-2B bronchial epithelial cells (ATCC, CRL-9609) were seeded into a 96 well plate at a density of 2 × 104 cells/well and cultured overnight in 100 µl BMEM complete medium (Lonza, CC-3171, containing rhEGF, insulin, triiodothyrone-T3, BPE, transferrin, retinic acid, epinephrine, hydrocortisone, GA-1000, with 1% PS added) at 37 °C, and 5% CO2. The epithelial cell culture medium was removed, 50 µl of His huLIGHT antigen with a concentration of 600ng/ml and 50 µl of gradient diluted humanized anti-LIGHT antibody to be tested (starting concentration of 300 µg/ml, followed by a 1:3 gradient dilution) were added to each well, of which F19 antibody was used as the positive control, and 50 µl of culture medium/well was added. After gently shaking and mixing, the cells were cultured at 37 °C and 5% CO₂ for 48 hours. The supernatant of cell culture medium was collected and the level of human MMP-9 was detected using an ELISA kit (Doctor De, EK0465) according to the manufacturer's operating instructions. In short, 100 µl/well of cell culture supernatant was added to the enzyme-linked immunosorbent assay plate, gradient diluted MMP-9 standard samples were added to the blank control wells, and the plates were reacted at 37 °C for 90 minutes. After washing the plate with 1 x washing buffer, 100 µl/well MMP-9 antibody working solution was added, and the plate was reacted at 37 °C for 60 minutes. After washing the plate with 1 x washing buffer, 100 µl/well of ABC working solution was added, and the plate was reacted at 37 °C for 30 minutes. After washing the plate with 1 x washing buffer, 90 µl/well TMB color development solution was added, and the plate was reacted at 37 °C in the dark for 5-10 minutes, and 100 µl/well termination solution was immediately added. OD 450nm was detected, MMP-9 level antibody concentration curve was generated using GraphPad Prism 5.0, and the IC50 values of each antibody inhibiting the production of MMP-9 in BEAS-2B bronchial epithelial cells induced by human LIGHT was calculated.

As shown in Figure 11 and Table 17, the inhibitory activity of the exemplary humanized anti-LIGHT antibodies (human IgG4 form) hum_LT-m37-13, hum_LT-m37-14, hum_LT-m37-15, and hum_LT-m37-16 on the induction of MMP-9 production in BEAS-2B bronchial epithelial cells by LIGHT was superior to or comparable to the chimeric antibody LT-m37, and the inhibitory activity was comparable to the positive control antibody F19.

**Table 17: Anti-LIGHT antibody inhibits human LIGHT induced MMP-9 production in BEAS-2B bronchial epithelial cells**

| **Antibody name** | **IC₅₀ value (µg/ml)** |
|---|---|
| hum_LT-m37-13 | 0.3576 |
| hum_LT-m37-14 | 0.3524 |
| hum_LT-m37-15 | 0.1973 |
| hum_LT-m37-16 | 0.3054 |
| LT-m37 | 0.3592 |
| F19 | 0.3567 |

### Activity detection of humanized anti-LIGHT antibody inhibiting the activation and proliferation of CD3+T cells stimulated by human LIGHT

The activation and proliferation of CD3+T cells require co-stimulate signals. On the one hand, CD3 molecules on the surface of CD3+T cells are stimulated by agonists; On the other hand, CD3+T cells express HVEM, and when LIGHT binds to HVEM, it releases a co-stimulate signal, thereby jointly inducing the activation and proliferation of T cells. The inhibitory activity of humanized anti-LIGHT antibodies on LIGHT induced T cell activation and proliferation was detected, as a measure of the inhibitory effect of humanized antibodies on the LIGHT-activated T cells-mediated inflammatory mechanism.

Firstly, 100 µl/well of the anti-human CD3 agonist antibody (Biolegend, item number 317326) with a concentration of 2 µg/ml was coated onto a 96 well plate and incubated overnight at 4 °C. After washing the plate twice with DPBS buffer, 100 µl of His-huLIGHT protein with a concentration of 30 µg/ml was added to each well, and incubated at 37 °C for 4 hours. After washing the plate, the liquid in the well was removed for later use. 100 µl/well of CD3+T cells (Miaoshun/TPCS, PB-N-1C) with a density of 1.5 × 106 cells/ml were inoculated into the 96 well plate mentioned above. 50 µl/well gradient diluted humanized anti-LIGHT antibody to be tested (initial concentration of 36 µg/ml, followed by 1:3 gradient dilution) were added, of which F19 antibody was used as the positive control and unrelated antibody IgG (from Staidson) was used as the negative control (NC). After slight shaking and mixing, cultured at 37 °C and 5% CO2 for 4 days. Subsequently, the proliferation of CD3+T cells was detected using the CellTiter Glo luminescent cell viability assay (Promega, G7573). In short, 50 µl/well CellTiter Glo reagent detection reagent was added, shaken in the dark for 2 minutes, let stand for 10 minutes, and 70 µl/well of reaction supernatant was transferred to a white transparent 96 well plate. Luminescence was detected for 1000ms, and Luminescence antibody concentration curves was generated using GraphPad Prism 5.0, and the IC50 values of each antibody for inhibiting the proliferation of human LIGHT stimulated CD3+T cell was calculated.

As shown in Figure 12 and Table 18, the exemplary humanized anti-LIGHT antibodies (human IgG4 form) hum_LT-m37-13, hum_LT-m37-14, hum_LT-m37-15, and hum_LT-m37-16 can effectively inhibit the activation and proliferation of CD3+T cells stimulated by LIGHT, and the inhibitory activity was comparable to the positive control antibody F19.

**Table 18: Humanized anti-LIGHT antibodies inhibit human LIGHT stimulated CD3+T cell proliferation**

| **Antibody name** | **IC₅₀ (µg/ml)** |
|---|---|
| hum_LT-m37-13 | 0.3109 |
| hum_LT-m37-14 | 0.3317 |
| hum_LT-m37-15 | 0.1953 |
| hum_LT-m37-16 | 0.3854 |
| F19 | 0.3364 |
| NC | ~4.192 |

### Example 5: Validation of the in vivo activity of anti-LIGHT antibodies in LPS induced mouse ARDS model

LPS induced mouse ARDS model: Lipopolysaccharide (LPS) induced lung inflammation and immune system activation in mice can lead to the expression of LIGHT on the surface of immune cells, as well as an increase in free LIGHT levels. The binding of LIGHT to its receptors LTβR or HVEM stimulates inflammatory cell activation, which is an important factor in exacerbating inflammation and promoting the progression of acute respiratory distress syndrome (ARDS). By detecting the inhibitory activity of anti-LIGHT antibodies on the activation of inflammatory cells in LPS induced mouse ARDS models, the ability of the tested antibodies to suppress inflammatory responses in vivo was measured.

Preparation of modeling agent: 5.5mg LPS (Sigma Aldrich, L2630-25MG) was taken and fully dissolve it in 1.1ml sterile physiological saline to prepare a 5mg/ml LPS solution for airway injection. Then, 144 µl of LPS solution was taken from it and mixed with 8.856ml of sterile physiological saline to prepare a 0.08mg/ml LPS solution for intraperitoneal injection. Prepare it before use.

LPS induced ARDS model in mice and administration: 7-week-old C57 mice were intraperitoneally injected with the modeling agent LPS 0.8mpk, with an injection volume of 10ml/kg. After 16 hours, 5mg/ml of modeling agent was administered through the airway, with an injection volume of 25 µl/mouse. One hour after administering the modeling agent through the airway, anti-LIGHT antibodies were injected into the tail vein of the experimental group mice at a dose of 15mpk. The negative control group was injected with the same dose of unrelated antibodies (from Staidson) in the same manner, while the model control group was injected with an equal volume of PBS solution in the same manner. After 48 hours of administration, the mice were euthanized and bronchoalveolar lavage fluid was collected for white blood cell counting, including the levels of total white blood cells, intermediate cells, neutrophils, and lymphocytes.

As shown in Figure 13, compared with the model control group, the negative control group did not show significant differences, but the exemplary anti-LIGHT antibodies LT-m17 and hum_LT-m37-12 in this application significantly and effectively reduced the levels of total white blood cells (Figure 13A), intermediate cells (Figure 13B), lymphocytes (Figure 13C), and neutrophils (Figure 13D) in the bronchoalveolar lavage fluid of LPS induced mouse ARDS model.

Other exemplary anti-LIGHT antibodies, hum_LT-m37-13, hum_LT-m37-14, hum_LT-m37-15, hum_LT-m37-16 (human IgG4 form), hum_LT-m58-1, hum_LT-m58-2, hum_LT-m58-5, hum_LT-m58-6, hum_LT-m85-1, hum_LT-m85-2, hum_LT-m85-3, and hum_LT-m85-4, can also effectively inhibit the levels of total white blood cells, intermediate cells, neutrophils, and lymphocytes in the bronchoalveolar lavage fluid of LPS induced mouse ARDS models (data not shown).

## Claims

1. An isolated anti-LIGHT antibody, wherein the anti-LIGHT antibody comprises a heavy chain variable domain (V_{H}) comprising:
a heavy chain complementarity determining region (HC-CDR) 1 comprising GYFIN (SEQ ID NO: 1);
an HC-CDR2 comprising RIYPYNVNTFYNQNFKG (SEQ ID NO: 8) or RIYPYNVDTFYNQNFKG (SEQ ID NO: 9); and
an HC-CDR3 comprising GTHYYGSSGAMDY (SEQ ID NO: 16);
and a light chain variable domain (V_{L}) comprising:
a light chain complementarity determining region (LC-CDR) 1 comprising KASQNVGTAVA (SEQ ID NO: 23);
an LC-CDR2 comprising SASNRYT (SEQ ID NO: 30); and
an LC-CDR3 comprising QQYSSYPYT (SEQ ID NO: 37).

2. The isolated anti-LIGHT antibody of claim 1, comprising:
(i) a V_{H} comprising an HC-CDR1 comprising the amino acid sequence of SEQ ID NO: 1, an HC-CDR2 comprising the amino acid sequence of SEQ ID NO: 8, and an HC-CDR3 comprising the amino acid sequence of SEQ ID NO: 16; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37; or
(ii) a V_{H} comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 16; and a V_{L} comprising an LC-CDR1 comprising the amino acid sequence of SEQ ID NO: 23, an LC-CDR2 comprising the amino acid sequence of SEQ ID NO: 30, and an LC-CDR3 comprising the amino acid sequence of SEQ ID NO: 37.

3. The isolated anti-LIGHT antibody of any one of claims 1-2, comprising:
(i) a V_{H} comprising the amino acid sequence of SEQ ID NO: 46, or a variant thereof having at least about 80% sequence identity to the amino acid sequence of SEQ ID NO: 46; and a V_{L} comprising the amino acid sequence of SEQ ID NO: 62, or a variant thereof having at least about 80% sequence identity to the amino acid sequence of SEQ ID NO: 62;
(ii) a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 63, or a variant thereof having at least about 80% sequence identity to the amino acid sequence of SEQ ID NO: 63;
(iii) a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 63, or a variant thereof having at least about 80% sequence identity to the amino acid sequence of SEQ ID NO: 63;
(iv) a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 63, or a variant thereof having at least about 80% sequence identity to the amino acid sequence of SEQ ID NO: 63;
(v) a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 63, or a variant thereof having at least about 80% sequence identity to the amino acid sequence of SEQ ID NO: 63;
(vi) a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 64, or a variant thereof having at least about 80% sequence identity to the amino acid sequence of SEQ ID NO: 64;
(vii) a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 64, or a variant thereof having at least about 80% sequence identity to the amino acid sequence of SEQ ID NO: 64;
(viii)a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 64, or a variant thereof having at least about 80% sequence identity to the amino acid sequence of SEQ ID NO: 64;
(ix) a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 64, or a variant thereof having at least about 80% sequence identity to the amino acid sequence of SEQ ID NO: 64;
(x) a V_{H} comprising the amino acid sequence of SEQ ID NO: 47, or a variant thereof having at least about 80% sequence identity to the amino acid sequence of SEQ ID NO: 47; and a V_{L} comprising the amino acid sequence of SEQ ID NO: 65, or a variant thereof having at least about 80% sequence identity to the amino acid sequence of SEQ ID NO: 65;
(xi) a V_{H} comprising the amino acid sequence of SEQ ID NO: 48, or a variant thereof having at least about 80% sequence identity to the amino acid sequence of SEQ ID NO: 48; and a V_{L} comprising the amino acid sequence of SEQ ID NO: 65, or a variant thereof having at least about 80% sequence identity to the amino acid sequence of SEQ ID NO: 65;
(xii) a V_{H} comprising the amino acid sequence of SEQ ID NO: 49, or a variant thereof having at least about 80% sequence identity to the amino acid sequence of SEQ ID NO: 49; and a V_{L} comprising the amino acid sequence of SEQ ID NO: 65, or a variant thereof having at least about 80% sequence identity to the amino acid sequence of SEQ ID NO: 65;
(xiii)a V_{H} comprising the amino acid sequence of SEQ ID NO: 50, or a variant thereof having at least about 80% sequence identity to the amino acid sequence of SEQ ID NO: 50; and a V_{L} comprising the amino acid sequence of SEQ ID NO: 65, or a variant thereof having at least about 80% sequence identity to the amino acid sequence of SEQ ID NO: 65;
(xiv) a V_{H} comprising the amino acid sequence of SEQ ID NO: 51, or a variant thereof having at least about 80% sequence identity to the amino acid sequence of SEQ ID NO: 51; and a V_{L} comprising the amino acid sequence of SEQ ID NO: 63, or a variant thereof having at least about 80% sequence identity to the amino acid sequence of SEQ ID NO: 63;
(xv) a V_{H} comprising the amino acid sequence of SEQ ID NO: 52, or a variant thereof having at least about 80% sequence identity to the amino acid sequence of SEQ ID NO: 52; and a V_{L} comprising the amino acid sequence of SEQ ID NO: 65, or a variant thereof having at least about 80% sequence identity to the amino acid sequence of SEQ ID NO: 65;
(xvi)a V_{H} comprising the amino acid sequence of SEQ ID NO: 53, or a variant thereof having at least about 80% sequence identity to the amino acid sequence of SEQ ID NO: 53; and a V_{L} comprising the amino acid sequence of SEQ ID NO: 65, or a variant thereof having at least about 80% sequence identity to the amino acid sequence of SEQ ID NO: 65; or
(xvii) a V_{H} comprising the amino acid sequence of SEQ ID NO: 51, or a variant thereof having at least about 80% sequence identity to the amino acid sequence of SEQ ID NO: 51; and a V_{L} comprising the amino acid sequence of SEQ ID NO: 65, or a variant thereof having at least about 80% sequence identity to the amino acid sequence of SEQ ID NO: 65.

4. An isolated anti-LIGHT antibody, comprising:
(i) a V_{H} comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and a V_{L} comprising an LC-CDR1 comprising the amino acid sequence of SEQ ID NO: 24, an LC-CDR2 comprising the amino acid sequence of SEQ ID NO: 31, and an LC-CDR3 comprising the amino acid sequence of SEQ ID NO: 38;
(ii) a V_{H} comprising an HC-CDR1 comprising the amino acid sequence of SEQ ID NO: 2, an HC-CDR2 comprising the amino acid sequence of SEQ ID NO: 10, and an HC-CDR3 comprising the amino acid sequence of SEQ ID NO: 17; and a V_{L} comprising an LC-CDR1 comprising the amino acid sequence of SEQ ID NO: 25, an LC-CDR2 comprising the amino acid sequence of SEQ ID NO: 31, and an LC-CDR3 comprising the amino acid sequence of SEQ ID NO: 39;
(iii) a V_{H} comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 40;
(iv) a V_{H} comprising an HC-CDR1 comprising the amino acid sequence of SEQ ID NO: 3, an HC-CDR2 comprising the amino acid sequence of SEQ ID NO: 11, and an HC-CDR3 comprising the amino acid sequence of SEQ ID NO: 18; and a V_{L} comprising an LC-CDR1 comprising the amino acid sequence of SEQ ID NO: 26, an LC-CDR2 comprising the amino acid sequence of SEQ ID NO: 32, and an LC-CDR3 comprising the amino acid sequence of SEQ ID NO: 41;
(v) a V_{H} comprising an HC-CDR1 comprising the amino acid sequence of SEQ ID NO: 4, an HC-CDR2 comprising the amino acid sequence of SEQ ID NO: 12, and an HC-CDR3 comprising the amino acid sequence of SEQ ID NO: 19; and a V_{L} comprising an LC-CDR1 comprising the amino acid sequence of SEQ ID NO: 27, an LC-CDR2 comprising the amino acid sequence of SEQ ID NO: 33, and an LC-CDR3 comprising the amino acid sequence of SEQ ID NO: 42; or
(vi) a V_{H} comprising an HC-CDR1 comprising the amino acid sequence of SEQ ID NO: 5, an HC-CDR2 comprising the amino acid sequence of SEQ ID NO: 13, and an HC-CDR3 comprising the amino acid sequence of SEQ ID NO: 20; and a V_{L} comprising an LC-CDR1 comprising the amino acid sequence of SEQ ID NO: 28, an LC-CDR2 comprising the amino acid sequence of SEQ ID NO: 34, and an LC-CDR3 comprising the amino acid sequence of SEQ ID NO: 43.

5. The isolated anti-LIGHT antibody of claim 4, comprising:
(i) a V_{H} comprising the amino acid sequence of SEQ ID NO: 54, or a variant thereof having at least about 80% sequence identity to the amino acid sequence of SEQ ID NO: 54; and a V_{L} comprising the amino acid sequence of SEQ ID NO: 66, or a variant thereof having at least about 80% sequence identity to the amino acid sequence of SEQ ID NO: 66;
(ii) a V_{H} comprising the amino acid sequence of SEQ ID NO: 55, or a variant thereof having at least about 80% sequence identity to the amino acid sequence of SEQ ID NO: 55; and a V_{L} comprising the amino acid sequence of SEQ ID NO: 67, or a variant thereof having at least about 80% sequence identity to the amino acid sequence of SEQ ID NO: 67;
(iii) a V_{H} comprising the amino acid sequence of SEQ ID NO: 56, or a variant thereof having at least about 80% sequence identity to the amino acid sequence of SEQ ID NO: 56; and a V_{L} comprising the amino acid sequence of SEQ ID NO: 68, or a variant thereof having at least about 80% sequence identity to the amino acid sequence of SEQ ID NO: 68;
(iv) a V_{H} comprising the amino acid sequence of SEQ ID NO: 57, or a variant thereof having at least about 80% sequence identity to the amino acid sequence of SEQ ID NO: 57; and a V_{L} comprising the amino acid sequence of SEQ ID NO: 69, or a variant thereof having at least about 80% sequence identity to the amino acid sequence of SEQ ID NO: 69;
(v) a V_{H} comprising the amino acid sequence of SEQ ID NO: 58, or a variant thereof having at least about 80% sequence identity to the amino acid sequence of SEQ ID NO: 58; and a V_{L} comprising the amino acid sequence of SEQ ID NO: 70, or a variant thereof having at least about 80% sequence identity to the amino acid sequence of SEQ ID NO: 70; or
(vi) a V_{H} comprising the amino acid sequence of SEQ ID NO: 59, or a variant thereof having at least about 80% sequence identity to the amino acid sequence of SEQ ID NO: 59; and a V_{L} comprising the amino acid sequence of SEQ ID NO: 71, or a variant thereof having at least about 80% sequence identity to the amino acid sequence of SEQ ID NO: 71.

6. An isolated anti-LIGHT antibody, wherein the anti-LIGHT antibody comprises a heavy chain variable domain (V_{H}) comprising:
a heavy chain complementarity determining region (HC-CDR) 1 comprising DHIMN (SEQ ID NO: 6);
an HC-CDR2 comprising RIYPVSGETNYNQKFMG (SEQ ID NO: 14); and
an HC-CDR3 comprising GSYYWNAMDY (SEQ ID NO: 21);
and a light chain variable domain (V_{L}) comprising:
a light chain complementarity determining region (LC-CDR) 1 comprising KASQSVDFDGDSYMN (SEQ ID NO: 29) or KASQSVDFDGESYMN (SEQ ID NO: 83);
an LC-CDR2 comprising SASNLES (SEQ ID NO: 35); and
an LC-CDR3 comprising QQSIEDPWT (SEQ ID NO: 43).

7. The isolated anti-LIGHT antibody of claim 6, comprising:
(i) a V_{H} comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and a V_{L} comprising an LC-CDR1 comprising the amino acid sequence of SEQ ID NO: 29, an LC-CDR2 comprising the amino acid sequence of SEQ ID NO: 35, and an LC-CDR3 comprising the amino acid sequence of SEQ ID NO: 43; or
(ii) a VH comprising an HC-CDR1 comprising the amino acid sequence of SEQ ID NO: 6, an HC-CDR2 comprising the amino acid sequence of SEQ ID NO: 14, and an HC-CDR3 comprising the amino acid sequence of SEQ ID NO: 21; and a V_{L} comprising an LC-CDR1 comprising the amino acid sequence of SEQ ID NO: 83, an LC-CDR2 comprising the amino acid sequence of SEQ ID NO: 35, and an LC-CDR3 comprising the amino acid sequence of SEQ ID NO: 43.

8. The isolated anti-LIGHT antibody of any one of claims 6-7, comprising:
(i) a V_{H} comprising the amino acid sequence of SEQ ID NO: 60, or a variant thereof having at least about 80% sequence identity to the amino acid sequence of SEQ ID NO: 60; and a V_{L} comprising the amino acid sequence of SEQ ID NO: 72, or a variant thereof having at least about 80% sequence identity to the amino acid sequence of SEQ ID NO: 72;
(ii) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 86, or a variant thereof having at least about 80% sequence identity to the amino acid sequence of SEQ ID NO: 86;
(iii) a V_{H} comprising the amino acid sequence of SEQ ID NO: 85, or a variant thereof having at least about 80% sequence identity to the amino acid sequence of SEQ ID NO: 85; and a V_{L} comprising the amino acid sequence of SEQ ID NO: 87, or a variant thereof having at least about 80% sequence identity to the amino acid sequence of SEQ ID NO: 87;
(iv) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 88, or a variant thereof having at least about 80% sequence identity to the amino acid sequence of SEQ ID NO: 88;
(v) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 89, or a variant thereof having at least about 80% sequence identity to the amino acid sequence of SEQ ID NO: 89;
(vi) a V_{H} comprising the amino acid sequence of SEQ ID NO: 85, or a variant thereof having at least about 80% sequence identity to the amino acid sequence of SEQ ID NO: 85; and a V_{L} comprising the amino acid sequence of SEQ ID NO: 90, or a variant thereof having at least about 80% sequence identity to the amino acid sequence of SEQ ID NO: 90; or
(vii) a V_{H} comprising the amino acid sequence of SEQ ID NO: 84, or a variant thereof having at least about 80% sequence identity to the amino acid sequence of SEQ ID NO: 84; and a V_{L} comprising the amino acid sequence of SEQ ID NO: 91, or a variant thereof having at least about 80% sequence identity to the amino acid sequence of SEQ ID NO: 91.

9. An isolated anti-LIGHT antibody, wherein the anti-LIGHT antibody comprises a heavy chain variable domain (V_{H}) comprising:
a heavy chain complementarity determining region (HC-CDR) 1 comprising SYNVH (SEQ ID NO: 7);
an HC-CDR2 comprising AVYPGNGDTSYNQKFKG (SEQ ID NO: 15); and
an HC-CDR3 comprising GSYYYTSSYFDH (SEQ ID NO: 22);
and a light chain variable domain (V_{L}) comprising:
a light chain complementarity determining region (LC-CDR) 1 comprising KASQSVDFDGDSYMN (SEQ ID NO: 29);
an LC-CDR2 comprising TASNLES (SEQ ID NO: 36); and
an LC-CDR3 comprising QQSYEDPFT (SEQ ID NO: 44).

10. The isolated anti-LIGHT antibody of claim 9, comprising:
(i) a V_{H} comprising the amino acid sequence of SEQ ID NO: 61, or a variant thereof having at least about 80% sequence identity to the amino acid sequence of SEQ ID NO: 61; and a V_{L} comprising the amino acid sequence of SEQ ID NO: 73, or a variant thereof having at least about 80% sequence identity to the amino acid sequence of SEQ ID NO: 73;
(ii) a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 95, or a variant thereof having at least about 80% sequence identity to the amino acid sequence of SEQ ID NO: 95;
(iii) a VH comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 96, or a variant thereof having at least about 80% sequence identity to the amino acid sequence of SEQ ID NO: 96;
(iv) a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 96, or a variant thereof having at least about 80% sequence identity to the amino acid sequence of SEQ ID NO: 96;
(v) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 97, or a variant thereof having at least about 80% sequence identity to the amino acid sequence of SEQ ID NO: 97;
(vi) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 98, or a variant thereof having at least about 80% sequence identity to the amino acid sequence of SEQ ID NO: 98;
(vii) a V_{H} comprising the amino acid sequence of SEQ ID NO: 93, or a variant thereof having at least about 80% sequence identity to the amino acid sequence of SEQ ID NO: 93; and a V_{L} comprising the amino acid sequence of SEQ ID NO: 98, or a variant thereof having at least about 80% sequence identity to the amino acid sequence of SEQ ID NO: 98;
(viii)a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 98, or a variant thereof having at least about 80% sequence identity to the amino acid sequence of SEQ ID NO: 98;
(ix) a V_{H} comprising the amino acid sequence of SEQ ID NO: 94, or a variant thereof having at least about 80% sequence identity to the amino acid sequence of SEQ ID NO: 94; and a V_{L} comprising the amino acid sequence of SEQ ID NO: 99, or a variant thereof having at least about 80% sequence identity to the amino acid sequence of SEQ ID NO: 99; or
(x) a V_{H} comprising the amino acid sequence of SEQ ID NO: 92, or a variant thereof having at least about 80% sequence identity to the amino acid sequence of SEQ ID NO: 92; and a V_{L} comprising the amino acid sequence of SEQ ID NO: 99, or a variant thereof having at least about 80% sequence identity to the amino acid sequence of SEQ ID NO: 99.

11. The isolated anti-LIGHT antibody of any one of claims 1-10, wherein the anti- LIGHT antibody binds to the LIGHT with a Kd from about 0.1 pM to about 10 nM.

12. The isolated anti-LIGHT antibody of any one of claims 1-11, wherein the anti-LIGHT antibody comprises an Fc fragment.

13. The isolated anti-LIGHT antibody of claim 12, wherein the anti-LIGHT antibody is a full-length IgA, IgD, IgE, IgG or IgM antibody.

14. The isolated anti-LIGHT antibody of claim 13, wherein the anti-LIGHT antibody is a full-length IgG1, IgG2, IgG3 or IgG4 antibody.

15. The isolated anti-LIGHT antibody of any one of claims 1-14, wherein the anti-LIGHT antibody is chimeric, human, or humanized.

16. The isolated anti-LIGHT antibody according to any one of claims 1-11, wherein the anti-LIGHT antibody is an antigen binding fragment selected from the group consisting of a Fab, a Fab', a F(ab)'2, a Fab'-SH, a single-chain Fv (scFv), an Fv fragment, a dAb, a Fd, a nanobody, a diabody, and a linear antibody.

17. An isolated nucleic acid molecule that encodes the anti-LIGHT antibody according to any one of claims 1-16.

18. A vector comprising the nucleic acid molecule of claim 17.

19. An isolated host cell comprising the anti-LIGHT antibody of any one of claims 1-16, the nucleic acid molecule of claim 17, or the vector of claim 18.

20. A method of producing an anti-LIGHT antibody, comprising:
a) culturing the host cell of claim 19 under conditions effective to express the anti-LIGHT antibody; and
b) obtaining the expressed anti-LIGHT antibody from the host cell.

21. A pharmaceutical composition comprising the anti-LIGHT antibody according to any one of claims 1-16, the nucleic acid molecule of claim 17, the vector of claim 18, the isolated host cell of claim 19, or the antibody produced according to the method of claim 20, and a pharmaceutically acceptable carrier.

22. A method of treating a disease or condition in an individual in need thereof, comprising administering to the individual an effective amount of the anti-LIGHT antibody according to any one of claims 1-16, the nucleic acid molecule of claim 17, the vector of claim 18, the isolated host cell of claim 19, the antibody produced according to the method of claim 20 or the pharmaceutical composition of claim 21.

23. Use of the anti-LIGHT antibody according to any one of claims 1-16, the nucleic acid molecule of claim 17, the vector of claim 18, the isolated host cell of claim 19, the antibody produced according to the method of claim 20 or the pharmaceutical composition of claim 21 in the manufacture of a medicament for treating a disease or condition in an individual in need thereof.

24. The method of claim 22 or the use of claim 23, wherein the disease or condition is a disease and/or condition caused by the disorder of the LIGHT signaling pathway, such as autoimmune disease and/or inflammatory disease.

25. The method of claim 22 or the use of claim 23, wherein the disease or condition is selected from the group consisting of inflammatory bowel disease (IBD), Crohn's disease, ulcerative colitis, ankylosing spondylitis, atopic dermatitis, rheumatoid arthritis, bronchiolitis, transplant rejection, allograft rejection, graft-versus-host disease (GVHD), asthma, eosinophilic esophagitis, acute respiratory distress syndrome (ARDS), chronic heart failure, chronic obstructive pulmonary disease (COPD), septic shock, fibrotic disease, skin fibrosis, idiopathic pulmonary fibrosis, renal fibrosis, chronic kidney disease, systemic sclerosis, multiple sclerosis, Sjögren's syndrome, lupus, airway inflammation, psoriasis, hepatitis, celiac disease, primary biliary cirrhosis, autoimmune hemolytic anemia, autoimmune neonatal thrombocytopenia, primary thrombocytopenic purpura, non-alcoholic fatty liver disease, and COVID-19.
